# EUROPEAN PATENT APPLICATION

(11) **EP 3 689 907 A1**
(43) Date of publication of application: **05.08.2020**
(21) Application number: 19154852.8
(22) Date of filing: 31.01.2019
(51) Int. Cl.: C07K 16/24

(54) **ANTIBODIES TARGETING IL-17A AND METHODS OF USE THEREOF**

(71) Applicant: Numab Therapeutics AG, 8820 Wädenswil (CH)
(72) Inventor: The designation of the inventor has not yet been filed
(74) Representative: Virnekäs, Bernhard

(57) **Abstract**

The present invention relates to an isolated antibody which specifically binds human IL-17A, pharmaceutical compositions and methods of use thereof. The present invention further relates to a kit comprising said antibody, a nucleic acid comprising a nucleotide sequence encoding said antibody, a vector comprising said nucleic acid, a host cell comprising said nucleic acid or said vector, and a method of producing said antibody.

SEQUENCE LISTING

<110>  Numab Innovation AG

<120>  ANTIBODIES TARGETING IL-17A AND METHODS OF USE THEREOF

<130>  115527P877EP

<160>  38

<170>  PatentIn version 3.5

<210>  1
<211>  11
<212>  PRT
<213>  Artificial Sequence

<220>
<223>  artificial antibody-based sequence

<400>  1

Gly Phe Ser Phe Ser Gly Phe Tyr Tyr Met Cys
1               5                   10


<210>  2
<211>  17
<212>  PRT
<213>  Artificial Sequence

<220>
<223>  artificial antibody-based sequence

<400>  2

Cys Ile Asp Thr Gly Asp Gly Thr Thr Tyr Tyr Ala Ser Trp Ala Lys
1               5                   10                  15


Gly




<210>  3
<211>  17
<212>  PRT
<213>  Artificial Sequence

<220>
<223>  artificial antibody-based sequence

<400>  3

Arg Asp Ala Ala Tyr Gly Gly Tyr Ala Tyr Pro Ala His Gly Met Asp
1               5                   10                  15


Leu



<210>  4

<211>  6
<212>  PRT
<213>  Artificial Sequence

<220>
<223>  artificial antibody-based sequence

<400>  4

Gly Phe Tyr Tyr Met Cys
1               5


<210>  5
<211>  17
<212>  PRT
<213>  Artificial Sequence

<220>
<223>  artificial antibody-based sequence

<400>  5

Cys Ile Asp Thr Gly Asp Gly Thr Thr Tyr Tyr Ala Ser Trp Ala Lys
1               5                   10                  15


Gly




<210>  6
<211>  16
<212>  PRT
<213>  Artificial Sequence

<220>
<223>  artificial antibody-based sequence

<400>  6

Asp Ala Ala Tyr Gly Gly Tyr Ala Tyr Pro Ala His Gly Met Asp Leu
1               5                   10                  15


<210>  7
<211>  8
<212>  PRT
<213>  Artificial Sequence

<220>
<223>  artificial antibody-based sequence

<400>  7

Gly Phe Ser Phe Ser Gly Phe Tyr
1               5


<210>  8
<211>  4
<212>  PRT
<213>  Artificial Sequence

<220>
<223>  artificial antibody-based sequence

<400>  8

Thr Gly Asp Gly
1


<210>  9
<211>  14
<212>  PRT
<213>  Artificial Sequence

<220>
<223>  artificial antibody-based sequence

<400>  9

Ala Ala Tyr Gly Gly Tyr Ala Tyr Pro Ala His Gly Met Asp
1               5                   10


<210>  10
<211>  126
<212>  PRT
<213>  Artificial Sequence

<220>
<223>  artificial antibody-based sequence

<400>  10

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Phe Ser Gly Phe
            20                  25                  30


Tyr Tyr Met Cys Trp Val Arg Gln Ala Pro Gly Lys Gly Leu Glu Trp
        35                  40                  45


Ile Gly Cys Ile Asp Thr Gly Asp Gly Thr Thr Tyr Tyr Ala Ser Trp
    50                  55                  60


Ala Lys Gly Arg Phe Thr Ile Ser Arg Asp Asn Ser Lys Asn Thr Val
65                  70                  75                  80


Tyr Leu Gln Met Asn Ser Leu Arg Ala Glu Asp Thr Ala Val Tyr Tyr
                85                  90                  95


Cys Ala Arg Asp Ala Ala Tyr Gly Gly Tyr Ala Tyr Pro Ala His Gly
            100                 105                 110


Met Asp Leu Trp Gly Gln Gly Thr Leu Val Thr Val Ser Ser

115                 120                 125

<210>  11
<211>  126
<212>  PRT
<213>  Artificial Sequence

<220>
<223>  artificial antibody-based sequence

<400>  11

Glu Val Gln Leu Val Glu Ser Gly Gly Gly Leu Val Gln Pro Gly Gly
1               5                   10                  15

Ser Leu Thr Leu Ser Cys Ala Ala Ser Gly Phe Ser Phe Ser Gly Phe
            20                  25                  30

Tyr Tyr Met Cys Trp Val Arg Gln Ala Pro Gly Lys Gly Leu Glu Trp
        35                  40                  45

Ile Gly Cys Ile Asp Thr Gly Asp Gly Thr Thr Tyr Tyr Ala Ser Trp
    50                  55                  60

Ala Lys Gly Arg Phe Thr Ile Ser Arg Asp Asn Ser Lys Asn Thr Val
65                  70                  75                  80

Tyr Leu Gln Met Asn Ser Leu Arg Ala Glu Asp Thr Ala Val Tyr Tyr
                85                  90                  95

Cys Ala Arg Asp Ala Ala Tyr Gly Gly Tyr Ala Tyr Pro Ala His Gly
            100                 105                 110

Met Asp Leu Trp Gly Pro Gly Thr Leu Val Thr Val Ser Ser
        115                 120                 125

<210>  12
<211>  11
<212>  PRT
<213>  Artificial Sequence

<220>
<223>  artificial antibody-based sequence

<400>  12

Gln Ala Ser Glu Ser Ile Tyr Arg Tyr Leu Ser
1               5                   10

<210>  13
<211>  7
<212>  PRT
<213>  Artificial Sequence

<220>
<223>  artificial antibody-based sequence

<400>  13

Leu Ala Ser Thr Leu Thr Ser
1               5


<210>  14
<211>  14
<212>  PRT
<213>  Artificial Sequence

<220>
<223>  artificial antibody-based sequence

<400>  14

Gln Ser Asn Phe Gly Thr Ala Ser Thr Thr Tyr Tyr Asn Thr
1               5                   10


<210>  15
<211>  11
<212>  PRT
<213>  Artificial Sequence

<220>
<223>  artificial antibody-based sequence

<400>  15

Gln Ala Ser Glu Ser Ile Tyr Arg Tyr Leu Ser
1               5                   10


<210>  16
<211>  7
<212>  PRT
<213>  Artificial Sequence

<220>
<223>  artificial antibody-based sequence

<400>  16

Leu Ala Ser Thr Leu Thr Ser
1               5


<210>  17
<211>  14
<212>  PRT
<213>  Artificial Sequence

<220>
<223>  artificial antibody-based sequence

<400>  17

Gln Ser Asn Phe Gly Thr Ala Ser Thr Thr Tyr Tyr Asn Thr

1               5                   10


<210>  18
<211>  7
<212>  PRT
<213>  Artificial Sequence

<220>
<223>  artificial antibody-based sequence

<400>  18

Ser Glu Ser Ile Tyr Arg Tyr
1               5


<210>  19
<211>  3
<212>  PRT
<213>  Artificial Sequence

<220>
<223>  artificial antibody-based sequence

<400>  19

Leu Ala Ser
1


<210>  20
<211>  11
<212>  PRT
<213>  Artificial Sequence

<220>
<223>  artificial antibody-based sequence

<400>  20

Asn Phe Gly Thr Ala Ser Thr Thr Tyr Tyr Asn
1               5                   10


<210>  21
<211>  113
<212>  PRT
<213>  Artificial Sequence

<220>
<223>  artificial antibody-based sequence

<400>  21

Asp Ile Gln Met Thr Gln Ser Pro Ser Ser Leu Ser Ala Ser Val Gly
1               5                   10                  15


Asp Arg Val Thr Ile Thr Cys Gln Ala Ser Glu Ser Ile Tyr Arg Tyr
            20                  25                  30

Leu Ser Trp Tyr Gln Gln Lys Pro Gly Lys Ala Pro Lys Leu Leu Ile
        35                  40                  45

Tyr Leu Ala Ser Thr Leu Thr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Gly Thr Ala Ser
                85                  90                  95

Thr Thr Tyr Tyr Asn Thr Phe Gly Gly Gly Thr Lys Leu Thr Val Leu
            100                 105                 110

Gly

<210>  22
<211>  113
<212>  PRT
<213>  Artificial Sequence

<220>
<223>  artificial antibody-based sequence

<400>  22

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Ser Ile Tyr Arg Tyr
            20                  25                  30

Leu Ser Trp Tyr Gln Gln Lys Pro Gly Lys Ala Pro Lys Leu Leu Ile
        35                  40                  45

Tyr Leu Ala Ser Thr Leu Thr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Gly Thr Ala Ser
                85                  90                  95

Thr Thr Tyr Tyr Asn Thr Phe Gly Gly Gly Thr Lys Leu Thr Val Leu
            100                 105                 110

Gly

<210>  23
<211>  20
<212>  PRT
<213>  Artificial Sequence

<220>
<223>  artificial antibody-based sequence

<400>  23

Gly Gly Gly Gly Ser Gly Gly Gly Gly Ser Gly Gly Gly Gly Ser Gly
1               5                   10                  15

Gly Gly Gly Ser
            20

<210>  24
<211>  259
<212>  PRT
<213>  Artificial Sequence

<220>
<223>  artificial antibody-based sequence

<400>  24

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Ser Ile Tyr Arg Tyr
            20                  25                  30

Leu Ser Trp Tyr Gln Gln Lys Pro Gly Lys Ala Pro Lys Leu Leu Ile
        35                  40                  45

Tyr Leu Ala Ser Thr Leu Thr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Gly Thr Ala Ser
                85                  90                  95

Thr Thr Tyr Tyr Asn Thr Phe Gly Gly Gly Thr Lys Leu Thr Val Leu
            100                 105                 110

Gly Gly Gly Gly Gly Ser Gly Gly Gly Gly Ser Gly Gly Gly Gly Ser
        115                 120                 125

Gly Gly Gly Gly Ser Glu Val Gln Leu Val Glu Ser Gly Gly Gly Leu
    130                 135                 140

Val Gln Pro Gly Gly Ser Leu Arg Leu Ser Cys Ala Ala Ser Gly Phe
145                 150                 155                 160

Ser Phe Ser Gly Phe Tyr Tyr Met Cys Trp Val Arg Gln Ala Pro Gly
                165                 170                 175

Lys Gly Leu Glu Trp Ile Gly Cys Ile Asp Thr Gly Asp Gly Thr Thr
            180                 185                 190

Tyr Tyr Ala Ser Trp Ala Lys Gly Arg Phe Thr Ile Ser Arg Asp Asn
        195                 200                 205

Ser Lys Asn Thr Val Tyr Leu Gln Met Asn Ser Leu Arg Ala Glu Asp
    210                 215                 220

Thr Ala Val Tyr Tyr Cys Ala Arg Asp Ala Ala Tyr Gly Gly Tyr Ala
225                 230                 235                 240

Tyr Pro Ala His Gly Met Asp Leu Trp Gly Gln Gly Thr Leu Val Thr
                245                 250                 255

Val Ser Ser

<210>  25
<211>  259
<212>  PRT
<213>  Artificial Sequence

<220>
<223>  artificial antibody-based sequence

<400>  25

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Ser Ile Tyr Arg Tyr
            20                  25                  30

Leu Ser Trp Tyr Gln Gln Lys Pro Gly Lys Ala Pro Lys Leu Leu Ile
        35                  40                  45

Tyr Leu Ala Ser Thr Leu Thr Ser Gly Val Pro Ser Arg Phe Ser Gly
    50                  55                  60

Ser Gly Ser Gly Thr Asp Phe Thr Leu Thr Ile Ser Ser Leu Gln Pro

65                  70                  75                  80

Glu Asp Phe Ala Thr Tyr Tyr Cys Gln Ser Asn Phe Gly Thr Ala Ser
                85                  90                  95

Thr Thr Tyr Tyr Asn Thr Phe Gly Gly Gly Thr Lys Leu Thr Val Leu
            100                 105                 110

Gly Gly Gly Gly Gly Ser Gly Gly Gly Gly Ser Gly Gly Gly Gly Ser
        115                 120                 125

Gly Gly Gly Gly Ser Glu Val Gln Leu Val Glu Ser Gly Gly Gly Leu
    130                 135                 140

Val Gln Pro Gly Gly Ser Leu Thr Leu Ser Cys Ala Ala Ser Gly Phe
145                 150                 155                 160

Ser Phe Ser Gly Phe Tyr Tyr Met Cys Trp Val Arg Gln Ala Pro Gly
                165                 170                 175

Lys Gly Leu Glu Trp Ile Gly Cys Ile Asp Thr Gly Asp Gly Thr Thr
            180                 185                 190

Tyr Tyr Ala Ser Trp Ala Lys Gly Arg Phe Thr Ile Ser Arg Asp Asn
        195                 200                 205

Ser Lys Asn Thr Val Tyr Leu Gln Met Asn Ser Leu Arg Ala Glu Asp
    210                 215                 220

Thr Ala Val Tyr Tyr Cys Ala Arg Asp Ala Ala Tyr Gly Gly Tyr Ala
225                 230                 235                 240

Tyr Pro Ala His Gly Met Asp Leu Trp Gly Pro Gly Thr Leu Val Thr
                245                 250                 255

Val Ser Ser

<210>  26
<211>  11
<212>  PRT
<213>  Artificial Sequence

<220>
<223>  artificial antibody-based sequence

<400>  26

Phe Gly Thr Gly Thr Lys Val Thr Val Leu Gly
1               5                   10

<210>  27
<211>  11
<212>  PRT
<213>  Artificial Sequence

<220>
<223>  artificial antibody-based sequence

<400>  27

Phe Gly Gly Gly Thr Lys Leu Thr Val Leu Gly
1               5                   10


<210>  28
<211>  11
<212>  PRT
<213>  Artificial Sequence

<220>
<223>  artificial antibody-based sequence

<400>  28

Phe Gly Gly Gly Thr Gln Leu Ile Ile Leu Gly
1               5                   10


<210>  29
<211>  11
<212>  PRT
<213>  Artificial Sequence

<220>
<223>  artificial antibody-based sequence

<400>  29

Phe Gly Glu Gly Thr Glu Leu Thr Val Leu Gly
1               5                   10


<210>  30
<211>  11
<212>  PRT
<213>  Artificial Sequence

<220>
<223>  artificial antibody-based sequence

<400>  30

Phe Gly Ser Gly Thr Lys Val Thr Val Leu Gly
1               5                   10


<210>  31
<211>  11
<212>  PRT
<213>  Artificial Sequence

<220>
<223>  artificial antibody-based sequence

<400>  31

Phe Gly Gly Gly Thr Gln Leu Thr Val Leu Gly
1               5                   10


<210>  32
<211>  11
<212>  PRT
<213>  Artificial Sequence

<220>
<223>  artificial antibody-based sequence

<400>  32

Phe Gly Gly Gly Thr Gln Leu Thr Ala Leu Gly
1               5                   10


<210>  33
<211>  155
<212>  PRT
<213>  Homo sapiens

<400>  33

Met Thr Pro Gly Lys Thr Ser Leu Val Ser Leu Leu Leu Leu Leu Ser
1               5                   10                  15

Leu Glu Ala Ile Val Lys Ala Gly Ile Ala Ile Pro Gln Asn Pro Gly
            20                  25                  30

Cys Pro Asn Ser Glu Asp Lys Asn Phe Pro Arg Thr Val Met Val Asn
        35                  40                  45

Leu Asn Ile His Asn Arg Asn Thr Asn Thr Asn Pro Lys Arg Ser Ser
    50                  55                  60

Asp Tyr Tyr Asn Arg Ser Thr Ser Pro Trp Asn Leu His Arg Asn Glu
65                  70                  75                  80

Asp Pro Glu Arg Tyr Pro Ser Val Ile Trp Glu Ala Lys Cys Arg His
                85                  90                  95

Leu Gly Cys Val Asn Ala Asp Gly Lys Val Asp Tyr His Met Asn Ser
            100                 105                 110

Val Pro Ile Gln Gln Glu Ile Leu Val Leu Arg Arg Glu Pro Pro His
        115                 120                 125

Cys Pro Asn Ser Phe Arg Leu Glu Lys Ile Leu Val Ser Val Gly Cys

130                 135                 140

Thr Cys Val Thr Pro Ile Val His His Val Ser
145                 150                 155

<210>  34
<211>  180
<212>  PRT
<213>  Homo sapiens

<400>  34

Met Asp Trp Pro His Asn Leu Leu Phe Leu Leu Thr Ile Ser Ile Phe
1               5                   10                  15

Leu Gly Leu Gly Gln Pro Arg Ser Pro Lys Ser Lys Arg Lys Gly Gln
            20                  25                  30

Gly Arg Pro Gly Pro Leu Ala Pro Gly Pro His Gln Val Pro Leu Asp
        35                  40                  45

Leu Val Ser Arg Met Lys Pro Tyr Ala Arg Met Glu Glu Tyr Glu Arg
    50                  55                  60

Asn Ile Glu Glu Met Val Ala Gln Leu Arg Asn Ser Ser Glu Leu Ala
65                  70                  75                  80

Gln Arg Lys Cys Glu Val Asn Leu Gln Leu Trp Met Ser Asn Lys Arg
                85                  90                  95

Ser Leu Ser Pro Trp Gly Tyr Ser Ile Asn His Asp Pro Ser Arg Ile
            100                 105                 110

Pro Val Asp Leu Pro Glu Ala Arg Cys Leu Cys Leu Gly Cys Val Asn
        115                 120                 125

Pro Phe Thr Met Gln Glu Asp Arg Ser Met Val Ser Val Pro Val Phe
    130                 135                 140

Ser Gln Val Pro Val Arg Arg Arg Leu Cys Pro Pro Pro Pro Arg Thr
145                 150                 155                 160

Gly Pro Cys Arg Gln Arg Ala Val Met Glu Thr Ile Ala Val Gly Cys
                165                 170                 175

Thr Cys Ile Phe
            180

<210>  35

<211>  197
<212>  PRT
<213>  Homo sapiens

<400>  35

Met Thr Leu Leu Pro Gly Leu Leu Phe Leu Thr Trp Leu His Thr Cys
1               5                   10                  15

Leu Ala His His Asp Pro Ser Leu Arg Gly His Pro His Ser His Gly
            20                  25                  30

Thr Pro His Cys Tyr Ser Ala Glu Glu Leu Pro Leu Gly Gln Ala Pro
        35                  40                  45

Pro His Leu Leu Ala Arg Gly Ala Lys Trp Gly Gln Ala Leu Pro Val
    50                  55                  60

Ala Leu Val Ser Ser Leu Glu Ala Ala Ser His Arg Gly Arg His Glu
65                  70                  75                  80

Arg Pro Ser Ala Thr Thr Gln Cys Pro Val Leu Arg Pro Glu Glu Val
                85                  90                  95

Leu Glu Ala Asp Thr His Gln Arg Ser Ile Ser Pro Trp Arg Tyr Arg
            100                 105                 110

Val Asp Thr Asp Glu Asp Arg Tyr Pro Gln Lys Leu Ala Phe Ala Glu
        115                 120                 125

Cys Leu Cys Arg Gly Cys Ile Asp Ala Arg Thr Gly Arg Glu Thr Ala
    130                 135                 140

Ala Leu Asn Ser Val Arg Leu Leu Gln Ser Leu Leu Val Leu Arg Arg
145                 150                 155                 160

Arg Pro Cys Ser Arg Asp Gly Ser Gly Leu Pro Thr Pro Gly Ala Phe
                165                 170                 175

Ala Phe His Thr Glu Phe Ile His Val Pro Val Gly Cys Thr Cys Val
            180                 185                 190

Leu Pro Arg Ser Val
        195


<210>  36
<211>  202
<212>  PRT
<213>  Homo sapiens

<400>  36

Met Leu Val Ala Gly Phe Leu Leu Ala Leu Pro Pro Ser Trp Ala Ala
1               5                   10                  15

Gly Ala Pro Arg Ala Gly Arg Arg Pro Ala Arg Pro Arg Gly Cys Ala
            20                  25                  30

Asp Arg Pro Glu Glu Leu Leu Glu Gln Leu Tyr Gly Arg Leu Ala Ala
        35                  40                  45

Gly Val Leu Ser Ala Phe His His Thr Leu Gln Leu Gly Pro Arg Glu
    50                  55                  60

Gln Ala Arg Asn Ala Ser Cys Pro Ala Gly Gly Arg Pro Ala Asp Arg
65                  70                  75                  80

Arg Phe Arg Pro Pro Thr Asn Leu Arg Ser Val Ser Pro Trp Ala Tyr
                85                  90                  95

Arg Ile Ser Tyr Asp Pro Ala Arg Tyr Pro Arg Tyr Leu Pro Glu Ala
            100                 105                 110

Tyr Cys Leu Cys Arg Gly Cys Leu Thr Gly Leu Phe Gly Glu Glu Asp
        115                 120                 125

Val Arg Phe Arg Ser Ala Pro Val Tyr Met Pro Thr Val Val Leu Arg
    130                 135                 140

Arg Thr Pro Ala Cys Ala Gly Gly Arg Ser Val Tyr Thr Glu Ala Tyr
145                 150                 155                 160

Val Thr Ile Pro Val Gly Cys Thr Cys Val Pro Glu Pro Glu Lys Asp
                165                 170                 175

Ala Asp Ser Ile Asn Ser Ser Ile Asp Lys Gln Gly Ala Lys Leu Leu
            180                 185                 190

Leu Gly Pro Asn Asp Ala Pro Ala Gly Pro
        195                 200


<210>  37
<211>  177
<212>  PRT
<213>  Homo sapiens

<400>  37

Met Arg Glu Arg Pro Arg Leu Gly Glu Asp Ser Ser Leu Ile Ser Leu
1               5                   10                  15

Phe Leu Gln Val Val Ala Phe Leu Ala Met Val Met Gly Thr His Thr
            20                  25                  30

Tyr Ser His Trp Pro Ser Cys Cys Pro Ser Lys Gly Gln Asp Thr Ser
        35                  40                  45

Glu Glu Leu Leu Arg Trp Ser Thr Val Pro Val Pro Pro Leu Glu Pro
    50                  55                  60

Ala Arg Pro Asn Arg His Pro Glu Ser Cys Arg Ala Ser Glu Asp Gly
65                  70                  75                  80

Pro Leu Asn Ser Arg Ala Ile Ser Pro Trp Arg Tyr Glu Leu Asp Arg
                85                  90                  95

Asp Leu Asn Arg Leu Pro Gln Asp Leu Tyr His Ala Arg Cys Leu Cys
            100                 105                 110

Pro His Cys Val Ser Leu Gln Thr Gly Ser His Met Asp Pro Arg Gly
        115                 120                 125

Asn Ser Glu Leu Leu Tyr His Asn Gln Thr Val Phe Tyr Arg Arg Pro
    130                 135                 140

Cys His Gly Glu Lys Gly Thr His Lys Gly Tyr Cys Leu Glu Arg Arg
145                 150                 155                 160

Leu Tyr Arg Val Ser Leu Ala Cys Val Cys Val Arg Pro Arg Val Met
                165                 170                 175

Gly

<210>  38
<211>  163
<212>  PRT
<213>  Homo sapiens

<400>  38

Met Thr Val Lys Thr Leu His Gly Pro Ala Met Val Lys Tyr Leu Leu
1               5                   10                  15

Leu Ser Ile Leu Gly Leu Ala Phe Leu Ser Glu Ala Ala Ala Arg Lys
            20                  25                  30

Ile Pro Lys Val Gly His Thr Phe Phe Gln Lys Pro Glu Ser Cys Pro
        35                  40                  45

Pro Val Pro Gly Gly Ser Met Lys Leu Asp Ile Gly Ile Ile Asn Glu
    50                  55                  60

Asn Gln Arg Val Ser Met Ser Arg Asn Ile Glu Ser Arg Ser Thr Ser
65                  70                  75                  80

Pro Trp Asn Tyr Thr Val Thr Trp Asp Pro Asn Arg Tyr Pro Ser Glu
                85                  90                  95

Val Val Gln Ala Gln Cys Arg Asn Leu Gly Cys Ile Asn Ala Gln Gly
            100                 105                 110

Lys Glu Asp Ile Ser Met Asn Ser Val Pro Ile Gln Gln Glu Thr Leu
        115                 120                 125

Val Val Arg Arg Lys His Gln Gly Cys Ser Val Ser Phe Gln Leu Glu
    130                 135                 140

Lys Val Leu Val Thr Val Gly Cys Thr Cys Val Thr Pro Val Ile His
145                 150                 155                 160

His Val Gln

## Description

### FIELD OF THE INVENTION

The present invention relates to an isolated antibody which specifically binds human IL-17A, pharmaceutical compositions and methods of use thereof. The present invention further relates to a kit comprising said antibody, a nucleic acid comprising a nucleotide sequence encoding said antibody, a vector comprising said nucleic acid, a host cell comprising said nucleic acid or said vector, and a method of producing said antibody.

### BACKGROUND OF THE INVENTION

The interleukin 17 (IL-17) family in human and in mice is composed of six cytokines, IL-17A, IL-17B, IL-17C, IL-17D, IL-17E (also called IL-25) and IL-17F, and plays roles in acute and chronic inflammatory responses. The interleukin 17 receptor (IL-17R) family consists of five members, namely IL-17RA, IL-17RB, IL-17RC, IL-17RD and IL-17RE.

Interleukin-17A (IL-17A or IL17A, synonymous with IL-17 or cytotoxic T-lymphocyte-associated antigen-8 (CTLA-8)) is a homodimeric pro-inflammatory cytokine. IL-17A is produced by a subset of memory CD4+ T cells (termed Th17), CD8+ T cells (Tc17), invariant NKT cells, γδ T cells, non-T non-B lymphocytes (termed type 3 innate lymphoid cells) and neutrophils. IL-17A and IL-17F form a distinct subgroup within the IL-17 family. They share the greatest sequence homology and identity within IL-17 family, while other members of the IL-17 family have a significantly lower sequence identity to IL-17A (Starnes, T., et al., J Immunol. 167(8):4137-40 (2001); Aggarwal, S. and Gurney, A. L., J. Leukoc Biol, 71(1): 1-8 (2002)). Both IL-17A and IL-17F signal through a heterodimeric receptor complex composed of IL-17RA and IL-17RC (Toy D., et al., J Immunol. 2006; Wright JF., et al., J Immunol. 2008; 181(4):2799-2805). IL-17A and IL-17F can form IL-17A/A or IL-17F/F disulphide linked homodimers and IL-17A/F disulphide linked heterodimers (Wright JF. et al., J Immunol. 2008; 181(4):2799-2805; Liang SC. et al., J Immunol. 2007; 179(11):7791-7799). IL-17A as well as IL-17F induces expression of pro-inflammatory cytokines and anti-microbial peptides.

Human IL-17A (CTLA-8, Swiss Prot Q 16552, also referred to as IL-17 or IL17; SEQ ID NO: 33) is implicated in various inflammatory conditions such as autoimmune diseases, metabolic disorders and cancer (Ouyang W., et al., Immunity. 2008; 28(4):454-467; Milner JD., Curr Opin Immunol. 2011; 23(6):784-788; Kuchroo VK., et al., Nat Med. 2012; 18(1):42-47; Ahmed M. and Gaffen SL., Cytokine Growth Factor Rev. 2010; 21(6):449-453; Trinchieri G., Annu Rev Immunol. 2012; 30:677-706; Gallimore AM, Godkin A., N Engl J Med. 2013; 368(3):282-284; Ye P., et al., J Exp Med. 2001; 194(4):519-527; Chung DR., et al., J Immunol. 2003; 170(4):1958-1963; Huang W., et al., J Infect Dis. 2004; 190(3):624-631; Ishigame H., et al., Immunity. 2009; 30(1):108-119; see for review Gu C., et al., Cytokine. 2013 Nov 64(2)). IL-17A plays a role in the induction of other inflammatory cytokines and chemokines for neutrophil recruitment, acute phase proteins, anti-microbial peptides, mucins, matrix metalloproteinases and adhesion molecules. IL-17A also synergizes with other cytokines including TNFα and IL-1 beta to further induce chemokine expression (Chabaud M., et al., J. Immunol. 161(1):409-14 (1998)).

Pathological production of IL-17A leads to excessive inflammation and tissue damage (see for review Gu et al., Cytokine. 2013 November; 64(2)). High IL-17A levels were found in multiple sclerosis (MS), psoriasis, asthma, Crohn's disease and rheumatoid arthritis patients. Treatment of animals with IL-17A neutralizing antibodies decreases disease incidence and severity in autoimmune encephalomyelitis (Komiyama, Y. et al, J. Immunol. 177 (2006) 566-573). In addition, IL-17A neutralizing antibodies reduce severity and incidence of mouse rheumatoid arthritis model of collagen induced arthritis, and high levels of IL-17A can be detected in the synovial fluid of inflamed joints from rheumatoid arthritis patients (Ziolkowska, M. et al, J. Immunol. 164 (2000) 2832-2838; Kotake, S. et al, J. Clin. Invest. 103 (1999) 1345-1352; Hellings P.W. et al, Am. J. Resp. Cell Mol. Biol. 28 (2003) 42-50).

Numerous experimental evidence in human and animal models has supported the development of IL-17A-targeted therapies. Several anti-IL-17 antibodies were developed including AIN457 (secukinumab; see U.S. Patent No.7, 807,155 and WO 06/013107), LY2439821 (ixekizumab; see U.S. Patent Nos. 7,838,638 and 8,110,191 and WO 07/070750), SCH900117 (Merck), RG4943 (Roche), etc. Examples of anti-IL-17A antibodies are disclosed in WO 2006/013107, WO 2006/054059, WO 2007/070750, WO 2007/149032, WO 2008/001063, WO 2008/021156, WO 2010/034443, WO 2010/102251, WO 2012/018767, WO 2014/161570, WO 2014/001368, WO 2014/122613, WO 2015/070697, WO 2015/137843, WO 2016/048188, WO 2016/113557, WO 2016/138842, WO 2017/068472.

Several clinical trials with various molecules blocking IL-17A signaling have been conducted or are still ongoing. The biologics targeting either IL-17A or its receptor and their efficacies are being evaluated in the setting of inflammatory or autoimmune disorders, such as rheumatoid arthritis, ankylosing spondyloarthropathy, Crohn's disease, psoriasis, multiple sclerosis and ozone-induced neutrophilia.

For example, secukinumab, a fully human IgGlK anti-IL-17A monoclonal antibody (U.S. Patent No.7, 807,155 and WO 2006/013107), is now approved for the treatment of psoriasis, psoriatic arthritis and ankylosing spondylitis behavior (see for review Wang et al., Eur J Rheumatol 2017 (4) 272-7). In phase III studies assessing the long-term efficacy and safety of secukinumab in subjects with psoriatic arthritis (FUTURE I and FUTURE II), secukinumab was significantly more effective than placebo in improving the signs and symptoms of psoriatic arthritis (Mease PJ. et al. N Engl J Med 2015; 373: 1329-39; McInnes IB. et al. The Lancet; 386:1137-46).

Ixekizumab, a humanized anti-IL-17A monoclonal antibody (U.S. Patent Nos. 7,838,638 and 8,110,191 and WO 2007/070750), was studied in biologic-naive patients with active psoriatic arthritis in a 24-week phase III trial (SPIRIT-PI) (Mease PJ. Et al., Ann Rheum Dis. 2017 Jan;76(1):79-87). It was demonstrated that in biologic-naive patients with active psoriatic arthritis, ixekizumab treatment resulted in improvements in disease activity and physical function, as well as in the inhibition of structural damage progression. Ixekizumab was also shown to be effective in treating patients with moderate-to-severe plaque psoriasis (Griffiths CEM, et al., The Lancet; 386: 541-51).

Brodalumab is a fully human IL-17 receptor (IL-17RA) monoclonal antibody (see U.S. Patent No. 7,767,206), and has been proven effective in the treatment of psoriasis (Papp KA. et al. N Engl J Med 2012; 366: 1181-9). It also showed significant and sustained response in psoriatic arthritis patients in a placebo-controlled phase II study (Mease PJ. et al., N Engl J Med 2014; 370: 2295-306). However, treatment with brodalumab has been coupled to strong adverse events, such as upper respiratory tract infection, fatigue, diarrhea, and reported suicidal thoughts and behavior (see for review Wang et al., Eur J Rheumatol 2017 (4) 272-7).

Accordingly, IL-17A is a promising target in the therapy of inflammatory and autoimmune disorders. Although a number of anti-IL-17A antibodies have been identified up to date, there is still a need for the development of improved therapeutic antibodies being able to effectively reduce or eliminate IL-17A activity in inflammatory responses and autoimmune diseases, and at the same time having improved safety profile and being suitable for development. The therapeutic antibodies should have, in addition to beneficial affinity, efficacy and immunogenicity, improved biophysical properties leading to better developability, producibility in high yields and protein stability.

### SUMMARY OF THE INVENTION

It is an object of the present invention to provide an anti-IL-17A antibody with improved affinity, efficacy and improved biophysical properties, e.g. improved solubility, developability and stability.

Anti-IL-17A antibodies of the present invention have improved properties beneficial for use in therapies, such as higher affinity, improved efficacy, selectivity, improved biophysical properties, such as solubility, developability, and stability.

Therefore, in one aspect, the disclosure provides an isolated antibody having a binding specificity for human IL-17A, in particular which comprises a set of CDRs: HCDR1, HCDR2, HCDR3, LCDR1, LCDR2 and LCDR3, wherein the set of CDRs has 10 or fewer, preferably 0, amino acid substitutions from a set of CDRs in which HCDR1' is amino acid sequence selected from any one of SEQ ID Nos: 1, 4, and 7; HCDR2' is amino acid sequence selected from any one of SEQ ID Nos: 2, 5, and 8; HCDR3' is amino acid sequence selected from any one of SEQ ID Nos: 3, 6, and 9; LCDR1' is amino acid sequence selected from any one of SEQ ID Nos: 12, 15, and 18; LCDR2' is amino acid sequence selected from any one of SEQ ID Nos: 13, 16, and 19; LCDR3' having the amino acid sequence selected from any one of SEQ ID Nos: 14, 17, and 20.In one aspect, the present disclosure relates to a multispecific molecule comprising the isolated antibody of the disclosure.

In one aspect, the present disclosure relates to a pharmaceutical composition comprising the isolated antibody of the disclosure, and a pharmaceutically acceptable carrier.

In another aspect, the present disclosure relates to the antibody of the disclosure , or the composition of the disclosure for use as a medicament.

In one aspect, the present disclosure relates to the antibody of the disclosure, or the composition of the disclosure for use in the treatment of a disorder mediated by IL-17A or a disorder that can be treated by inhibiting Gro-α secretion.

In one aspect, the present disclosure relates to use of the antibody of the disclosure, or the composition of the disclosure in the manufacture of a medicament for use in the treatment of a disorder mediated by IL-17A or a disorder that can be treated by inhibiting Gro-α secretion.

In another aspect, the present disclosure relates to a method of treating a disorder mediated by IL-17A, said method comprising administering an effective amount of the antibody of the disclosure, or the composition of the disclosure to a subject in need thereof.

In yet another aspect, the present disclosure relates to a nucleic acid encoding the antibody of the disclosure. In a further aspect, the present disclosure relates to a vector comprising said nucleic acid. In a further aspect, the present disclosure relates to a host cell comprising said nucleic acid or said vector.

In another aspect, the present disclosure relates to a method of producing the antibody of the disclosure, the method comprising the step of culturing a host cell comprising a nucleic acid or a vector encoding the antibody of the disclosure.

The aspects, advantageous features and preferred embodiments of the present disclosure summarized in the following items, respectively alone or in combination, further contribute to solving the object of the invention:
1. An isolated antibody having a binding specificity for human IL-17A, comprising a set of CDRs: HCDR1, HCDR2, HCDR3, LCDR1, LCDR2 and LCDR3, wherein the set of CDRs has 10 or fewer amino acid substitutions from a set of CDRs in which
   HCDR1' is amino acid sequence selected from any one of SEQ ID Nos: 1, 4, and 7;
   HCDR2' is amino acid sequence selected from any one of SEQ ID Nos: 2, 5, and 8;
   HCDR3' is amino acid sequence selected from any one of SEQ ID Nos: 3, 6, and 9;
   LCDR1' is amino acid sequence selected from any one of SEQ ID Nos: 12, 15, and 18;
   LCDR2' is amino acid sequence selected from any one of SEQ ID Nos: 13, 16, and 19;
   LCDR3' having the amino acid sequence selected from any one of SEQ ID Nos: 14, 17, and 20.
2. The isolated antibody of item 1, comprising a set of CDRs: HCDR1, HCDR2, HCDR3, LCDR1, LCDR2 and LCDR3, wherein the set of CDRs has 10 or fewer amino acid substitutions from a set of CDRs in which
   HCDR1' is as set forth in SEQ ID Nos: 1;
   HCDR2' is as set forth in SEQ ID Nos: 2;
   HCDR3' is as set forth in SEQ ID Nos: 3;
   LCDR1' is as set forth in SEQ ID Nos: 12;
   LCDR2' is as set forth in SEQ ID Nos: 13;
   LCDR3' is as set forth in SEQ ID Nos: 14.
3. The isolated antibody of item 1 or item 2, which comprises a heavy chain variable region (VH) and a light chain variable region (VL), wherein:
   (a) said VH comprises, in sequence, the three complementary determining regions HCDR1, HCDR2 and HCDR3, and
   (b) said VL comprises, in sequence, the three complementary determining regions LCDR1, LCDR2 and LCDR3.
4. The antibody of item 3, wherein
   (a) said HCDR1 is as set forth in the amino acid sequence selected from any one of SEQ ID Nos: 1, 4, and 7;
   (b) said HCDR2 is as set forth in the amino acid sequence selected from any one of SEQ ID Nos: 2, 5, and 8;
   (c) said HCDR3 is as set forth in the amino acid sequence selected from any one of SEQ ID Nos: 3, 6, and 9;
   (d) said LCDR1 is as set forth in the amino acid sequence selected from any one of SEQ ID Nos: 12, 15, and 18;
   (e) said LCDR2 is as set forth in the amino acid sequence selected from any one of SEQ ID Nos: 13, 16, and 19; and
   (f) said LCDR3 is as set forth in the amino acid sequence selected from any one of SEQ ID Nos: 14, 17, and 20.
5. The antibody of item 4, wherein the antibody comprises HCDR1, HCDR2, and HCDR3 sequences of SEQ ID NOs: 1, 2, and 3, respectively, and LCDR1, LCDR2, and LCDR3 sequences of SEQ ID NOs: 12, 13, and 14, respectively.
6. The antibody of anyone of items 3 to 5, wherein said VH is VH3 or VH4, preferably VH3.
7. The antibody of anyone of items 3 to 6, wherein said VL comprises Vκ frameworks FR1, FR2 and FR3, particularly Vκ1 or Vκ3 FR1 to FR3, preferably Vκ1 FR1 to FR3, and a framework FR4, which is selected from a Vκ FR4, particularly Vκ1 FR4, Vκ3 FR4, and Vλ FR4, particularly Vλ FR4 comprising the amino acid sequence having at least 60, 70, 80, 90 percent identity to an amino acid sequence selected from any of SEQ ID NO: 26 to SEQ ID NO: 32, preferably Vλ FR4 as set forth in any of SEQ ID NO: 26 to SEQ ID NO: 32, preferably Vλ FR4 as set forth in SEQ ID NO: 26 or 27, more preferably Vλ FR4 as set forth in SEQ ID NO: 27.
8. The antibody of anyone of items 3 to 7, wherein said VH comprises an amino acid sequence that is at least 90 percent identical to the amino acid sequence SEQ ID NO: 10; and/or said VL comprises an amino acid sequence that is at least 90 percent identical to the amino acid sequence SEQ ID NO: 21.
9. The antibody of item 8, wherein said VH comprises an amino acid sequence selected from the group consisting of SEQ ID NOs: 10 and 11; and/or said VL comprises an amino acid sequence selected from the group consisting of SEQ ID NOs: 21 and 22.
10. The antibody of item 9, comprising a VH sequence of SEQ ID NO: 10 and/or a VL sequence of SEQ ID NO: 21.
11. The antibody of item 9, comprising a VH sequence of SEQ ID NO: 11 and/or a VL sequence of SEQ ID NO: 22.
12. The antibody of any one of the preceding items, wherein the antibody has a binding specificity for cynomolgus monkey IL-17A.
13. The antibody of any one of the preceding items, wherein said antibody selectively binds to human IL-17A over human IL-17B, IL-17C, IL-17D, IL-17E and IL-17F as measured by ELISA.
14. The antibody of any one of the preceding items, wherein binding to IL-17A
   (a) inhibits or blocks binding between IL-17A and its receptor (IL-17RA), and
   (b) reduces or neutralizes IL-17A activity.
15. The antibody of item 14, wherein said antibody is capable of inhibiting Gro-α secretion when assessed *in vitro* in HT-29 assay.
16. The antibody of any of the preceding items, wherein said antibody:
   (a) has the ability to block interaction between IL-17A and IL-17RA with a potency relative to that of secukinumab (relative potency), determined in ELISA assay, greater than 5, preferably greater than 10, more preferably greater than 20, and wherein said relative potency is the ratio of the IC₅₀ value in ng/mL of secukinumab as measured by ELISA to the IC₅₀ value in ng/mL of the antibody of the invention in the scFv format as measured by ELISA; and/or
   (b) has the ability to neutralize IL-17A with a potency relative to that of secukinumab (relative potency), determined by measuring Gro-α secretion in an HT-29 assay, greater than 50, preferably greater than 100, more preferably greater than 150, and wherein said relative potency is the ratio of the IC₅₀ value in ng/mL of secukinumab as measured in the HT-29 assay to the IC₅₀ value in ng/mL of the antibody of the invention in the scFv format as measured in the HT-29 assay;
      and/or
   (c) capable of inhibiting the activity of 1 ng human IL-17A at a concentration of 1 ng/mL or less, preferably 0.5 ng/mL or less, more preferably 0.2 ng/mL or less, by 50%, said inhibitory activity is determined by measuring Gro-α secretion induced by human IL-17A in HT-29 assay in the presence of 50 pg/ml TNFα.
17. The antibody of any of the preceding items, wherein said antibody:
   (a) binds to human IL-17A with a dissociation constant (K_{D}) of less than 5 nM, e.g., less than 1 nM, less than 0.5 nM, less than 0.2 nM, preferably less than 100 pM, more preferably less than 50 pM, as measured by surface plasmon resonance, preferably as measured by surface plasmon resonance in a direct setup; and
   (b) optionally, binds to Cynomolgus IL-17A with a K_{D} of less than 10 nM, e.g., less than 7 nM, less than 5 nM, less than 2 nM, less than 1 nM, preferably less than 0.5 nM as measured by surface plasmon resonance, preferably as measured by surface plasmon resonance in a capture setup.
18. The antibody of item 17, wherein said antibody binds to human IL-17A with a dissociation constant (K_{D}) of less than 100 pM, preferably less than 50 pM as measured by surface plasmon resonance, in particular as measured by surface plasmon resonance in a direct set-up.
19. The antibody of any of the preceding items, wherein said antibody:
   (a) when in scFv format, has a melting temperature (Tm), determined by differential scanning fluorimetry, of at least 60°C, preferably of at least 62°C, more preferably at least 65°C, in particular wherein said antibody is in phosphate-citrate buffer at pH 6.4, 150 mM NaCl;
   (b) when in scFv format, has a loss in monomer content, after five consecutive freeze-thaw cycles, of less than 5%, preferably less than 3%, when the antibody of the invention is at a starting concentration of 10 mg/ml, in particular wherein said antibody is in phosphate buffered saline (PBS), pH 7.4; and/or
   (c) when in scFv format, has a loss in monomer content, after storage for at least two weeks, particularly for at least four weeks, at 4°C, of 5% or less, when the antibody of the invention is at a starting concentration of 10 mg/ml, in particular wherein said antibody is in phosphate buffered saline (PBS), pH 7.4.
20. The antibody of any of the preceding items, wherein the antibody is selected from the group consisting of: a monoclonal antibody, a chimeric antibody, a Fab, an Fv, an scFv, dsFv, an scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. F-star's Modular Antibody Technology™), preferably scFv.
21. The antibody of item 20, wherein said scFv has the amino acid sequence selected from the group consisting of SEQ ID NO:24 and SEQ ID NO: 25, preferably wherein said scFv has the amino acid sequence of SEQ ID NO: 24.
22. The isolated antibody of item 20, wherein the antibody is an IgG selected from the group consisting of an IgG1, an IgG2, an IgG3 and an IgG4, preferably wherein the antibody is an IgG1 or IgG4.
23. The isolated antibody of any of the preceding items, wherein the antibody is humanized.
24. The antibody of any one of items 1 to 23 which is a multispecific molecule.
25. The antibody of item 24, wherein said antibody is in a format selected from the group consisting of a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)₂) or bibody (Fab-(scFv)₁), Fab,, Fab-Fv₂, Morrison (IgG CH₃-scFv fusion (Morrison L) or IgG CL-scFv fusion (Morrison H)), triabody, scDb-scFv, bispecific Fab₂, di-miniantibody, tetrabody, scFv-Fc-scFv fusion, scFv-HSA-scFv fusion, di-diabody, DVD-Ig, COVD, IgG-scFab, scFab-dsscFv, Fv₂-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), TslAb (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs); an Fv, scFv, scDb, tandem-di-scFv, tandem tri-scFv, Fab-(scFv)₂, Fab-(scFv)₁, Fab, Fab-Fv₂, COVD fused to the N- and/or the C-terminus of either chain of a heterodimeric Fc domain or any other heterodimerization domain, a MATCH and DuoBodies.
26. A pharmaceutical composition comprising the antibody of any one of items 1 to 25, and a pharmaceutically acceptable carrier.
27. The antibody of any one of items 1 to 25, or the composition of item 26 for use as a medicament.
28. The antibody of any one of items 1 to 25, or the composition of item 26 for use in the treatment of a disorder mediated by IL-17A or a disorder that can be treated by inhibiting Gro-α secretion.
29. The antibody of any one of items 1 to 25, or the composition of item 26 for use in the treatment of an inflammatory condition or an autoimmune disease.
30. The antibody of any one of items 1 to 25or the composition of item 26 for use in the treatment of a cancer, arthritis, rheumatoid arthritis, osteoarthritis, reactive arthritis, psoriasis, chronic obstructive pulmonary disease, systemic lupus erythematosus (SLE), lupus nephritis, an autoimmune inflammatory bowel disease, asthma, multiple sclerosis, or cystic fibrosis, bone loss, airways hypersensitivity, a demyelinating disorder, dermal hypersensitivity, acute transplant rejection, allograft rejection, graft-versus host disease, systemic sclerosis, an urological inflammatory disorder, a cardiovascular disease, vasculitis, a periodic fever, a glucose metabolism disorder, a pulmonary disease, peridontitis, hepatic stromal keratitis, an allergy, inflammatory pain, a spondyloarthropathy, septicaemia, septic or endotoxic shock, meningitis, surgical trauma, an autoimmune haematological disorder, Alzheimer's disease, sarcoidosis, cirrhosis, hepatitis, glomerulonephritis or dislipidemia.
31. Use of the antibody of any one of items 1 to 25, or the composition of item 26 in the manufacture of a medicament for use in the treatment of a disorder mediated by IL-17A or a disorder that can be treated by inhibiting Gro-α secretion.
32. Use of the antibody of any one of items 1 to 25, or the composition of item 26 in the manufacture of a medicament for use in the treatment of an inflammatory condition or an autoimmune disease.
33. Use of the antibody of any one of items 1 to 25, or the composition of item 26 in the manufacture of a medicament for use in the treatment of a cancer, arthritis, rheumatoid arthritis, osteoarthritis, reactive arthritis, psoriasis, chronic obstructive pulmonary disease, systemic lupus erythematosus (SLE), lupus nephritis, an autoimmune inflammatory bowel disease, asthma, multiple sclerosis, cystic fibrosis, bone loss, airways hypersensitivity, a demyelinating disorder, dermal hypersensitivity, acute transplant rejection, allograft rejection, graft-versus host disease, systemic sclerosis, an urological inflammatory disorder, a cardiovascular disease, vasculitis, a periodic fever, a glucose metabolism disorder, a pulmonary disease, peridontitis, hepatic stromal keratitis, an allergy, inflammatory pain, a spondyloarthropathy, septicaemia, septic or endotoxic shock, meningitis, surgical trauma, an autoimmune haematological disorder, Alzheimer's disease, sarcoidosis, cirrhosis, hepatitis, glomerulonephritis or dislipidemia.
34. A method of treating a disorder mediated by IL-17A, said method comprising administering an effective amount of the antibody of any one of items 1 to 25, or the composition of item 26, such that the condition is alleviated.
35. The method according to item 34, wherein the disorder mediated by IL-17A is inflammatory condition or an autoimmune disease.
36. The method according to item 34, wherein the disorder mediated by IL-17A is a cancer, arthritis, rheumatoid arthritis, osteoarthritis, reactive arthritis, psoriasis, chronic obstructive pulmonary disease, systemic lupus erythematosus (SLE), lupus nephritis, an autoimmune inflammatory bowel disease, asthma, multiple sclerosis, cystic fibrosis, bone loss, airways hypersensitivity, a demyelinating disorder, dermal hypersensitivity, acute transplant rejection, allograft rejection, graft-versus host disease, systemic sclerosis, an urological inflammatory disorder, a cardiovascular disease, vasculitis, a periodic fever, a glucose metabolism disorder, a pulmonary disease, peridontitis, hepatic stromal keratitis, an allergy, inflammatory pain, a spondyloarthropathy, septicaemia, septic or endotoxic shock, meningitis, surgical trauma, an autoimmune haematological disorder, Alzheimer's disease, sarcoidosis, cirrhosis, hepatitis, glomerulonephritis or dislipidemia.
37. A nucleic acid encoding the antibody of items 1-25.
38. A vector comprising the nucleic acid of item 37.
39. A host cell comprising the nucleic acid of item 37 or the vector of item 38.
40. A method of producing the antibody of items 1- 25, the method comprising the step of culturing a host cell comprising a nucleic acid or a vector encoding the antibody of items 1-25.
41. A kit comprising the antibody of any one of items 1 to 25, or the composition of item 26.

The disclosure contemplates all combinations of any one or more of the foregoing aspects and/or embodiments, as well as combinations with any one or more of the embodiments set forth in the detailed description and examples.

Other features, objects, and advantages of the compositions and methods herein will be apparent from the description and drawings, and from the claims.

### BRIEF DESCRIPTION OF THE DRAWINGS

**FIG. 1** Labeled IL-7A is suitable for use in the sorting process. Biological activity of labeled IL-7A in the HT-29 assay. 3-fold serial dilutions of labeled and un-labeled IL-7A were tested in parallel for their potential to induce GROα secretion in HT-29 cells. EC₅₀ values for labeled (IL-7A-RPE) and unlabeled (IL-7A) IL-7A are 82.8 ng/ml and 55 ng/ml respectively.
**FIG. 2** Potency of 27-31-C04 rabbit IgG to neutralize IL-17A in the HT-29 assay.
**FIG. 3** Potency of 27-31-C04 rabbit IgG to inhibit the interaction between IL-17A and IL-7RA.
**FIG. 4** Potencies of the humanized scFvs PRO571 and PRO592 to neutralize IL-17A in the HT-29 assay.
**FIG. 5** Potencies of anti-IL-17A scFv PRO571 and PRO592 to inhibit the interaction between human IL-17A and IL-17RA (ELISA).
**FIG. 6** Target specificity of the scFvs PRO571 and PRO592 is shown. The potential to inhibit the interaction of biotinylated IL-17A with the scFvs by IL-17B to IL-17F was analyzed by competition ELISA. Dose-dependent effects of IL-17A and IL-17B to IL-17F are shown.
**FIG. 7** Thermal unfolding curves from DSF measurements of the scFv PRO571 and PRO592.
The resulting Tm values have been determined by fitting the data to a Boltzmann equation to obtain the midpoint of transition.
**FIG. 8** Storage stability study of the scFvs PRO571 and PRO592 performed at a concentration of >10 mg/mL for 4 weeks at three temperatures (37°C, 4°C and -80°C).
Monomeric content over time at different storage temperatures is shown on the left; protein concentration at different storage temperatures over the course of time is shown on the right.
Monomeric content was determined by integration of SE-HPLC peak areas and protein concentrations were calculated by UV₂₈₀ measurement.

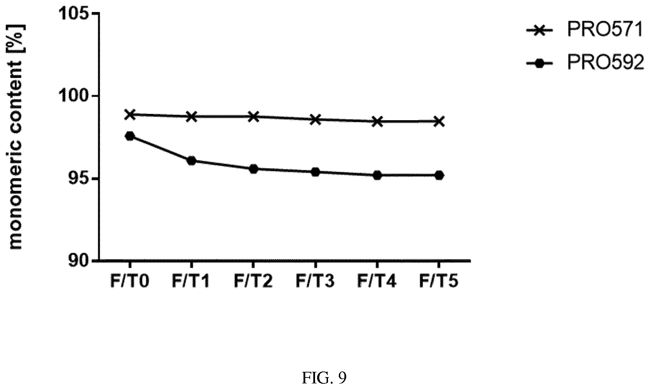

**FIG. 9** Monitoring of monomeric content of the scFvs PRO571 and PRO592 over 5 repeated freezing and thawing cycles.

### DETAILED DESCRIPTION OF THE INVENTION

The present disclosure provides antibodies that specifically bind to human IL-17A protein, and pharmaceutical compositions, production methods, and methods of use of such antibodies and compositions.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

### Antibodies of the disclosure

The present disclosure is based on the discovery of antibody molecules that specifically bind to human IL-17A and have improved affinity, efficacy, and selectivity. Moreover, the antibody of the present disclosure has improved biophysical properties, e.g. improved solubility, developability and producibility in high with relatively low impurities (> 98% monomer as detected by SE-HPLC), and stability.

In one aspect, the disclosure provides an isolated antibody having a binding specificity for human IL-17A, comprising a set of CDRs: HCDR1, HCDR2, HCDR3, LCDR1, LCDR2 and LCDR3, wherein the set of CDRs has 10 or fewer amino acid substitutions, e.g., 9 or fewer amino acid substitutions, 8 or fewer amino acid substitutions, 7 or fewer amino acid substitutions, 6 or fewer amino acid substitutions, 5 or fewer amino acid substitutions, 4 or fewer amino acid substitutions, 3 or fewer amino acid substitutions, 2 or fewer amino acid substitutions, 1 or 0 amino acid substitutions, preferably 0 amino acid substitutions, from a set of CDRs in which HCDR1' is amino acid sequence selected from any one of SEQ ID Nos: 1, 4, and 7, preferably SEQ ID NO: 1; HCDR2' is amino acid sequence selected from any one of SEQ ID Nos: 2, 5, and 8, preferably SEQ ID NO: 2; HCDR3' is amino acid sequence selected from any one of SEQ ID Nos: 3, 6, and 9, preferably SEQ ID NO: 3; LCDR1' is amino acid sequence selected from any one of SEQ ID Nos: 12, 15, and 18, preferably SEQ ID NO: 12; LCDR2' is amino acid sequence selected from any one of SEQ ID Nos: 13, 16, and 19, preferably SEQ ID NO: 13; LCDR3' having the amino acid sequence selected from any one of SEQ ID Nos: 14, 17, and 20, preferably SEQ ID NO: 14.

The term "IL-17A" refers in particular to human IL-17A with UniProt ID number Q16552 reproduced herein as SEQ ID NO: 33.The term "cynomolgus IL-17A" or "cynomolgus monkey IL-17A" refers to Macaca fascicularis IL-17A with UniProt ID number G1QUS7.

The term "IL-17B" refers in particular to human IL-17B with UniProt ID number Q9UHF5 reproduced herein as SEQ ID NO: 34. The term "IL-17C" refers in particular to human IL-17C with UniProt ID number Q9P0M4 reproduced herein as SEQ ID NO: 35. The term "IL-17D" refers in particular to human IL-17D with UniProt ID number Q8TAD2 reproduced herein as SEQ ID NO: 36. The term "IL-17E" refers in particular to human IL-17E with UniProt ID number Q9H293 reproduced herein as SEQ ID NO: 37. The term "IL-17F" refers in particular to human IL-17F with UniProt ID number Q96PD4 reproduced herein as SEQ ID NO: 38.

The term "epitope" refers to a localized region of an antigen to which an antibody can specifically bind. An epitope can be, for example, contiguous amino acids of a polypeptide, or an epitope can, for example, come together from two or more non-contiguous regions of a polypeptide or polypeptides.

The term "antibody" and the like, as used herein, includes: whole antibodies; any antigen-binding fragments (i.e., "antigen-binding portions") or single chains of whole antibodies; and molecules comprising antibody CDRs, VH regions or VL regions (including without limitation multispecific antibodies). A naturally occurring "whole antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino terminus to carboxy terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "isotype", as used herein, refers to the antibody class (e.g., IgM, IgE, IgD, IgA, IgY, and IgG, such as IgG1 or IgG4) that is provided by the heavy chain constant region genes. Isotype also includes modifies versions of one of these classes, where modifications have been made to alter the Fc function, for example, to enhance or reduce effector functions or binding to Fc receptors. Suitably, the antibody of the disclosure is an IgG selected from the group consisting of an IgG1, an IgG2, an IgG3 and an IgG4. More suitably, the antibody of the disclosure is an IgG1 or IgG4.

The terms "antigen-binding fragment", "antigen binding portion", and the like, as used herein, refer to one or more fragments of an intact whole antibody that retain the ability to specifically bind to a given antigen (e.g., IL-17A). Examples of binding fragments encompassed within the term "antigen binding portion" of an antibody include a Fab fragment, a monovalent fragment consisting of the VL, VH, CL and CH1 domains; a F(ab)2 fragment, a bivalent fragment comprising two Fab fragments linked by a disulfide bridge at the hinge region; an Fd fragment consisting of the VH and CH1 domains; an Fv fragment consisting of the VL and VH domains of a single arm of an antibody; a single domain antibody (dAb) fragment (Ward et al., 1989 Nature 341:544-546), which consists of a VH domain; an isolated complementarity determining region (CDR), dsFv, an scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. F-star's Modular Antibody Technology™).

The term "Complementarity Determining Regions" ("CDRs") are amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273, 927-948 ("Chothia" numbering scheme), ImMunoGenTics (IMGT) numbering (Lefranc, M.-P., The Immunologist, 7, 132-136 (1999); Lefranc, M.-P. et al., Dev. Comp. Immunol., 27, 55-77 (2003) ("IMGT" numbering scheme) and numbering scheme described in Honegger & Plückthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present disclosure, the numbering system suggested by Honegger & Plückthun ("AHo") is used (Honegger & Plückthun, J. Mol. Biol. 309 (2001) 657-670), unless specifically mentioned otherwise. Furthermore, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H27-H42; HCDR2 (also referred to as CDR-H2): H57-H76; HCDR3 (also referred to as CDR-H3): H108-H138. For the sake of clarity, the numbering system according to Honegger & Plückthun takes the length diversity into account that is found in naturally occurring antibodies, both in the different VH and VL subfamilies and, in particular, in the CDRs, and provides for gaps in the sequences. Thus, in a given antibody variable domain usually not all positions 1 to 149 will be occupied by an amino acid residue.

Preferably, the "antigen-binding region" comprises at least amino acid residues 4 to 138 of the variable light (VL) chain and 5 to 138 of the variable heavy (VH) chain (in each case numbering according to Honegger & Plückthun), more preferably amino acid residues 3 to 144 of VL and 4 to 144 of VH, and particularly preferred are the complete VL and VH chains (amino acid positions 1 to 149 of VL and 1 to 149 of VH). Antigen-binding portions can also be incorporated into single domain antibodies, maxibodies, minibodies, intrabodies, diabodies, triabodies, tetrabodies, v-NAR and bis-scFv (see, e.g., Hollinger and Hudson, 2005, Nature Biotechnology, 23, 9, 1 Hel 136). Antigen binding portions of antibodies can be grafted into scaffolds based on polypeptides such as Fibronectin type III (Fn3) (see U.S. Pat. No. 6,703,199, which describes fibronectin polypeptide monobodies). Antigen binding portions can be incorporated into single chain molecules comprising a pair of tandem Fv segments (VH-CH1-VH-CH1) which, together with complementary light chain polypeptides, form a pair of antigen binding regions (Zapata et al., 1995 Protein Eng. 8 (10): 1057-1062; and U.S. Pat. No. 5,641,870).

The term "binding specificity", as used herein, refers to the ability of an individual antibody to react with one antigenic determinant and not with a different antigenic determinant. As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" is referring to the ability of the antibody to discriminate between the target of interest and an unrelated molecule, as determined, for example, in accordance with a specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard colour development (e.g. secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, preferably at least 100-fold difference between a background and signal indicates on specific binding. Typically, determination of binding specificity is performed by using not a single reference molecule, but a set of about three to five unrelated molecules, such as milk powder, transferrin or the like. The antibody of the disclosure has a binding specificity for human IL-17A.

In one embodiment, the antibody of the disclosure has a binding specificity for human IL-17A and Macaca fascicularis (also known as Cynomolgus monkey or "Cynomolgus") IL-17A.

Suitably, the antibody of the disclosure is an isolated antibody. The term "isolated antibody", as used herein, refers to an antibody that is substantially free of other antibodies having different antigenic specificities (e.g., an isolated antibody that specifically binds IL-17A is substantially free of antibodies that specifically bind antigens other than IL-17A). An isolated antibody that specifically binds IL-17A may, however, have cross-reactivity to other antigens, such IL-17A molecules from other species. Moreover, an isolated antibody may be substantially free of other cellular material and/or chemicals.

Suitably, the antibody of the disclosure is a monoclonal antibody. The term "monoclonal antibody" or "monoclonal antibody composition" as used herein refers to antibodies that are substantially identical to amino acid sequence or are derived from the same genetic source. A monoclonal antibody composition displays a binding specificity and affinity for a particular epitope, or binding specificities and affinities for specific epitopes.

Antibodies of the disclosure include, but are not limited to, the chimeric, and humanized.

The term "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

A "humanized" antibody, as used herein, is an antibody that retains the reactivity of a non-human antibody while being less immunogenic in humans. This can be achieved, for instance, by retaining the non-human CDRs and replacing the remaining parts of the antibody with their human counterparts (i.e., the constant region as well as the framework portions of the variable region). Additional framework region modifications may be made within the human framework sequences as well as within the CDR sequences derived from the germline of another mammalian species. The humanized antibodies of the disclosure may include amino acid residues not encoded by human sequences (e.g., mutations introduced by random or site-specific mutagenesis in vitro or by somatic mutation in vivo, or a conservative substitution to promote stability or manufacturing). See, e.g., Morrison et al., Proc. Natl. Acad. Sci. U.S.A, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include, but is not limited to Xoma technology disclosed in U.S. Pat. No. 5,766,886.

The term "recombinant humanized antibody", as used herein, includes all humanized antibodies of the disclosure that are prepared, expressed, created or isolated by recombinant means, such as antibodies isolated from an animal (e.g. a rabbit); antibodies expressed using a recombinant expression vector transfected into a host cell, antibodies isolated from a recombinant, combinatorial human antibody library, or antibodies prepared, expressed, created or isolated by any other means that involves splicing of human immunoglobulin gene sequences to other DNA sequences.

Suitably, the antibody of the disclosure is humanized. Suitably, the antibody of the disclosure is humanized and comprises rabbit-derived CDRs.

Antibodies of the disclosure include, but are not limited to, the humanized monoclonal antibodies isolated as described herein, including in the Examples. Examples of such antihuman IL-17A antibodies are antibodies whose sequences are listed in Table 1. Additional details regarding the generation and characterization of the antibodies described herein are provided in the Examples.

The isolated antibody of the disclosure having a binding specificity for human IL-17A comprises a heavy chain variable region (VH) and a light chain variable region (VL), wherein: (a) said VH comprises, in sequence, the three complementary determining regions HCDR1, HCDR2 and HCDR3, and (b) said VL comprises, in sequence, the three complementary determining regions LCDR1, LCDR2 and LCDR3.

The present disclosure provides antibodies that specifically bind to IL-17A protein, said antibodies comprising a VH CDR having an amino acid sequence of any one of the VH CDRs listed in Table 1. In particular, the disclosure provides antibodies that specifically bind to IL-17A protein, said antibodies comprising one, two, three, or more VH CDRs having an amino acid sequence of any of the VH CDRs listed in Table 1.

The present disclosure provides an isolated antibody having a binding specificity for human IL-17A, which comprises a heavy chain variable region (VH), wherein said VH comprises, in sequence, the three complementary determining regions HCDR1, HCDR2 and HCDR3, said HCDR1 having the amino acid sequence selected from any one of SEQ ID Nos: 1, 4, and 7, said HCDR2 having the amino acid sequence selected from any one of SEQ ID Nos: 2, 5, and 8, said HCDR3 having the amino acid sequence selected from any one of SEQ ID Nos: 3, 6, and 9. In particular, the disclosure provides antibodies that have a binding specificity for human IL-17A and comprises HCDR1, HCDR2, and HCDR3 sequences of SEQ ID NOs: 1, 2, and 3, respectively.

The present disclosure also provides antibodies that specifically bind to IL-17A protein, said antibodies comprising a VL CDR having an amino acid sequence of any one of the VL CDRs listed in Table 1. In particular, the disclosure provides antibodies that specifically bind to IL-17A protein, said antibodies comprising one, two, three or more VL CDRs having an amino acid sequence of any of the VL CDRs listed in Table 1.

The present disclosure provides an isolated antibody having a binding specificity for human IL-17A, which comprises a light chain variable region (VL), wherein said VL comprises, in sequence, the three complementary determining regions LCDR1, LCDR2 and LCDR3, said LCDR1 having the amino acid sequence selected from any one of SEQ ID Nos: 12, 15, and 18, said LCDR2 having the amino acid sequence selected from any one of SEQ ID Nos: 13, 16, and 19, said LCDR3 having the amino acid sequence selected from any one of SEQ ID Nos: 14, 17, and 20. In particular, the disclosure provides antibodies that have a binding specificity for human IL-17A and comprises LCDR1, LCDR2, and LCDR3 sequences of SEQ ID NOs: 12, 13, and 14, respectively.

Suitably, the present disclosure provides an isolated antibody having a binding specificity for human IL-17A, which comprises a heavy chain variable region (VH) and a light chain variable region (VL), wherein:
(a) said VH comprises, in sequence, the three complementary determining regions HCDR1, HCDR2 and HCDR3, said HCDR1 having the amino acid sequence selected from any one of SEQ ID Nos: 1, 4, and 7, said HCDR2 having the amino acid sequence selected from any one of SEQ ID Nos: 2, 5, and 8, said HCDR3 having the amino acid sequence selected from any one of SEQ ID Nos: 3, 6, and 9; and
(b)said VL comprises, in sequence, the three complementary determining regions LCDR1, LCDR2 and LCDR3, said LCDR1 having the amino acid sequence selected from any one of SEQ ID Nos: 12, 15, and 18, said LCDR2 having the amino acid sequence selected from any one of SEQ ID Nos: 13, 16, and 19, said LCDR3 having the amino acid sequence selected from any one of SEQ ID Nos: 14, 17, and 20.

In particular, the disclosure provides antibodies that have a binding specificity for human IL-17A and comprises (a) HCDR1, HCDR2, and HCDR3 sequences of SEQ ID NOs: 1, 2, and 3, respectively, and (b) LCDR1, LCDR2, and LCDR3 sequences of SEQ ID NOs: 12, 13, and 14, respectively.

Other antibodies of the disclosure include amino acids that have been mutated, yet have at least 60, 70, 80, 90, 91, 92, 93, 94, 95, 96, 97, 98 or 99 percent identity in the CDR regions with the CDR regions depicted in the sequences described in Table 1. Suitably, other antibodies of the disclosure includes mutant amino acid sequences wherein no more than 1, 2, 3, 4, 5 or 10 amino acids have been mutated by amino acid deletion, insertion or substitution in the CDR regions when compared with the CDR regions depicted in the sequences described in Table 1. Mutations, e.g., substitutions, may potentially be made at any residue within the set of CDRs, and may be within CDR1, CDR2 and/or CDR3.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, gamma-carboxyglutamate, and O-phosphoserine. The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

The techniques required to make mutations, e.g., substitutions, within amino acid sequences of CDRs, antibody VH or VL domains and antibodies generally are available in the art. Variant sequences may be made, with mutations, e.g., substitutions, that may or may not be predicted to have a minimal or beneficial effect on activity, and tested for ability to bind and/or neutralize Il-17A and/or for any other desired property. Suitable mutations, e.g., substitutions, within CDRs do not result in loss of function, so an antibody of the disclosure comprising a thus-mutated amino acid sequence retains an ability to bind and/or neutralize IL-17A. For example, it may retain the same quantitative binding and/or neutralizing ability as an antibody of the disclosure in which the alteration is not made, e.g., as measured in the assays described herein. Suitably, an antibody of the disclosure comprising a thus-mutated amino acid sequence may have an improved ability to bind and/or neutralize IL-17A. Suitably, an antibody of the disclosure comprising a thus-mutated amino acid sequence is capable of inhibiting the activity of 1 ng human IL-17A at a concentration of 50 ng/ml, preferably 20 ng/ml, preferably 10 ng/ml, preferably 5 ng/ml more preferably 1 ng/ml, more preferably 0.5 ng/ml, even more preferably 0.2 ng/ml or less of said antibody by 50%, said inhibitory activity is determined by measuring Gro-α secretion induced by human IL-17A in HT-29 assay in the presence of 50 pg/ml TNFα.

In addition, suitable mutations, e.g., substitutions, within CDRs do not result in loss in solubility, stability, and producibility in high yields of an antibody of the disclosure, so an antibody of the disclosure comprising a thus-mutated amino acid sequence retains the biophysical characteristics. For example, it may retain the same producibility in high yields and/or stability as an antibody of the disclosure in which the alteration is not made, e.g., as measured in the assays described herein. Suitably, an antibody of the disclosure comprising a thus-mutated amino acid sequence may have improved biophysical characteristics.

The terms "identical" or percent "identity", in the context of two or more nucleic acids or polypeptide sequences, refer to two or more sequences or subsequences that are the same. "Percent (%) amino acid sequence identity" and "homology" with respect to nucleic acid, a peptide, polypeptide or antibody sequence are defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2 or ALIGN software. Those skilled in the art can determine appropriate parameters for measuring alignment, including any algorithms needed to achieve maximal alignment over the full length of the sequences being compared.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

Two examples of algorithms that are suitable for determining percent sequence identity and sequence similarity are the BLAST and BLAST 2.0 algorithms, which are described in Altschul et al., Nucleic Acids Res. 25:3389-3402, 1977; and Altschul et al., J. Mol. Biol. 215:403-410, 1990, respectively. Software for performing BLAST analyses is publicly available through the National Center for Biotechnology Information.

The percent identity between two amino acid sequences can also be determined using the algorithm of E. Meyers and W. Miller (Comput. Appl. Biosci., 4: 11-17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453, 1970) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Suitably, the isolated antibody of the disclosure having a binding specificity for human IL-17A comprises: a heavy chain variable region (VH) and a light chain variable region (VL), wherein:
(a) said VH comprises, in sequence, the three complementary determining regions HCDR1, HCDR2 and HCDR3,
   said HCDR1 having the amino acid sequence having at least 60, 70, 80, 90, 91, 92, 93, 94, 95, 96, 97, 98 or 99 percent identity to any one of SEQ ID NOs: 1, 4, and 7, preferably SEQ ID NO: 1;
   said HCDR2 having the amino acid sequence having at least 60, 70, 80, 90, 91, 92, 93, 94, 95, 96, 97, 98 or 99 percent identity to any one of SEQ ID NOs: 2, 5, and 8, preferably SEQ ID NO: 2;
   said HCDR3 having the amino acid sequence having at least 60, 70, 80, 90, 91, 92, 93, 94, 95, 96, 97, 98 or 99 percent identity to any one of SEQ ID NOs: 3, 6, and 9, preferably SEQ ID NO: 3; and/or
(b) said VL comprises, in sequence, the three complementary determining regions LCDR1, LCDR2 and LCDR3,
   said LCDR1 having the amino acid sequence having at least 60, 70, 80, 90, 91, 92, 93, 94, 95, 96, 97, 98 or 99 percent identity to any one of SEQ ID NOs: 12, 15, and 18, preferably SEQ ID NO: 12;
   said LCDR2 having the amino acid sequence having at least 60, 70, 80, 90, 91, 92, 93, 94, 95, 96, 97, 98 or 99 percent identity to any one of SEQ ID NOs: 13, 16, and 19, preferably SEQ ID NO: 13;
   said LCDR3 having the amino acid sequence having at least 60, 70, 80, 90, 91, 92, 93, 94, 95, 96, 97, 98 or 99 percent identity to any one of SEQ ID NOs: 14, 17, and 20, preferably SEQ ID NO: 14.

Suitably, the isolated antibody of the disclosure having a binding specificity for human IL-17A comprises: HCDR1, HCDR2, and HCDR3 having at least 60, 70, 80, 90, 91, 92, 93, 94, 95, 96, 97, 98 or 99 percent identity to sequences of SEQ ID NOs: 1, 2, and 3, respectively, and/or LCDR1, LCDR2, and LCDR3 having at least 60, 70, 80, 90, 91, 92, 93, 94, 95, 96, 97, 98 or 99 percent identity to sequences of SEQ ID NOs: 12, 13, and 14, respectively.

In a further embodiment, the disclosure provides an isolated antibody that specifically binds human IL-17A, wherein said antibody comprises a VH domain and a VL domain. In the context of the present disclosure the terms "VH" (variable heavy chain), "VL" (variable light chain), "Vκ" and "Vλ" refer to families of antibody heavy and light chain sequences that are grouped according to sequence identity and homology. Methods for the determination of sequence homologies, for example by using a homology search matrix such as BLOSUM (Henikoff, S. & Henikoff, J. G., Proc. Natl. Acad. Sci. U.S.A 89 (1992) 10915-10919), and methods for the grouping of sequences according to homologies are well known to one of ordinary skill in the art. For VH, Vκ and Vλ different subfamilies can be identified, as shown, for example, in Knappik et al., J. Mol. Biol. 296 (2000) 57-86, which groups VH in VH1A, VH1B and VH2 to VH6, Vκ in Vκ1 to Vκ4 and Vλ in Vλ1 to Yλ3. In vivo, antibody Vκ chains, Vλ chains, and VH chains are the result of the random rearrangement of germline κ chain V and J segments, germline λ chain V and J segments, and heavy chain V, D and J segments, respectively. To which subfamily a given antibody variable chain belongs is determined by the corresponding V segment, and in particular by the framework regions FR1 to FR3. Thus, any VH sequence that is characterized in the present application by a particular set of framework regions HFR1 to HFR3 only, may be combined with any HFR4 sequence, for example a HFR4 sequence taken from one of the heavy chain germline J segments, or a HFR4 sequence taken from a rearranged VH sequence.

Suitably, the present disclosure provides an isolated that specifically binds human IL-17A, wherein said antibody comprises a VH1A, VH1B, VH3 or VH4. In one embodiment, an isolated antibody of the present disclosure comprises VH4 domain. In a preferred embodiment, an isolated antibody of the present disclosure comprises VH3 domain.

Suitably, the present disclosure provides an isolated antibody that specifically binds human IL-17A, wherein said antibody comprises Vκ frameworks FR1, FR2 and FR3, particularly Vκ1 or Vκ3 FR1 to FR3, preferably Vκ1 frameworks FR1 to FR3, and a framework FR4, which is selected from a Vκ FR4, particularly Vκ1 FR4, Vκ3 FR4, and a Vλ FR4. Suitable Vλ FR4 are as set forth in SEQ ID NO: 26 to SEQ ID NO: 32. In one embodiment the present disclosure provides an isolated antibody that specifically binds human IL-17A, wherein said antibody comprises Vλ FR4 comprising the amino acid sequence having at least 60, 70, 80, 90 percent identity to an amino acid sequence selected from any of SEQ ID NO: 26 to SEQ ID NO: 32, preferably to SEQ ID NO: 26 or SEQ ID NO:27, more preferably to SEQ ID NO: 27. Suitably, the present disclosure provides an isolated antibody that specifically binds human IL-17A, wherein said antibody comprises Vλ FR4 comprising the amino acid sequence selected from any of SEQ ID NO: 26 to SEQ ID NO: 32, preferably Vλ FR4 as set forth in SEQ ID NO: 26 or SEQ ID NO:27, more preferably Vλ FR4 as set forth in SEQ ID NO: 27.

Thus, in one embodiment, the disclosure provides an antibody comprising:
(i) the HCDR1, HCDR2, and HCDR3 sequences of SEQ ID NOs: 1, 2, and 3, respectively, and the LCDR1, LCDR2, and LCDR3 sequences of SEQ ID NOs: 12, 13, and 14, respectively;
(ii) a VH3 or VH4 domain, preferably VH3 domain; and
(iii) a VL domain comprising a VL framework comprising Vκ frameworks FR1, FR2 and FR3, particularly Vκ1 or Vκ3 FR1 to FR3, preferably Vκ1 FR1 to FR3, and a framework FR4, which is selected from a Vκ FR4, particularly Vκ1 FR4, Vκ3 FR4, and a Vλ FR4, particularly Vλ FR4 comprising the amino acid sequence having at least 60, 70, 80, 90 percent identity to an amino acid sequence selected from any of SEQ ID NO: 26 to SEQ ID NO: 32, preferably Vλ FR4 as set forth in amino acid sequence selected from any one of SEQ ID NO: 26 to SEQ ID NO: 32, more preferably Vλ FR4 as set forth in SEQ ID NO: 27.

Suitably, the present disclosure provides an isolated antibody that specifically binds human IL-17A, wherein said antibody comprises a VH domain listed in Table 1.

Suitably, the present disclosure also provides an isolated antibody that specifically binds human IL-17A, wherein said antibody comprises a VH amino acid sequence listed in Table 1, wherein no more than about 10 amino acids in a framework sequence (for example, a sequence which is not a CDR) have been mutated (wherein a mutation is, as various non-limiting examples, an addition, substitution or deletion).

Suitably, the present disclosure also provides an isolated antibody that specifically binds human IL-17A, wherein said antibody comprises a VH amino acid sequence listed in Table 1, wherein no more than about 20 amino acids in a framework sequence (for example, a sequence which is not a CDR) have been mutated (wherein a mutation is, as various non-limiting examples, an addition, substitution or deletion).

Other antibodies of the disclosure include amino acids that have been mutated, yet have at least 60, 70, 80, 90, 91, 92, 93, 94, 95, 96, 97, 98 or 99 percent identity in the VH regions with the VH regions depicted in the sequences described in Table 1.

Suitably, the present disclosure provides an isolated antibody that specifically binds human IL-17A, wherein said antibody comprises a VL domain listed in Table 1.

Suitably, the present disclosure also provides an isolated antibody that specifically binds human IL-17A, wherein said antibody comprises a VL amino acid sequence listed in Table 1, wherein no more than about 10 amino acids in a framework sequence (for example, a sequence which is not a CDR) have been mutated (wherein a mutation is, as various non-limiting examples, an addition, substitution or deletion).

Suitably, the present disclosure also provides an isolated antibody that specifically binds human IL-17A, wherein said antibody comprises a VL amino acid sequence listed in Table 1, wherein no more than about 20 amino acids in a framework sequence (for example, a sequence which is not a CDR) have been mutated (wherein a mutation is, as various non-limiting examples, an addition, substitution or deletion).

Other antibodies of the disclosure include amino acids that have been mutated, yet have at least 60, 70, 80, 90, 91, 92, 93, 94, 95, 96, 97, 98 or 99 percent identity in the VL regions with the VL regions depicted in the sequences described in Table 1.

In one embodiment, the disclosure provides an isolated antibody that specifically binds human IL-17A wherein said antibody comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 10 or SEQ ID NO: 11, preferably SEQ ID NO: 10, and in particular wherein said antibody comprises HCDR1, HCDR2, and HCDR3 sequences of SEQ ID NOs: 1, 2, and 3, respectively. In a further embodiment, the disclosure provides an isolated antibody that specifically binds human IL-17A wherein said antibody comprises a heavy chain variable region comprising an amino acid sequence that is at least 90 percent identical to the amino acid sequence SEQ ID NO: 11 and wherein said heavy chain variable region comprises R20T and Q141P (AHo numbering).

In another embodiment, the disclosure provides an isolated antibody that specifically binds human IL-17A wherein said antibody comprises a light chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 21 or SEQ ID NO: 22, preferably SEQ ID NO: 21, and in particular wherein said antibody comprises LCDR1, LCDR2, and LCDR3 sequences of SEQ ID NOs: 12, 13, and 14, respectively.

In a further embodiment, the disclosure provides an isolated that specifically binds human IL-17A wherein said antibody comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 10; and a light chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 21.

Thus, the present disclosure provides an isolated antibody that specifically binds human IL-17A, wherein said antibody comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 10; and a light chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 21, and wherein the antibody comprises HCDR1, HCDR2, and HCDR3 sequences of SEQ ID NOs: 1, 2, and 3, respectively, and/or LCDR1, LCDR2, and LCDR3 sequences of SEQ ID NOs: 12, 13, and 14, respectively.

In a further embodiment, the disclosure provides an isolated antibody that specifically binds human IL-17A wherein said antibody comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 11; and a light chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 22, particularly wherein said heavy chain variable region comprises R20T and Q141P (AHo numbering).

Thus, the present disclosure provides an isolated antibody that specifically binds human IL-17A, wherein said antibody comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 11; and a light chain variable region comprising an amino acid sequence that is at least 60, 70, 80, 90, 91, 92, 93, 94, 95, 96, 97, 98 or 99 percent, preferably at least 90 percent, identical to the amino acid sequence SEQ ID NO: 22, particularly wherein said heavy chain variable region comprises R20T and Q141P (AHo numbering), and wherein the antibody comprises HCDR1, HCDR2, and HCDR3 sequences of SEQ ID NOs: 1, 2, and 3, respectively, and/or LCDR1, LCDR2, and LCDR3 sequences of SEQ ID NOs: 12, 13, and 14, respectively.

In a specific embodiment, the disclosure provides an isolated antibody that specifically binds human IL-17A and comprises a VH comprising an amino acid sequence selected from the group consisting of SEQ ID NOs: 10 and 11; and/or a VL thereof comprising an amino acid sequence selected from the group consisting of SEQ ID NOs: 21 and 22. In particular embodiment, the antibody of the present disclosure comprises a VH sequence of SEQ ID NO: 10 and a VL sequence of SEQ ID NO: 21. In yet another particular embodiment, the antibody of the present disclosure comprises a VH sequence of SEQ ID NO: 11 and a VL sequence of SEQ ID NO: 22.

In one embodiment, an antibody that specifically binds to human IL-17A is an antibody that is described in Table 1. In one embodiment, an antibody that specifically binds to human IL-17A comprises an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 24 and 25, preferably SEQ ID NO: 24. In one embodiment, an antibody that specifically binds to human IL-17A is as set forth in SEQ ID NO: 24 or SEQ ID NO: 25, preferably SEQ ID NO: 24.

Other antibodies of the disclosure having a binding specificity for human IL-17A include those wherein the amino acids or nucleic acids encoding the amino acids have been mutated, yet have at least 60, 70, 80, 90 or 95 percent identity to the sequences described in Table 1. In one embodiment, it includes mutant amino acid sequences wherein no more than 1, 2, 3, 4 or 5 amino acids have been mutated in the variable regions when compared with the variable regions depicted in the sequence described in Table 1, while retaining substantially the same activity. The term "substantially the same activity" as used herein refers to the activity as indicated by substantially the same activity being at least 50%, at least 60%, at least 70%, at least 80%, at least 90%, at least 95%, at least 98% or even at least 100% or at least 110%, or at least 120%, or at least 130%, or at least 140%, or at least 150%, or at least 160%, or at least 170%, or at least 180%, or at least 190%, e.g. up to 200% of the activity as determined for the parent antibody, e.g., the antibody of the disclosure, in particular the antibody of the disclosure described in Table 1.

In yet another embodiment, the present disclosure provides an antibody comprising amino acid sequences that are homologous to the sequences described in Table 1, and said antibody binds to human IL-17A, and retains the desired functional properties of those antibodies described in Table 1.

In one embodiment, an antibody of the disclosure has a heavy chain variable region comprising CDR1, CDR2, and CDR3 sequences and a light chain variable region comprising CDR1, CDR2, and CDR3 sequences, wherein one or more of these CDR sequences have specified amino acid sequences based on the antibodies described herein or conservative modifications thereof, and wherein the antibodies retain the desired functional properties of the antibodies of the disclosure.

The term "conservatively modified variant" or "conservative variants" applies to both amino acid and nucleic acid sequences. With respect to particular nucleic acid sequences, conservatively modified variants refer to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations", which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" or "conservative variants" include individual substitutions, deletions or additions to a polypeptide sequence which result in the substitution of an amino acid with a chemically similar amino acid. Conservative substitution tables providing functionally similar amino acids are well known in the art. Such conservatively modified variants (i.e. having one or more "conservative modifications") are in addition to and do not exclude polymorphic variants, interspecies homologs, and alleles of the disclosure. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). In one embodiment, the term "conservative sequence modifications" are used to refer to amino acid modifications that do not significantly affect or alter the binding characteristics of the antibody containing the amino acid sequence.

Accordingly, the disclosure provides an isolated monoclonal antibody comprising:
a heavy chain variable region (VH) comprising, in sequence, the three complementary determining regions HCDR1, HCDR2 and HCDR3, wherein said HCDR1 is amino acid sequence SEQ ID No: 1, or a conservative variant thereof; said HCDR2 is amino acid sequence SEQ ID No: 2, or a conservative variant thereof; said HCDR3 is amino acid sequence selected from any one of SEQ ID No: 3, or a conservative variant thereof; and
a light chain variable region (VL) comprising, in sequence, the three complementary determining regions LCDR1, LCDR2 and LCDR3, wherein said LCDR1 is amino acid sequence SEQ ID No: 12, or a conservative variant thereof; said LCDR2 is amino acid sequence SEQ ID No: 13, or a conservative variant thereof; said LCDR3 having the amino acid sequence SEQ ID No: 14, or a conservative variant thereof;
wherein the antibody specifically binds to human IL-17A and/or neutralize IL-17A.

In one embodiment, an antibody of the disclosure is optimized for expression in a mammalian cell has a heavy chain variable region and a light chain variable region, wherein one or more of these sequences have specified amino acid sequences based on the antibodies described herein or conservative modifications thereof, and wherein the antibodies retain the desired functional properties of the antibodies of the disclosure. Accordingly, the disclosure provides an isolated monoclonal antibody optimized for expression in a mammalian cell comprising a heavy chain variable region and a light chain variable region wherein: the heavy chain variable region comprises an amino acid sequence selected from any of SEQ ID NOs: 10 and 11, and conservative modifications thereof; and the light chain variable region comprises an amino acid sequence selected from any of SEQ ID NOs: 21 and 22, and conservative modifications thereof; wherein the antibody specifically binds to human IL-17A and/or neutralize IL-17A.

In one embodiment, an antibody of the disclosure is optimized for expression in a mammalian cell has a full length heavy chain sequence and a full length light chain sequence, wherein one or more of these sequences have specified amino acid sequences based on the antibodies described herein or conservative modifications thereof, and wherein the antibodies retain the desired functional properties of the antibodies of the disclosure.

As used herein, the term, "optimized" means that a nucleotide sequence has been altered to encode an amino acid sequence using codons that are preferred in the production cell or organism, generally a eukaryotic cell, for example, a cell of Pichia, a Chinese Hamster Ovary cell (CHO) or a human cell. The optimized nucleotide sequence is engineered to retain completely or as much as possible the amino acid sequence originally encoded by the starting nucleotide sequence, which is also known as the "parental" sequence. The optimized sequences herein have been engineered to have codons that are preferred in mammalian cells. However, optimized expression of these sequences in other eukaryotic cells or prokaryotic cells is also envisioned herein. The amino acid sequences encoded by optimized nucleotide sequences are also referred to as optimized.

Another type of variable region modification is to mutate amino acid residues within the VH and/or VL CDR1, CDR2 and/or CDR3 regions to thereby improve one or more binding properties (e.g., affinity) of the antibody of interest, known as "affinity maturation". Site-directed mutagenesis or PCR-mediated mutagenesis can be performed to introduce the mutation(s) and the effect on antibody binding, or other functional property of interest, can be evaluated in in vitro or in vivo assays as described herein and provided in the Examples. Conservative modifications (as discussed above) can be introduced. The mutations may be amino acid substitutions, additions or deletions. Moreover, typically no more than one, two, three, four or five residues within a CDR region are altered.

An "affinity-matured" antibody is one with one or more alterations in one or more variable domains thereof that result in an improvement in the affinity of the antibody for antigen, compared to a parent antibody that does not possess those alteration(s). In one embodiment, an affinity-matured antibody has nanomolar or even picomolar affinities for the target antigen. Affinity-matured antibodies are produced by procedures known in the art. For example, Marks et al, Bio/Technology 10:779-783 (1992) describes affinity maturation by VH- and VL-domain shuffling. Random mutagenesis of HVR and/or framework residues is described by, for example: Barbas et al. Proc Nat. Acad. ScL USA 91:3809-3813 (1994); Schier et al. Gene 169:147-155 (1995); Jackson et al, J. Immunol. 154(7):3310- 9 (1995); and Hawkins et al, J. Mol. Biol. 226:889-896 (1992). Accordingly, the disclosure provides the antibody, wherein said antibody is affinity-matured.

An antibody of the disclosure further can be prepared using an antibody having one or more of the VH and/or VL sequences shown herein as starting material to engineer a modified antibody, which modified antibody may have altered properties from the starting antibody. An antibody can be engineered by modifying one or more residues within one or both variable regions (i.e., VH and/or VL), for example within one or more CDR regions and/or within one or more framework regions. Additionally or alternatively, an antibody can be engineered by modifying residues within the constant region(s), for example to alter the effector function(s) of the antibody.

One type of variable region engineering that can be performed is CDR grafting. Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al., 1998 Nature 332:323-327; Jones, P. et al., 1986 Nature 321:522- 525; Queen, C. et al., 1989 Proc. Natl. Acad. Sci. U.S.A. 86: 10029-10033; U.S. Pat. No. 5,225,539 to Winter, and U.S. Pat. Nos. 5,530,101; 5,585,089; 5,693,762 and 6,180,370 to Queen et al.).

Such framework sequences can be obtained from public DNA databases or published references that include germline antibody gene sequences or rearranged antibody sequences. For example, germline DNA sequences for human heavy and light chain variable region genes can be found in the "VBase" human germline sequence database (available on the Internet at www.mrc-cpe.cam.ac.uk/vbase), as well as in Kabat, E. A., et al., 1991 Sequences of Proteins of Immunological Interest, Fifth Edition, U.S. Department of Health and Human Services, NIH Publication No. 91-3242; Tomlinson, I. M., et al., 1992 J. fol. Biol. 227:776-798; and Cox, J. P. L. et al., 1994 Eur. J Immunol. 24:827-836; the contents of each of which are expressly incorporated herein by reference. For example, germline DNA sequences for human heavy and light chain variable region genes and rearranged antibody sequences can be found in "IMGT" database (available on the Internet at www.imgt.org; see Lefranc, M.P. et al., 1999 Nucleic Acids Res. 27:209-212; the contents of each of which are expressly incorporated herein by reference).

An example of framework sequences for use in the antibodies of the disclosure are those that are structurally similar to the framework sequences used by selected antibodies of the disclosure, e.g., consensus sequences and/or framework sequences used by monoclonal antibodies of the disclosure. The VH CDR1, 2 and 3 sequences, and the VL CDR1, 2 and 3 sequences, can be grafted onto framework regions that have the identical sequence as that found in the germline immunoglobulin gene from which the framework sequence derive, or the CDR sequences can be grafted onto framework regions that contain one or more mutations as compared to the germline sequences. For example, it has been found that in certain instances it is beneficial to mutate residues within the framework regions to maintain or enhance the antigen binding ability of the antibody (see e.g., U.S. Pat. Nos. 5,530,101; 5,585,089; 5,693,762 and 6,180,370 to Queen et al).

A wide variety of antibody /immunoglobulin frameworks or scaffolds can be employed so long as the resulting polypeptide includes at least one binding region which specifically binds to IL-17A. Such frameworks or scaffolds include the five main idiotypes of human immunoglobulins, antigen-binding fragments thereof, and include immunoglobulins of other animal species, preferably having humanized aspects.

In one aspect, the disclosure pertains to a method of generating non-immunoglobulin based antibodies using non-immunoglobulin scaffolds onto which CDRs of the disclosure can be grafted. Known or future non-immunoglobulin frameworks and scaffolds may be employed, as long as they comprise a binding region specific for the target IL-17A protein. Known non-immunoglobulin frameworks or scaffolds include, but are not limited to, fibronectin (Compound Therapeutics, Inc., Waltham, Mass.), ankyrin (Molecular Partners AG, Zurich, Switzerland), domain antibodies (Domantis, Ltd., Cambridge, Mass., and Ablynx nv, Zwijnaarde, Belgium), lipocalin (Pieris Proteolab AG, Freising, Germany), small modular immuno-pharmaceuticals (Trubion Pharmaceuticals Inc., Seattle, Wash.), maxybodies (Avidia, Inc., Mountain View, Calif), Protein A (Affibody AG, Sweden), and affilin (gamma-crystallin or ubiquitin) (Scil Proteins GmbH, Halle, Germany).

The antibodies according to the disclosure have valuable properties predicted to be beneficial for human patients in need of a human IL-17A targeting therapy. The antibody according to the disclosure is characterized by one or more of the following properties (as determined in Examples 1 and 2):
the antibody specifically binds to human IL-17A and:
(a) has a binding specificity for cynomolgus monkey IL-17A;
(b) selectively binds to human IL-17A over human IL-17B, IL-17C, IL-17D, IL-17E and IL-17F as measured by ELISA;
(c) inhibits or blocks binding between IL-17A and its receptor (IL-17RA);
(d) reduces or neutralizes IL-17A activity;
(e) capable of inhibiting Gro-α secretion when assessed *in vitro* in HT-29 assay;
(f) has the ability to block interaction between IL-17A and IL-17RA with a potency relative to that of secukinumab (relative potency), determined in ELISA assay, greater than 5, preferably greater than 10, more preferably greater than 20, and wherein said relative potency is the ratio of the IC₅₀ value in ng/mL of secukinumab as measured by ELISA to the IC₅₀ value in ng/mL of the antibody of the invention in the scFv format as measured by ELISA;
(g) has the ability to neutralize IL-17A with a potency relative to that of secukinumab (relative potency), determined by measuring Gro-α secretion in an HT-29 assay, greater than 50, preferably greater than 100, more preferably greater than 150, and wherein said relative potency is the ratio of the IC₅₀ value in ng/mL of secukinumab as measured in the HT-29 assay to the IC₅₀ value in ng/mL of the antibody of the invention in the scFv format as measured in the HT-29 assay;
(h) capable of inhibiting the activity of 1 ng human IL-17A at a concentration of 1 ng/mL or less, preferably 0.5 ng/mL or less, more preferably 0.2 ng/mL or less, by 50%, said inhibitory activity is determined by measuring Gro-α secretion induced by human IL-17A in HT-29 assay in the presence of 50 pg/ml TNFα;
(i) binds to human IL-17A with a dissociation constant (K_{D}) of less than 5 nM, e.g., less than 1 nM, less than 0.5 nM, less than 0.2 nM, preferably less than 100 pM, more preferably less than 50 pM, as measured by surface plasmon resonance, preferably as measured by surface plasmon resonance in a direct setup;
(j) binds to Cynomolgus IL-17A with a K_{D} of less than 10 nM, e.g., less than 7 nM, less than 5 nM, less than 2 nM, less than 1 nM, preferably less than 0.5 nM as measured by surface plasmon resonance, preferably as measured by surface plasmon resonance in a capture setup;
(k) when in scFv format, has a melting temperature (Tm), determined by differential scanning fluorimetry, of at least 60°C, preferably of at least 62°C, more preferably at least 65°C, in particular wherein said antibody is in phosphate-citrate buffer at pH 6.4, 150 mM NaCl;
(l) when in scFv format, has a loss in monomer content, after five consecutive freeze-thaw cycles, of less than 5%, preferably less than 3%, when the antibody of the invention is at a starting concentration of 10 mg/ml, in particular wherein said antibody is in phosphate buffered saline (PBS), pH 7.4; and/or
(m) when in scFv format, has a loss in monomer content, after storage for at least two weeks, particularly for at least four weeks, at 4°C, of 5% or less, when the antibody of the disclosure is at a starting concentration of 10 mg/ml, in particular wherein said antibody is in phosphate buffered saline (PBS), pH 7.4.

In one embodiment, the antibody of the present disclosure selectively binds to human IL-17A over human IL-17B, IL-17C, IL-17D, IL-17E and IL-17F as measured by ELISA. As used herein, the terms "selectively binds to" shall mean that the antibody, composition, formulation, etc. does not significantly bind to IL-17B/C/D/E/F, but does bind to IL-17A. Selective binding is characterized by a high affinity (or low K_{D}) and a low to moderate IC₅₀ as distinguished from nonspecific binding which usually has a low affinity with a moderate to high IC₅₀. Typically, binding is considered selective when the antibody binds with a K_{D} of less than 10⁻⁷ M. Suitably, the antibody of the present disclosure binds to human IL-17A with a higher affinity or with a lower K_{D} than it binds to human IL-17B, IL-17C, IL-17D, IL-17E and IL-17F as measured by SPR. Suitably, the antibody of the present disclosure has IC₅₀s to IL-17B, IL-17C, IL-17D, IL-17E, and IL-17F of more than 100 greater, e.g., more than 200 greater, more than 300 greater, than the IC₅₀ to IL-17A as measured in ELISA.

The antibodies of the disclosure specifically bind to IL-17A, wherein binding to IL-17A (a) inhibits or blocks binding between IL-17A and its receptor (IL-17RA), and (b) reduces or neutralizes IL-17A activity.

As used herein, the term "neutralizing antibody" describes an antibody that is capable of neutralizing the biological signaling activity of IL-17A, for example by blocking binding of IL-17A to one or more of its receptors, in particular by blocking binding of IL-17A to IL-17RA. The antibody of the present disclosure are IL-17A neutralizing antibody. It will be appreciated that the term "neutralizing" as used herein refers to a reduction in biological signaling activity which may be partial or complete. Neutralization of IL-17A may be determined by a variety of assays, examples of which are described elsewhere herein.

Thus, the antibody of the disclosure is capable of inhibiting Gro-α secretion when assessed *in vitro* in HT-29 assay (described in Examples 1 and 2). In one embodiment, the antibody of the disclosure has the ability to neutralize IL-17A with a potency relative to that of secukinumab (relative potency), determined by measuring Gro-α secretion in an HT-29 assay, greater than 50, preferably greater than 100, more preferably greater than 150, and wherein said relative potency is the ratio of the IC₅₀ value in ng/mL of secukinumab as measured in the HT-29 assay to the IC₅₀ value in ng/mL of the antibody of the disclosure in the scFv format as measured in the HT-29 assay. In a further embodiment, the antibody of the disclosure is capable of inhibiting the activity of 1 ng human IL-17A at a concentration of 1 ng/mL or less, preferably 0.5 ng/mL or less, more preferably 0.2 ng/mL or less, by 50%, said inhibitory activity is determined by measuring Gro-α secretion induced by human IL-17A in HT-29 assay in the presence of 50 pg/ml TNFα.

In one embodiment, the antibody of the disclosure has the ability to block interaction between IL-17A and IL-17RA with a potency relative to that of secukinumab (relative potency), determined in ELISA assay, greater than 5, preferably greater than 10, more preferably greater than 20, and wherein said relative potency is the ratio of the IC₅₀ value in ng/mL of secukinumab as measured by ELISA to the IC₅₀ value in ng/mL of the antibody of the disclosure in the scFv format as measured by ELISA.

As used herein, the term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the sum total of non-covalent interactions between a single binding site of a molecule (e.g., of an antibody) and its binding partner (e.g., an antigen). Unless indicated otherwise, as used herein, "binding affinity", "bind to", "binds to" or "binding to" refers to intrinsic binding affinity that reflects a 1:1 interaction between members of a binding pair (e.g., an antibody fragment and antigen). The affinity of a molecule X for its partner Y can generally be represented by the dissociation constant (K_{D}). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present disclosure. Specific illustrative and exemplary embodiments for measuring binding affinity, i.e. binding strength are described in the following.

The term "k_{assoc}", "ka" or "kₒₙ", as used herein, is intended to refer to the association rate of a particular antibody-antigen interaction, whereas the term "k_{dis}", "kd" or "k_{off}", as used herein, is intended to refer to the dissociation rate of a particular antibody- antigen interaction. In one embodiment, the term "K_{D}", as used herein, is intended to refer to the dissociation constant, which is obtained from the ratio of kd to ka (i.e. kd/ka) and is expressed as a molar concentration (M). The "K_{D}" or "K_{D} value" or "KD" or "KD value" according to this disclosure is in one embodiment measured by using a T200 device (Biacore, GE Healthcare) To measure affinity of the humanized scFvs to human IL-7A , biotinylated human IL-17A is captured using the Biotin-CAPture kit from Biacore. After each analyte injection cycle the CAP sensor chip is regenerated and new antigen is captured. The scFvs are injected as analyte using a dose response multi-cycle kinetic assay with concentrations of the analyte ranging from 0.35 to 90 nM diluted in running buffer. Obtained sensorgrams are fitted using the 1:1 binding model. Alternatively or in addition, affinity of the humanized scFvs can be measured and analyzed using an alternative SPR assay setup: IL-17A is immobilized on a CM5 sensor chip (GE Healthcare) by amine-coupling, and serial dilutions of scFvs ranging from 0.35 to 90 nM arere injected over the immobilized IL-17A.

Suitably, the affinity of the antibody of the disclosure to IL-17A, may be higher than the affinity of IL-17A to IL-17RA. It will be appreciated that a higher affinity of the antibody of the disclosure as compared to the affinity of IL-17A to IL-17RA may be particularly useful for dissociating or neutralizing the pre-formed IL-17RA/IL-17A complexes. In one embodiment, the antibody of the disclosure neutralizes IL-17RA/IL-17A interaction. In another embodiment, the antibody of the disclosure inhibits or blocks binding between IL-17A and its receptor (IL-17RA). In one embodiment, the antibody of the disclosure neutralizes IL-17A activity.

Suitably, the affinity of the antibody of the disclosure to IL-17A may be comparable to or higher, preferably higher, than the affinity of secukinumab to IL-17A. In one embodiment, the antibody of the disclosure neutralizes IL-17A activity with potency equal to or higher, preferably higher, than secukinumab. In a further embodiment, the antibody of the disclosure neutralizes IL-17RA/IL-17A interaction with potency equal to or higher, preferably higher, than secukinumab.

The binding affinity of an antibody may be determined, for example, by the dissociation constant (K_{D}). A stronger affinity is represented by a lower K_{D}, while a weaker affinity is represented by a higher K_{D}.

Thus, in a suitable embodiment, the antibody of the disclosure may have a K_{D} of between 1 and 10000 pM, 1 and 7000 pM, 1 and 5000 pM, 1 and 2500 pM, 1 and 2000 pM, 1 and 1000 pM, 1 and 750 pM, 1 and 500 pM, 1 and 400 pM, 1 and 300 pM, 1 and 200 pM, 1 and 100 pM, 1 and 50 pM, preferably as measured by surface plasmon, more preferably as measured by surface plasmon resonance in a direct setup. In a suitable embodiment, the antibody of the disclosure has a K_{D} of between 1 and 100 pM as measured by surface plasmon resonance in a direct setup. In a preferred embodiment, the antibody of the disclosure has a K_{D} of between 1 and 50 pM as measured by surface plasmon resonance in a direct setup. In a suitable embodiment, the antibody of the disclosure may have a K_{D} of less than 5 nM, less than 4 nM, less than 3 nM, less than 2 nM, less than 1 nM, less than 0.5 nM, less than 0.4 nM, less than 0.3 nM, less than 0.25 nM, less than 0.2 nM, less than 150 pM, less than 100 pM, or less than 50 pM, preferably as measured by surface plasmon resonance, more preferably as measured by surface plasmon resonance in a direct setup. Suitably, the antibody of the disclosure has a K_{D} of less than 100 pM. Suitably, the antibody of the disclosure has a K_{D} of less than 50 pM. More suitably, the antibody of the disclosure has a K_{D} of less than 50 pM as measured by surface plasmon resonance in a direct setup.

In a further embodiment, the antibody of the disclosure binds to Cynomolgus IL-17A with a K_{D} of less than 10 nM, e.g. less than 7 nM, less than 5 nM, less than 2 nM, less than 1 nM, preferably less than 0.5 nM as measured by surface plasmon resonance (SPR) in a capture set-up.

Suitably, the antibody of the disclosure binds to human IL-17A with a Kₒₙ rate of at least 10³ M⁻¹s⁻¹ or greater, at least 10⁴ M⁻¹s⁻¹ or greater, at least 5x10⁴ M⁻¹s⁻¹ or greater, at least 10⁵ M⁻¹s⁻¹ or greater, at least 5x10⁵ M⁻¹s⁻¹ or greater, at least 10⁶ M⁻¹s⁻¹ or greater as measured by surface plasmon resonance (SPR), preferably as measured by surface plasmon resonance in a direct setup. Preferably, the antibody of the disclosure has a Kₒₙ rate of at least 5x10⁵ M⁻¹s⁻¹ or greater, preferably at least 10⁶ M⁻¹s⁻¹ or greater, as measured by SPR, preferably as measured by surface plasmon resonance in a direct setup.

Suitably, the antibody of the disclosure binds to human IL-17A with a K_{off} rate of 5x10⁻³ s⁻¹ or less, 3x10⁻³ s⁻¹ or less, 10⁻³ s⁻¹ or less, 5x10⁻⁴ s⁻¹ or less, 3x10⁻⁴ s⁻¹ or less, 10⁻⁴ s⁻¹ or less, 5x10⁻⁵ s⁻¹ or less, as measured by surface plasmon resonance (SPR), preferably as measured by surface plasmon resonance in a direct setup. Preferably, the antibody of the disclosure has a K_{off} rate of 10⁻⁴ s⁻¹ or less, in particular 5x10⁻⁵ s⁻¹ or less or less as measured by SPR, preferably as measured by surface plasmon resonance in a direct setup.

Suitably, the antibody of the disclosure has beneficial biophysical properties.

Suitably, the antibody of the disclosure has a melting temperature (Tm) of at least 60°C, preferably of at least 62°C, more preferably at least 65°C, when expressed in the scFv (single chain variable fragment) antibody format, as determined by differential scanning fluorimetry (DSF) as described earlier (Egan, et al., MAbs, 9(1) (2017), 68-84; Niesen, et al., Nature Protocols, 2(9) (2007) 2212-2221). The midpoint of transition for the thermal unfolding of the scFv constructs is determined by Differential Scanning Fluorimetry using the fluorescence dye SYPRO® Orange (see Wong & Raleigh, Protein Science 25 (2016) 1834-1840). Samples in phosphate-citrate buffer at pH 6.4 are prepared at a final protein concentration of 50 µg/mL and containing a final concentration of 5x SYPRO® Orange in a total volume of 100 µl. Twenty-five microliters of prepared samples are added in triplicate to white-walled AB gene PCR plates. The assay is performed in a qPCR machine used as a thermal cycler, and the fluorescence emission is detected using the software's custom dye calibration routine. The PCR plate containing the test samples is subjected to a temperature ramp from 25°C to 96°C in increments of 1°C with 30 s pauses after each temperature increment. The total assay time is about two hours. The Tm is calculated by the software GraphPad Prism using a mathematical second derivative method to calculate the inflection point of the curve. The reported Tm is an average of three measurements.

The antibody of the disclosure, in particular when expressed in the scFv (single chain variable fragment) antibody format, is characterized by a loss in monomer content, after five consecutive freeze-thaw cycles, of less than 5%, preferably less than 3%, when the antibody of the disclosure is at a starting concentration of 10 mg/ml.

After storage for at least two weeks, particularly for at least four weeks at 4°C, the antibody of the disclosure, in particular when expressed in the scFv (single chain variable fragment) antibody format, is characterized by a loss in monomer content of 5% or less, e.g. less than 4%, less than 3%, less than 2%, preferably less than 1%, when the antibody of the disclosure is at a starting concentration of 10 mg/ml. The loss in monomer content is as determined by area under the curve calculation of SE-HPLC chromatograms. SE-HPLC is a separation technique based on a solid stationary phase and a liquid mobile phase as outlined by the USP chapter 621. This method separates molecules based on their size and shape utilizing a hydrophobic stationary phase and aqueous mobile phase. The separation of molecules is occurring between the void volume (V0) and the total permeation volume (VT) of a specific column. Measurements by SE-HPLC are performed on a Chromaster HPLC system (Hitachi High-Technologies Corporation) equipped with automated sample injection and a UV detector set to the detection wavelength of 280 nm. The equipment is controlled by the software EZChrom Elite (Agilent Technologies, Version 3.3.2 SP2) which also supports analysis of resulting chromatograms. Protein samples are cleared by centrifugation and kept at a temperature of 4-6°C in the autosampler prior to injection. For the analysis of scFv samples the column Shodex KW403-4F (Showa Denko Inc., #F6989202) is employed with a standardized buffered saline mobile phase (50 mM sodium-phosphate pH 6.5, 300 mM sodium chloride) at the recommended flow rate of 0.35 mL/min. The target sample load per injection was 5 µg. Samples are detected by an UV detector at a wavelength of 280 nm and the data recorded by a suitable software suite. The resulting chromatograms are analyzed in the range of V0 to VT thereby excluding matrix associated peaks with >10 min elution time.

Suitably, the isolated antibody of the present disclosure is selected from the group consisting of: a monoclonal antibody, a chimeric antibody, a Fab, an Fv, an scFv, dsFv, an scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. F-star's Modular Antibody Technology™), preferably scFv.

Suitably, the antibody of the disclosure is an Fv. Suitably, the antibody of the disclosure is scFv antibody fragment. "Single-chain Fv" or "scFv" or "sFv" antibody fragments comprise the VH and VL domains of an antibody, wherein these domains are present in a single polypeptide chain. Generally, the Fv polypeptide further comprises a polypeptide linker between the VH and VL domains which enables the sFv to form the desired structure for target binding. "Single-chain Fv" or "scFv" antibody fragments comprise the VH and VL domains of antibody, wherein these domains are present in a single polypeptide chain. Generally, the scFv polypeptides further comprises a polypeptide linker between the VH and VL domains which enables the scFv to form the desired structure for antigen binding (see, for example, Plückthun, The pharmacology of Monoclonal Antibodies, vol. 113, Rosenburg and Moore eds., (Springer-Verlag, New York, 1994), pp. 269-315). In particular embodiments, said functional fragment is an scFv format comprising the linker according to SEQ ID NO: 23. In one embodiment, an antibody that specifically binds to human IL-17A is an antibody that is described in Table 1. In one embodiment, an antibody that specifically binds to human IL-17A comprises an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 24 and SEQ ID NO: 25. In a further embodiment, the antibody of the disclosure is a single-chain variable fragment (scFv) as shown in SEQ ID NO: 24, and SEQ ID NO: 25.

Suitably, the antibody of the disclosure is an IgG antibody. In one embodiment, the antibody of the disclosure is an IgG selected from the group consisting of an IgG1, an IgG2, an IgG3 and an IgG4, preferably an IgG1.

### Multispecific molecules of the disclosure

The present disclosure also provides an antibody which is a multispecific molecule, e.g., bispecific molecule, trispecific molecule, tetraspecific, pentaspecific, hexaspecific molecule.

The term "multispecific molecule" or "multispecific antibody" as used herein, refers to an antibody that binds to two or more different epitopes on at least two or more different targets (e.g., IL-17A and another target different from IL-17A), or binds to two or more different epitopes of the same target.

The term "multispecific molecule" includes bispecific, trispecific, tetraspecific, pentaspecific and hexaspecific antibodies. The term "bispecific antibody" as used herein, refers to an antibody that binds to two different epitopes on two different targets or on the same target. The term "trispecific antibody" as used herein, refers to an antibody that binds to three different epitopes on three different targets or on the same target.

An antibody of the disclosure can be derivatized or linked to another functional molecule, e.g., another peptide or protein (e.g., another antibody or ligand for a receptor) to generate a multispecific molecule that binds to at least two binding sites and/or different target molecules. The antibody of the disclosure may in fact be derivatized or linked to more than one other functional molecule to generate multispecific molecules that bind to more than two different binding sites and/or target molecules. To create a multispecific molecule of the disclosure, an antibody of the disclosure can be functionally linked (e.g., by chemical coupling, genetic fusion, noncovalent association or otherwise) to one or more other binding molecules, such as another antibody, peptide or binding mimetic, such that a multispecific molecule results.

Accordingly, the present disclosure includes multispecific molecules comprising at least one first binding specificity for IL-17A and a second binding specificity for a second target epitope. For example, the second target epitope is present on another target molecule different from IL-17A. Accordingly, the present disclosure includes multispecific molecules comprising at least one first binding specificity for IL-17A and a second binding specificity for a second target epitope. For example, the second target epitope is another epitope of IL-17A different from the first target epitope. The multispecific molecule can further include a third binding specificity, in addition to the first and second target epitope.

In a further embodiment, the present disclosure includes multispecific molecules monovalent, bivalent or multivalent for IL-17A specificity, preferably monovalent.

In another particular embodiment of the present disclosure, the antibody of the present disclosure is a monovalent or multivalent for IL-17A specificity molecule, e.g., bivalent, trivalent, tetravalent, pentavalent, hexavalent.

The term "monovalent molecule" or "monovalent antibody", as used herein, refers to an antibody that has a single antigen-binding moiety that binds to a single epitope on a target molecule, such as IL-17A.

The term "multivalent antibody" refers to a single binding molecule with more than one valency, where "valency" is described as the number of antigen-binding moieties that binds to epitopes on identical target molecules. As such, the single binding molecule can bind to more than one target molecule, or more than one binding site on a target molecule that contains multiple copies of the epitope. Examples of multivalent antibodies include, but are not limited to bivalent antibodies, trivalent antibodies, tetravalent antibodies, pentavalent antibodies, and the like. The term "bivalent antibody" as used herein, refers to an antibody that has two antigen binding moieties, each of which binds to an identical epitope.

Suitably, the multispecific molecule of the present disclosure, e.g., bispecific molecule, and / or a multivalent molecule, e.g., monovalent for IL-17A specificity molecule, bivalent for IL-17A specificity molecule, is an antibody format selected from any suitable multispecific, e.g. bispecific, format known in the art, including, by way of non-limiting example, formats based on a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)₂) or bibody (Fab-(scFv)₁), Fab, , Fab-Fv₂, Morrison (IgG CH3-scFv fusion (Morrison L) or IgG CL-scFv fusion (Morrison H)), triabody, scDb-scFv, bispecific Fab₂, di-miniantibody, tetrabody, scFv-Fc-scFv fusion, scFv-HSA-scFv fusion, di-diabody, DVD-Ig, COVD, IgG-scFab, scFab-dsscFv, Fv₂-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), TslAb (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs) (bispecific IgGs prepared by the KiH technology); an Fv, scFv, scDb, tandem-di-scFv, tandem tri-scFv, Fab-(scFv)₂, Fab-(scFv)₁, Fab, Fab-Fv₂, COVD fused to the N- and/or the C-terminus of either chain of a heterodimeric Fc domain or any other heterodimerization domain, a MATCH (described in WO2016/0202457; Egan T., et al., mAbs 9 (2017) 68-84) and DuoBodies (bispecific IgGs prepared by the Duobody technology) (MAbs. 2017 Feb/Mar;9(2): 182-212. doi: 10.1080/19420862.2016.1268307). Particularly suitable for use herein is a single-chain diabody (scDb) or scDb-scFv.

The term "diabodies" refers to antibody fragments with two antigen-binding sites, which fragments comprise a VH connected to VL in the same polypeptide chain (VH-VL). By using a linker that is too short to allow pairing between the two domains on the same chain, the domains are forced to pair with the complementary domains of another chain to create two antigen-binding sites. Diabodies may be bivalent or bispecific. Diabodies are described more fully in, for example, EP404097, WO 93/01161, Hudson et al., Nat. Med. 9:129-134 (2003), and Holliger et al., Proc. Natl. Acad. Sci. U.S.A 90: 6444-6448 (1993). Triabodies and tetrabodies are also described in Hudson et al., Nat. Med. 9:129-134 (2003). The term "scDb-scFv" refers to an antibody format, wherein a single-chain Fv (scFv) fragment is fused by a flexible Gly-Ser linker to a single-chain diabody (scDb).

The bispecific scDb, in particular the bispecific monomeric scDb, particularly comprises two variable heavy chain domains (VH) or fragments thereof and two variable light chain domains (VL) or fragments thereof connected by linkers L1, L2 and L3 in the order VHA-L1-VLB-L2-VHB-L3-VLA, VHA-L1-VHB-L2-VLB-L3-VLA, VLA-L1-VLB-L2-VHB-L3-VHA, VLA-L1-VHB-L2-VLB-L3-VHA, VHB-L1-VLA-L2-VHA-L3-VLB, VHB-L1-VHA-L2-VLA-L3-VLB, VLB-L1-VLA-L2-VHA-L3-VHB or VLB-L1-VHA-L2-VLA-L3-VHB, wherein the VLA and VHA domains jointly form the antigen binding site for the first antigen, and VLB and VHB jointly form the antigen binding site for the second antigen.

The linker L1 particularly is a peptide of 2-10 amino acids, more particularly 3-7 amino acids, and most particularly 5 amino acids, and linker L3 particularly is a peptide of 1-10 amino acids, more particularly 2-7 amino acids, and most particularly 5 amino acids. The middle linker L2 particularly is a peptide of 10-40 amino acids, more particularly 15-30 amino acids, and most particularly 20-25 amino acids.

In one embodiment, the multispecific and/or multivalent molecules of the present disclosure is in a MATCH format described in WO 2016/0202457; Egan T., et al., mAbs 9 (2017) 68-84.

Multispecific and/or multivalent molecules of the present disclosure can be produced using any convenient antibody manufacturing method known in the art (see, e.g., Fischer, N. & Leger, O., Pathobiology 74 (2007) 3-14 with regard to the production of bispecific constructs; Hornig, N. & Färber-Schwarz, A., Methods Mol. Biol. 907 (2012)713-727, and WO 99/57150 with regard to bispecific diabodies and tandem scFvs). Specific examples of suitable methods for the preparation of the bispecific construct of the present disclosure further include, inter alia, the Genmab (see Labrijn et al., Proc. Natl. Acad. Sci. U.S.A 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, e.g., Zhu et al., Cancer Lett. 86 (1994) 127-134); and Suresh et al., Methods Enzymol. 121 (1986) 210-228).

Other antibodies which can be employed in the multispecific and in the multivalent molecules of the disclosure are murine, chimeric and humanized monoclonal antibodies.

The multispecific molecules of the present disclosure can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N- succinimidyl-3- (2-pyridyldithio)propionate (SPDP), and sulfosuccinimidyl 4- (N-maleimidomethyl)cyclohaxane-1-carboxylate (sulfo-SMCC) (see e.g., Karpovsky et al., 1984 J. Exp. Med. 160: 1686; Liu, M A et al., 1985 Proc. Natl. Acad. Sci. U.S.A 82:8648). Other methods include those described in Paulus, 1985 Behring Ins. Mitt. No. 78, 118-132; Brennan et al., 1985 Science 229:81-83), and Glennie et al., 1987 J. Immunol. 139: 2367-2375). Conjugating agents are SATA and sulfo-SMCC, both available from Pierce Chemical Co. (Rockford, 111.).

When the binding specificities are antibodies, they can be conjugated by sulfhydryl bonding of the C-terminus hinge regions of the two heavy chains. In a particularly embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb X mAb, mAb X Fab, Fab X F (ab')₂ or ligand X Fab fusion protein. Suitably, the multispecific molecule of the present disclosure can be a single chain molecule comprising one single chain antibody and a binding determinant, or a single chain multispecific molecule comprising two binding determinants. Multispecific molecules may comprise at least two single chain molecules. Methods for preparing multispecific molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the multispecific molecules to their specific targets can be confirmed by, for example, enzyme-linked immunosorbent assay (ELISA), radioimmunoassay (REA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest.

### Nucleic acids, vectors, hosts cells and methods of production of the present disclosure

In a further aspect, the disclosure provides a nucleic acid encoding the antibody of the disclosure. The present disclosure also provides nucleic acid sequences that encode CDRs, VH, VL, the full length heavy chain, and the full length light chain of the antibodies that specifically bind to IL-17A protein. Such nucleic acid sequences can be optimized for expression in mammalian cells.

The term "nucleic acid" is used herein interchangeably with the term "polynucleotide" and refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The disclosure provides substantially purified nucleic acid molecules which encode polypeptides comprising segments or domains of the IL-17A-binding antibody chains described above. When expressed from appropriate expression vectors, polypeptides encoded by these nucleic acid molecules are capable of exhibiting IL-17A antigen binding capacity.

Also provided in the disclosure are polynucleotides which encode at least one CDR region and usually all three CDR regions from the heavy or light chain of the IL-17A-binding antibody set forth in Table 1. Some other polynucleotides encode all or substantially all of the variable region sequence of the heavy chain and/or the light chain of the IL-17A-binding antibody set forth in Table 1. Because of the degeneracy of the code, a variety of nucleic acid sequences will encode each of the immunoglobulin amino acid sequences.

The polynucleotide sequences can be produced by de novo solid-phase DNA synthesis or by PCR mutagenesis of an existing sequence (e.g., sequences as described in the Examples below) encoding an IL-17A-binding antibody. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetra. Lett., 22: 1859, 1981; and the solid support method of U.S. Pat. No. 4,458,066. Introducing mutations to a polynucleotide sequence by PCR can be performed as described in, e.g., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the disclosure are expression vectors and host cells for producing the antibody of the disclosure.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the disclosure is intended to include such other forms of expression vectors, such as viral vectors (e.g., replication defective retroviruses, adenoviruses and adeno- associated viruses), which serve equivalent functions.

The term "operably linked" refers to a functional relationship between two or more polynucleotide (e.g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis-acting. However, some transcriptional regulatory sequences, such as enhancers, need not be physically contiguous or located in close proximity to the coding sequences whose transcription they enhance.

Various expression vectors can be employed to express the polynucleotides encoding the antibody of the disclosure. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, e.g., Harrington et al., Nat Genet. 15:345, 1997). For example, nonviral vectors useful for expression of the IL-17A-binding polynucleotides and polypeptides in mammalian (e.g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adenoassociated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (e.g., enhancers) that are operably linked to the polynucleotides encoding an IL-17A-binding antibody. In one embodiment, an inducible promoter is employed to prevent expression of inserted sequences except under inducing conditions. Inducible promoters include, e.g., arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of an IL-17A-binding antibody. These elements typically include an ATG initiation codon and adjacent ribosome binding site or other sequences. In addition, the efficiency of expression may be enhanced by the inclusion of enhancers appropriate to the cell system in use (see, e.g., Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

The expression vectors may also provide a secretion signal sequence position to form a fusion protein with polypeptides encoded by inserted IL-17A-binding antibody sequences. More often, the inserted IL-17A-binding antibody sequences are linked to signal sequences before inclusion in the vector. Vectors to be used to receive sequences encoding IL-17A-binding antibody light and heavy chain variable domains sometimes also encode constant regions or parts thereof. Such vectors allow expression of the variable regions as fusion proteins with the constant regions thereby leading to production of intact antibodies. Typically, such constant regions are human.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the IL-17A-binding antibody chains can be either prokaryotic or eukaryotic. E. coli is one prokaryotic host useful for cloning and expressing the polynucleotides of the present disclosure. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express IL-17A-binding polypeptides of the disclosure. Insect cells in combination with baculovirus vectors can also be used.

In one embodiment, mammalian host cells are used to express and produce the IL-17A-binding polypeptides of the present disclosure. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, e.g., Winnacker, FROM GENES TO CLONES, VCH Publishers, N.Y., N.Y., 1987. Expression vectors for mammalian host cells can include expression control sequences, such as an origin of replication, a promoter, and an enhancer (see, e.g., Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP polIII promoter, the constitutive MPS V promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, et al., supra). Other methods include, e.g., electroporation, calcium phosphate treatment, liposome-mediated transformation, injection and microinjection, ballistic methods, virosomes, immunoliposomes, polycation:nucleic acid conjugates, naked DNA, artificial virions, fusion to the herpes virus structural protein VP22 (Elliot and O'Hare, Cell 88:223, 1997), agent-enhanced uptake of DNA, and ex vivo transduction. For long-term, high-yield production of recombinant proteins, stable expression will often be desired. For example, cell lines which stably express IL-17A-binding antibody can be prepared using expression vectors of the disclosure which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present disclosure thus provides a method of producing the antibody of the disclosure, wherein said method comprises the step of culturing a host cell comprising a nucleic acid or a vector encoding the antibody of the disclosure, whereby said antibody of the disclosure or a fragment thereof is expressed.

### Pharmaceutical compositions of the disclosure

In a further aspect, the present disclosure relates to a pharmaceutical composition comprising the antibody of the present disclosure , and a pharmaceutically acceptable carrier. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

A pharmaceutical composition of the present disclosure can be administered by a variety of methods known in the art. The route and/or mode of administration vary depending upon the desired results. Administration can be intravenous, intramuscular, intraperitoneal, or subcutaneous, or administered proximal to the site of the target. The pharmaceutically acceptable carrier should be suitable for intravenous, intramuscular, subcutaneous, parenteral, spinal or epidermal administration (e.g., by injection or infusion). Depending on the route of administration, the active compound, i.e., antibody, and multispecific molecule, may be coated in a material to protect the compound from the action of acids and other natural conditions that may inactivate the compound.

Pharmaceutical compositions of the disclosure can be prepared in accordance with methods well known and routinely practiced in the art. See, e.g., Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the IL-17A-binding antibody is employed in the pharmaceutical compositions of the disclosure. The IL-17A binding antibodies are formulated into pharmaceutically acceptable dosage forms by conventional methods known to those of skill in the art. Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the present disclosure can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present disclosure employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

Antibodies are usually administered on multiple occasions. Intervals between single dosages can be weekly, monthly or yearly. Intervals can also be irregular as indicated by measuring blood levels of IL-17A-binding antibody in the patient. Alternatively, an antibody can be administered as a sustained release formulation, in which case less frequent administration is required. Dosage and frequency vary depending on the half-life of the antibody in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

### Uses and methods of the disclosure

The antibodies of the present disclosure have *in vitro* and *in vivo* diagnostic and therapeutic utilities. For example, these molecules can be administered to cells in culture, e.g. *in vitro* or *in vivo,* or in a subject, e.g., *in vivo,* to treat, prevent or diagnose a variety of disorders.

The term "subject" includes human and non-human animals. Non-human animals include all vertebrates, e.g., mammals and non-mammals, such as non-human primates, sheep, dog, cow, chickens, amphibians, and reptiles. Except when noted the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, e.g., in a human, and includes: (a) inhibiting the disease, e.g., arresting its development; and (b) relieving the disease, e.g., causing regression of the disease.

The term "prevent" or "prevention" refers to a complete inhibition of development of a disease, or any secondary effects of disease. The term "prevent" or "prevention" as used herein covers prevention of a disease or condition from occurring in an individual who may be predisposed to the disease but has not yet been diagnosed as having it.

The term "therapeutically effective amount" or "efficacious amount" refers to the amount of an agent that, when administered to a mammal or other subject for treating a disease, is sufficient to effect such treatment for the disease. The "therapeutically effective amount" will vary depending on the agent, the disease and its severity and the age, weight, etc., of the subject to be treated.

In one aspect, the present disclosure relates to the antibody of the present disclosure, or the pharmaceutical composition of the present disclosure for use as a medicament.

In one aspect, the antibody of the disclosure, or the pharmaceutical composition of the disclosure are particularly suitable for use in the treatment, prevention or diagnosis, in particular treatment, of a disorder mediated by IL-17A or a disorder that can be treated by inhibiting Gro-α secretion.

In the present disclosure, the term "a disorder mediated by IL-17A" encompasses all diseases and medical conditions in which IL-17A plays a role, whether directly or indirectly, in the disease or medical condition, including causation, development, progress, persistence or pathology of the disease or condition. Accordingly, the term "a disorder mediated by IL-17A" include conditions associated with or characterized by aberrant IL-17A levels and/or diseases or conditions that can be treated by reducing or suppressing IL-17A induced activity in target cells or tissues, e.g., production of IL-6 or Gro-α. These include inflammatory conditions and autoimmune diseases, such as arthritis, rheumatoid arthritis, or psoriasis. These further include allergies and allergic conditions, hypersensitive reactions, chronic obstructive pulmonary disease, cystic fibrosis and organ or tissue transplant rejection. For example, the antibody of the disclosure may be used for the treatment of recipients of heart, lung, combined heart-lung, liver, kidney, pancreatic, skin or corneal transplants, including allograft rejection or xenograft rejection, and for the prevention of graft-versus-host disease, such as following bone marrow transplant, and organ transplant associated arteriosclerosis.

In one embodiment, the antibody of the disclosure, or the pharmaceutical composition of the disclosure are particularly suitable for use in the treatment, prevention or diagnosis, in particular treatment, of an inflammatory condition or an autoimmune disease.

The antibody of the disclosure is suitable for use in the treatment, prevention, or amelioration of autoimmune disease and of inflammatory conditions, in particular inflammatory conditions with an aetiology including an autoimmune component such as arthritis (for example rheumatoid arthritis, arthritis chronica progrediente and arthritis deformans) and rheumatic diseases, including inflammatory conditions and rheumatic diseases involving bone loss, inflammatory pain, spondyloarthropathies including ankylosing spondylitis, Reiter syndrome, reactive arthritis, psoriatic arthritis, juvenile idiopathic arthritis and enteropathic arthritis, enthesitis, hypersensitivity (including both airways hypersensitivity and dermal hypersensitivity) and allergies. Specific autoimmune diseases for which antibodies of the disclosure may be employed include autoimmune haematological disorders (including e.g. hemolytic anaemia, aplastic anaemia, pure red cell anaemia and idiopathic thrombocytopenia), systemic lupus erythematosus (SLE), lupus nephritis, inflammatory muscle diseases (dermatomyosytis), periodontitis, polychondritis, scleroderma, Wegener granulomatosis, dermatomyositis, chronic active hepatitis, myasthenia gravis, psoriasis, Steven-Johnson syndrome, idiopathic sprue, autoimmune inflammatory bowel disease (including e.g. ulcerative colitis, Crohn's disease and irritable bowel syndrome), endocrine ophthalmopathy, Graves' disease, sarcoidosis, multiple sclerosis, systemic sclerosis, fibrotic diseases, primary biliary cirrhosis, juvenile diabetes (diabetes mellitus type I, type 1 diabetes), uveitis, keratoconjunctivitis sicca and vernal keratoconjunctivitis, interstitial lung fibrosis, periprosthetic osteolysis, glomerulonephritis (with and without nephrotic syndrome, e.g. including idiopathic nephrotic syndrome or minimal change nephropathy), multiple myeloma other types of tumors, inflammatory disease of skin and cornea, myositis, loosening of bone implants, metabolic disorders, (such as obesity, atherosclerosis and other cardiovascular diseases including dilated cardiomyopathy, myocarditis, diabetes mellitus type II, and dyslipidemia), and autoimmune thyroid diseases (including Hashimoto thyroiditis), small and medium vessel primary vasculitis, large vessel vasculitides including giant cell arteritis, hidradenitis suppurativa, neuromyelitis optica, Sjogren's syndrome, Behcet's disease, atopic and contact dermatitis, bronchiolitis, inflammatory muscle diseases, autoimmune peripheral neuropathies, immunological renal, hepatic and thyroid diseases, inflammation and atherothrombosis, autoinflammatory fever syndromes, immunohematological disorders, and bullous diseases of the skin and mucous membranes. Anatomically, uveitis can be anterior, intermediate, posterior, or pan-uveitis. It can be chronic or acute. The etiology of uveitis can be autoimmune or non-infectious, infectious, associated with systemic disease, or a white-dot syndrome.

In a particular embodiment, the antibody of the disclosure is suitable for use in the treatment of multiple sclerosis, psoriasis, asthma, systemic lupus erythematosus (SLE), and lupus nephritis. In another specific embodiment, the antibody of the disclosure is suitable for use in the treatment of diabetes, in particular diabetes mellitus type I or type II.

The term "a disorder mediated by IL-17A" also includes inflammation-associated cancer. In a particular embodiment, the antibody of the disclosure is suitable for use in the treatment of cancer, in particular IL-17A-mediated cancer. In a particular embodiment, the antibody of the disclosure is suitable for use in the treatment of inflammation-associated cancer.

The term "cancer" refers to a disease characterized by the rapid and uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

Non-limiting examples of inflammation-associated cancers include gastric cancers, colorectal cancers, non-small cell lung cancers, hepatocellular carcinomas and adenocarcinomas (Wu et al., 2014 Tumour Biol. 35(6):5347-56; Wu et al., 2012 PLoS One 7(12); Zhang et al. 2012 Asian Pac J Cancer Prev 13(8):3955-60; Liu et al., 201 1 Biochem Biophys Res Commun.407(2):348-54).

The antibody of the disclosure is also suitable for use in the diagnosis and/or prognosis of certain cancers, such as inflammation-associated cancers for example gastric cancers, colorectal cancers, non-small cell lung cancers, hepatocellular carcinomas and adenocarcinomas. For example, IL-17A has been linked to the prognosis and poor survival in patients suffering from non-small cell lung cancer (Chen et al., 2010 Lung Cancer 69(3):348-54), colorectal carcinoma and hepatocellular carcinoma (Punt et al., 2015 Oncoimmunology, 4(2): e984547).

Thus, in a particular embodiment, the antibody of the disclosure is suitable for use in the treatment of a cancer, arthritis, rheumatoid arthritis, osteoarthritis, reactive arthritis, psoriasis, chronic obstructive pulmonary disease, systemic lupus erythematosus (SLE), lupus nephritis, an autoimmune inflammatory bowel disease, asthma, multiple sclerosis, or cystic fibrosis, bone loss, airways hypersensitivity, a demyelinating disorder, dermal hypersensitivity, acute transplant rejection, allograft rejection, graft-versus host disease, systemic sclerosis, an urological inflammatory disorder, a cardiovascular disease, vasculitis, a periodic fever, a glucose metabolism disorder, a pulmonary disease, peridontitis, hepatic stromal keratitis, an allergy, inflammatory pain, a spondyloarthropathy, septicaemia, septic or endotoxic shock, meningitis, surgical trauma, an autoimmune haematological disorder, Alzheimer's disease, sarcoidosis, cirrhosis, hepatitis, glomerulonephritis or dislipidemia.

In one aspect, the present disclosure relates to use of the antibody of the present disclosure, or the pharmaceutical composition of the present disclosure in a manufacture of a medicament.

In another aspect, the present disclosure relates to use of the antibody of the present disclosure, or the pharmaceutical composition of the present disclosure in a manufacture of a medicament for use in the treatment, prevention or diagnosis, in particular treatment, of a disorder mediated by IL-17A or a disorder that can be treated by inhibiting Gro-α secretion. In one embodiment, the present disclosure relates to use of the antibody of the present disclosure, or the pharmaceutical composition of the present disclosure in a manufacture of a medicament for use in the treatment, prevention or diagnosis, in particular treatment, of an inflammatory condition or an autoimmune disease. In another embodiment, the present disclosure relates to use of the antibody of the present disclosure, or the pharmaceutical composition of the present disclosure in a manufacture of a medicament for use in the treatment, prevention or diagnosis, in particular treatment, of multiple sclerosis, psoriasis, asthma, systemic lupus erythematosus (SLE), and lupus nephritis. In a further embodiment, the present disclosure relates to use of the antibody of the present disclosure, or the pharmaceutical composition of the present disclosure in a manufacture of a medicament for use in the treatment, prevention or diagnosis, in particular treatment, of diabetes, in particular diabetes mellitus type I or type II. In a further embodiment, the present disclosure relates to use of the antibody of the present disclosure, or the pharmaceutical composition of the present disclosure in a manufacture of a medicament for use in the treatment, prevention or diagnosis, in particular treatment, of cancer, in particular IL-17A-mediated cancer, in a particular inflammation-associated cancer.

In another aspect, the present disclosure relates to a method for the treatment of a subject in need of therapy, characterized by administering to the subject a therapeutically effective amount of an antibody of the disclosure.

In a further aspect, the present disclosure relates to a method of treating a disorder mediated by IL-17A, said method comprising administering an effective amount of the antibody of the present disclosure, or the pharmaceutical composition of the present disclosure, such that the condition is alleviated. In one embodiment, the present disclosure relates to a method of treating an inflammatory condition or an autoimmune disease, said method comprising administering an effective amount of the antibody of the present disclosure, or the pharmaceutical composition of the present disclosure, such that the condition is alleviated. In another embodiment, the present disclosure relates to a method of treating multiple sclerosis, psoriasis, asthma, systemic lupus erythematosus (SLE), and lupus nephritis, said method comprising administering an effective amount of the antibody of the present disclosure, or the pharmaceutical composition of the present disclosure, such that the condition is alleviated. In further embodiment, the present disclosure relates to a method of treating diabetes, in particular diabetes mellitus type I or type II, said method comprising administering an effective amount of the antibody of the present disclosure, or the pharmaceutical composition of the present disclosure, such that the condition is alleviated. In further embodiment, the present disclosure relates to a method of treating cancer, in particular IL-17A-mediated cancer, in particular inflammation-associated cancer, said method comprising administering an effective amount of the antibody of the present disclosure, or the pharmaceutical composition of the present disclosure, such that the condition is alleviated.

Also within this disclosure is a kit comprising the antibody of the disclosure described herein. Also within this disclosure is a kit comprising the pharmaceutical composition of the disclosure. The kit can include one or more other elements including: instructions for use; other reagents, e.g., a label, a therapeutic agent, or an agent useful for chelating, or otherwise coupling, an antibody to a label or therapeutic agent, or a radioprotective composition; devices or other materials for preparing the antibody molecule for administration; pharmaceutically acceptable carriers; and devices or other materials for administration to a subject. In a specific embodiment, the kit comprises the antibody of the disclosure in a pharmaceutically effective amount. In a further embodiment, the kit comprises a pharmaceutically effective amount of the antibody of the disclosure in lyophilized form and a diluent and, optionally, instructions for use. Said kit may further comprise a filter needle for reconstitution and a needle for injecting.

**TABLE 1 Sequence listing.**

| **SEQ ID NO:** | **Description** | **Sequence** |
|---|---|---|
| **27-31-C04** | | |
| 1 | HCDR1 | GFSFSGFYYMC |
| | (H27-H42; AHo numbering) | |
| 2 | HCDR2 | CIDTGDGTTYYASWAKG |
| | (H57-H76; AHo numbering) | |
| 3 | HCDR3 | RDAAYGGYAYPAHGMDL |
| | (H108-H138; AHo numbering) | |
| 4 | HCDR1 (Kabat) | GFYYMC |
| 5 | HCDR2 (Kabat) | CIDTGDGTTYYASWAKG |
| 6 | HCDR3 (Kabat) | DAAYGGYAYPAHGMDL |
| 7 | HCDR1 (Chothia) | GFSFSGFY |
| 8 | HCDR2 (Chothia) | TGDG |
| 9 | HCDR3 (Chothia) | AAYGGYAYPAHGMD |
| 10 | VH | EVQLVESGGGLVQPGGSLRLSCAASGFSFSGFYYMCWVRQAPGKGLEWIGCIDTGDGTT YYASWAKGRFTISRDNSKNTVYLQMNSLRAEDTAVYYCARDAAYGGYAYPAHGMDL WGQGTLVTVSS |
| | (VH3; CDR-graft; from PRO571) | |
| 11 | VH | EVQLVESGGGLVQPGGSLTLSCAASGFSFSGFYYMCWVRQAPGKGLEWIGCIDTGDGTT YYASWAKGRFTISRDNSKNTVYLQMNSLRAEDTAVYYCARDAAYGGYAYPAHGMDL WGPGTLVTVSS |
| | (VH3; STR-graft; from | |
| | PRO592) Mutations (AHo): R20T; Q141P | |
| 12 | LCDR1 (L24-L42; AHo numbering) | QASESIYRYLS |
| 13 | LCDR2 (L58-L72; AHo numbering) | LASTLTS |
| 14 | LCDR3 (L107-L138; AHo numbering) | QSNFGTASTTYYNT |
| 15 | LCDR1 (Kabat) | QASESIYRYLS |
| 16 | LCDR2 (Kabat) | LASTLTS |
| 17 | LCDR3 (Kabat) | QSNFGTASTTYYNT |
| 18 | LCDR1 (Chothia) | SESIYRY |
| 19 | LCDR2 (Chothia) | LAS |
| 20 | LCDR3 (Chothia) | NFGTASTTYYN |
| 21 | VL (Vk1-sk12; CDR-graft; from PRO571) | DIQMTQSPSSLSASVGDRVTITCQASESIYRYLSWYQQKPGKAPKLLIYLASTLTSGVPSR FSGSGSGTDFTLTISSLQPEDFATYYCQSNFGTASTTYYNTFGGGTKLTVLG |
| 22 | VL (Vk1-sk12; STR-graft; from PRO592) | DIQMTQSPSSLSASVGDRVTITCQASESIYRYLSWYQQKPGKAPKLLIYLASTLTSGVPSR FSGSGSGTDFTLTISSLQPEDFATYYCQSNFGTASTTYYNTFGGGTKLTVLG |
| 23 | **Linker** | GGGGSGGGGSGGGGSGGGGS |
| 24 | PRO571 (scFv; VL-linker-VH) | DIQMTQSPSSLSASVGDRVTITCQASESIYRYLSWYQQKPGKAPKLLIYLASTLTSGVPSR FSGSGSGTDFTLTISSLQPEDFATYYCQSNFGTASTTYYNTFGGGTKLTVLGGGGGSGGG GSGGGGSGGGGSEVQLVESGGGLVQPGGSLRLSCAASGFSFSGFYYMCWVRQAPGKGL EWIGCIDTGDGTTYYASWAKGRFTISRDNSKNTVYLQMNSLRAEDTAVYYCARDAAYG GYAYPAHGMDLWGQGTLVTVSS |
| 25 | PRO592 (scFv; VL-linker-VH) | DIQMTQSPSSLSASVGDRVTITCQASESIYRYLSWYQQKPGKAPKLLIYLASTLTSGVPSR FSGSGSGTDFTLTISSLQPEDFATYYCQSNFGTASTTYYNTFGGGTKLTVLGGGGGSGGG GSGGGGSGGGGSEVQLVESGGGLVQPGGSLTLSCAASGFSFSGFYYMCWVRQAPGKGL EWIGCIDTGDGTTYYASWAKGRFTISRDNSKNTVYLQMNSLRAEDTAVYYCARDAAYG |
| | | GYAYPAHGMDLWGPGTLVTVSS |

| **Vλ germline-based FR4** | | |
|---|---|---|
| 26 | Vλ germline-based FR4, Sk17 | FGTGTKVTVLG |
| 27 | Vλ germline-based FR4, Sk12 | FGGGTKLTVLG |
| 28 | Vλ germline-based FR4 | FGGGTQLIILG |
| 29 | Vλ germline-based FR4 | FGEGTELTVLG |
| 30 | Vλ germline-based FR4 | FGSGTKVTVLG |
| 31 | Vλ germline-based FR4 | FGGGTQLTVLG |
| 32 | Vλ germline-based FR4 | FGGGTQLTALG |

| **IL-7 family** | | |
|---|---|---|
| 33 | IL-17A UniProt ID NO: Q16552 | MTPGKTSLVSLLLLLSLEAIVKAGIAIPQNPGCPNSEDKNFPRTVMVNLNIHNRNTNTNPK RSSDYYNRSTSPWNLHRNEDPERYPSVIWEAKCRHLGCVNADGKVDYHMNSVPIQQEIL VLRREPPHCPNSFRLEKILVSVGCTCVTPIVHHVS |
| 34 | IL-17B UniProt ID NO: Q9UHF5 | MDWPHNLLFLLTISIFLGLGQPRSPKSKRKGQGRPGPLAPGPHQVPLDLVSRMKPYARM EEYERNIEEMVAQLRNSSELAQRKCEVNLQLWMSNKRSLSPWGYSINHDPSRIPVDLPEA RCLCLGCVNPFTMQEDRSMVSVPVFSQVPVRRRLCPPPPRTGPCRQRAVMETIAVGCTCI F |
| 35 | IL-17C UniProt ID NO: Q9P0M4 | MTLLPGLLFLTWLHTCLAHHDPSLRGHPHSHGTPHCYSAEELPLGQAPPHLLARGAKWG QALPVALVSSLEAASHRGRHERPSATTQCPVLRPEEVLEADTHQRSISPWRYRVDTDEDR YPQKLAFAECLCRGCIDARTGRETAALNSVRLLQSLLVLRRRPCSRDGSGLPTPGAFAFH TEFIHVPVGCTCVLPRSV |
| 36 | IL-17D UniProt ID NO: Q8TAD2 | MLVAGFLLALPPSWAAGAPRAGRRPARPRGCADRPEELLEQLYGRLAAGVLSAFHHTL QLGPREQARNASCPAGGRPADRRFRPPTNLRSVSPWAYRISYDPARYPRYLPEAYCLCR GCLTGLFGEEDVRFRSAPVYMPTVVLRRTPACAGGRSVYTEAYVTIPVGCTCVPEPEKD ADSINSSIDKQGAKLLLGPNDAPAGP |
| 37 | IL-17E UniProt ID NO: Q9H293 | MRERPRLGEDSSLISLFLQVVAFLAMVMGTHTYSHWPSCCPSKGQDTSEELLRWSTVPV |
| | | PPLEPARPNRHPESCRASEDGPLNSRAISPWRYELDRDLNRLPQDLYHARCLCPHCVSLQ TGSHMDPRGNSELLYHNQTVFYRRPCHGEKGTHKGYCLERRLYRVSLACVCVRPRVMG |
| 38 | IL-17F UniProt ID NO: Q96PD4 | MTVKTLHGPAMVKYLLLSILGLAFLSEAAARKIPKVGHTFFQKPESCPPVPGGSMKLDIG IINENQRVSMSRNIESRSTSPWNYTVTWDPNRYPSEVVQAQCRNLGCINAQGKEDISMNS VPIQQETLVVRRKHQGCSVSFQLEKVLVTVGCTCVTPVIHHVQ |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

### EXAMPLES

### Example 1: Generation of rabbit antibodies directed against human IL-17A

### 1.1 Immunization

Rabbits have been immunized with recombinantly produced and purified IL-17A (Peprotech, Cat. No. 200-17). During the course of the immunization, the strength of the humoral immune response against the antigen was qualitatively assessed by determining the maximal dilution (titer) for the serum of each rabbit that still results in detectable binding of the polyclonal serum antibodies to the antigen. Serum antibody titers against the immobilized antigen (recombinant human IL-17A) were assessed using an enzyme-linked immunosorbent assay (ELISA).

The ability of the rabbit sera to inhibit the biological activity of IL-17A was assessed using a cell-based assay with HT-29 cells that secrete Groα upon IL-17A-stimulation. 50'000 HT-29 cells per well were seeded in 96-well plates and stimulated with 50 ng/ml IL-17A. 5-fold serial dilutions of final bleeds of IL-17A immunized rabbits were tested for the potency to neutralize biological function of IL-17A. All five sera inhibited GROα secretion of HT-29 cells. Lymphocytes were isolated from spleens of immunized animals and were a subjectfor the subsequent Hit Identification procedures.

### 1.2 Identification of Anti-IL-17A Antibodies

### 1.2.1 Isolation of B Cells Expressing Anti-IL-17A Antibodies

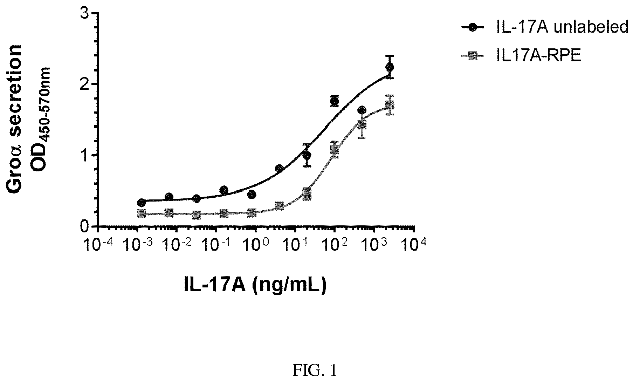

To identify IL-17A binding B-cells, IL-17A was labeled with R-Phycoerythrin (RPE). Since the IL-17 receptor A binding site on the labeled IL-17A could potentially be blocked by the bulky RPE label, accessibility of the epitope was confirmed by two approaches. In a first assay, binding of RPE labeled IL-17A to secukinumab and IL-17 receptor A was analyzed using flow-cytometry. Secukinumab and IL-17 receptor A extracellular domain fused to the Fc part of a human IgG1 were captured on protein G beads, and binding of RPE labeled IL-17A was confirmed by flow-cytometry. Binding of IL-17A to secukinumab and IL-17 receptor A Fc chimera was thereby confirmed while no binding to an unrelated IgG or cytokine receptor was detected. In a second approach, biological activity of the labeled IL-17A was confirmed in the HT-29 assay. As shown in FIG. 1, RPE labeled IL-17A showed only slightly reduced biological activity when compared to unlabeled IL-17A (1.5-fold higher EC₅₀ for induction of IL-17A-dependent GROα secretion). Therefore, it was confirmed that the labeled IL-17A is suitable for use in the sorting process.

### 1.2.2 Screening of IL-17A-Binding Supernatants

The results obtained during the screening phase are based on assays performed with non-purified antibodies from culture supernatants of antibody secreting cells (ASC). ASC supernatants were screened in a high-throughput ELISA for binding to recombinant human IL-17A. IL-17A-binding supernatants were further characterized for their binding kinetics and for their potential to neutralize the biological activity of IL-17A in the cell-based HT-29 assay and a competition ELISA. IL-17A-binding supernatants were further characterized for binding to Cynomolgus monkey IL-17A by ELISA.

### 1.2.2.1 IL-17A binding by ELISA

To identify B-cell clones that produce antibodies binding human IL-17A, cell culture supernatants of B-cell clones were analyzed for the presence of antibodies to human IL-17A by ELISA. The ELISA method used assesses the "quantity" of antibodies of the IgG subtype bound to recombinant human IL-17A, gives however no information about the affinity or the concentration of the antibodies.

### 1.2.2.2 Affinity to human IL-17A

Binding affinities of monoclonal rabbit antibodies toward human IL-17A from culture supernatants that qualified positive during the primary screening were determined by surface plasmon resonance (SPR) using a MASS-1 SPR device (Sierra Sensors). For affinity screening, an antibody specific for the Fc region of rabbit IgGs was immobilized on a sensor chip (SPR-2 Affinity Sensor, High Capacity Amine, Sierra Sensors) using a standard amine-coupling procedure. Rabbit monoclonal antibodies in B-cell supernatants were captured by the immobilized anti-rabbit IgG antibody. A minimal IgG concentration in the B-cell supernatants is required to allow sufficient capture. After capturing of the monoclonal antibodies, human IL-17A was injected into the flow cells for 3 min at a concentration of 90 nM, and dissociation of the protein from the IgG captured on the sensor chip was allowed to proceed for 5 min. The apparent dissociation (kd) and association (ka) rate constants and the apparent dissociation equilibrium constant (K_{D}) were calculated with the MASS-1 analysis software (Analyzer, Sierra Sensors) using one-to-one Langmuir binding.

### 1.2.2.3 Neutralization of IL-17A by the HT-29 assay and a competition ELISA

For the assessment of potency, a cell-based assay (HT-29 assay), as well as a receptor ligand competition-ELISA, were developed.

The ability of the antibodies in B cell supernatant to inhibit the biological activity of IL-17A was assessed using a cell-based assay with HT-29 cells that secrete Groα upon IL-17A stimulation. 50'000 HT-29 cells per well were seeded in 96-well plates and stimulated with 5 ng/ml IL-17A. B cell supernatants at a final concentration of 50% were analyzed for the potency to neutralize biological function of IL-17A.

The inhibition of hIL-17A binding to hIL-17RA was assessed by competitive ELISA. hIL-17RA was coated on the ELISA plate at a concentration of 4 µg/ml. Biotinylated hIL-17A (20 ng/ml) was preincubated with B cell supernatants (95%) for 1 h, the mixture was then added to the ELISA plate to allow binding to the hIL-17RA for 1.5 h. Then, Streptavidin-HRP, used to Biotinylated IL-17A was detected by Streptavidin-HRP.

### 1.2.2.4 Species cross-reactivity (binding to Cynomolgus monkey IL-17A by SPR)

Selected hits were analyzed for species cross-reactivity to cynomolgus monkey IL-17A by SPR. Binding affinities were determined by surface plasmon resonance (SPR) using a MASS-1 SPR device (Sierra Sensors) similarly as described in section 1.2.2.2 for human IL-17A with the exception that 90 nM cynomolgus monkey IL-17A was used instead of human IL-17A.

### 1.2.2.5 IL-17F binding by ELISA

To confirm specificity of the anti-IL-17A scFvs, binding to all the IL-17 family members (IL-17B, IL-17C, IL-17D, IL-17F and IL-17E) was assessed for the best performing humanized scFvs. The sort supernatants were screened in ELISA for binding to IL-17F because IL-17F shows the highest identity to IL-17A (47%, see

TABLE 2). Binding was quantified relative to the signal obtained with 2 µg/ml secukinumab.

**TABLE 2. Identity between IL-17A and the other members of the IL-17 family. Data was obtained by aligning the IL-17A sequence (UniProtKB/Swiss-Prot: Q16552.1) to IL-17B, IL-17C, IL-17D, IL-17F and IL-17E using EMBOSS NEEDLE (with std settings).**

| **IL-7 Family Member** | **Identitiy to IL-17A** | **Similarity to IL-17A** |
|---|---|---|
| IL-17B | 26.6 | 36.4 |
| IL-17C | 23.4 | 31.8 |
| IL-17D | 21.8 | 27.5 |
| IL-17E | 21.3 | 31.7 |
| IL-17F | 47.2 | 62 |

### 1.3 Functional Characterization

Based on pharmacologic properties of monoclonal antibodies in B-cell supernatant a number of clones were selected for hit confirmation analysis. Pharmacologic properties of monoclonal antibodies of the selected clone in B-cell supernatant are presented in TABLE 3.

### 1.3.1 IL-17A Binding Kinetics (by SPR)

Binding kinetics of a number purified monoclonal rabbit antibodies to human IL-17A were determined by SPR (Mass-1) measurements. Each IgG was captured via an anti-rabbit IgG (Bethyl Laboratories, Cat. No. A120-111A) coupled to a carboxylmethylated dextran surface (SPR-2 Affinity Sensor, High Capacity Amine, Sierra Sensors) and an IL-17A dose response was measured to allow accurate fitting of kinetic parameters. After capturing of the monoclonal antibodies, human IL-17A (Peprotech, Cat. No. 200-17) was injected into the flow cells for 3 min and dissociation of the protein from the IgG captured on the sensor chip was allowed to proceed for 5 min. After each injection cycle, surfaces were regenerated with two injections of 10 mM Glycine-HCl. The apparent dissociation (kd) and association (ka) rate constants and the apparent dissociation equilibrium constant (K_{D}) were calculated with the MASS-1 analysis software (Analyzer, Sierra Sensors) using one-to-one Langmuir binding model and quality of the fits was monitored based on relative Chi2 (Chi2 normalized to the extrapolated maximal binding level of the analyte), which is a measure for the quality of the curve fitting.

Monoclonal antibodies of the selected clone 27-31-C04 have a dissociation constant (K_{D}) of less than 0.5 pM, ka for on-rates of 1.99 x 10⁶ M⁻¹s⁻¹, and kd for off-rates less than 1 x 10⁻⁶ s⁻¹ as measured by SPR (see TABLE 3).

### 1.3.2 Cross-reactivity to cynomolgus monkey IL-17A (by SPR)

Species cross-reactivity for cynomolgus monkey IL-17A was determined for the selected purified monoclonal rabbit antibodies by SPR (Mass-1) measurements similarly as described above. Trenzyme company custom-produced cynomolgus monkey IL-17A was used in the assays. Monoclonal antibodies of the selected clone 27-31-C04 had a dissociation constant (K_{D}) of less than 25 pM, ka for on-rates of 1.69 x 10⁶ M⁻¹s⁻¹, and kd for off-rates 4.22 x 10⁻⁵ s^{- 1} as measured by SPR (see TABLE 3). The ration of K_{DcynoIL-17A}/K_{DhumanIL-17A} as measured by SPR was determined to be more than 5 (see TABLE 3).

### 1.3.3 Neutralization of human IL-17A (by the HT-29 assay)

The effects of purified rabbit monoclonal antibodies on IL-17A-induced GROα secretion of the human colon carcinoma cell line HT-29 was examined. The potency (IC₅₀) to neutralize IL-7A-induced GROα secretion (as quantified by a commercial ELISA) was analyzed for serial dilutions of all antibodies and compared to the potency of secukinumab. Data were analyzed using a four-parameter logistic curve fit, and the molar concentration of IL-17A inhibitor required to reduce GROα secretion to 50% (IC₅₀) was derived from inhibition curves. In order to render IC₅₀ values from different assay plates directly comparable to each other, individual IC₅₀ values on each plate were calibrated against the IC₅₀ of the reference molecule secukinumab that was taken along on each plate (relative IC₅₀: IC₅₀, _{secukinumab}/IC₅₀, _{test antibody}). Relative IC₅₀ values were calculated in mass units (ng/ml) of secukinumab and the scFvs.

Neutralization assays can distinguish potencies of target blocking antibodies only if they bind their target with an equilibrium binding constant (K_{D}) that is higher than the target concentration used in the potency assay (K_{D} > target concentration). For the HT-29 assay an IL-17A concentration of 20 ng/ml (= 645 pM) was used. Therefore, theoretically, the HT-29 assay can differentiate potencies between IgGs with K_{D} > 645 pM. Since all of the IgGs analyzed showed K_{D} values below 645 pM, potencies between IgGs with different affinities (but similar mechanism of action) cannot be differentiated with this. Thus, the HT-29 assay was further developed to include 50 pg/ml TNFα in order to better resolve potencies. This cytokine acts synergistically with IL-17A to induce GROα secretion, and as a result the IL-17A concentration could be reduced to 1 ng/ml (= 32 pM) in the presence of TNFα.

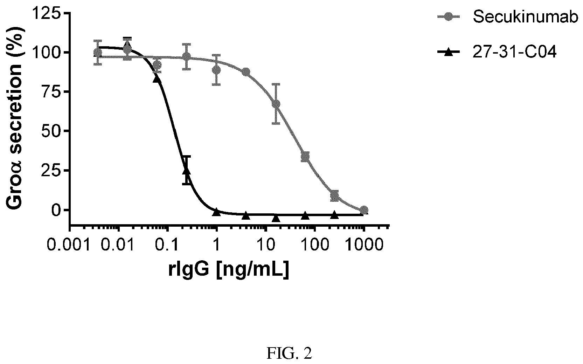

The selected clone 27-31-C04 showed a potency above 100-fold of secukinumab (see TABLE 3, FIG. 2). More specifically, the selected clone 27-31-C04 was shown to have a potency to neutralize IL-17A relative to that of secukinumab (relative potency), determined in an HT-29 assay, of 287 for IC₅₀ and 704 for IC₉₀, and wherein said relative potency is the ratio of the IC₅₀ or IC₉₀ value in ng/mL of secukinumab in the HT-29 assay to the IC₅₀ or IC₉₀ value in ng/mL of the monoclonal antibody of the clone 27-31-C04 in the HT-29 assay (see TABLE 3, FIG. 2).

### 1.3.4 Blocking of the human IL-7A/IL-7RA interaction (by competitive ELISA)

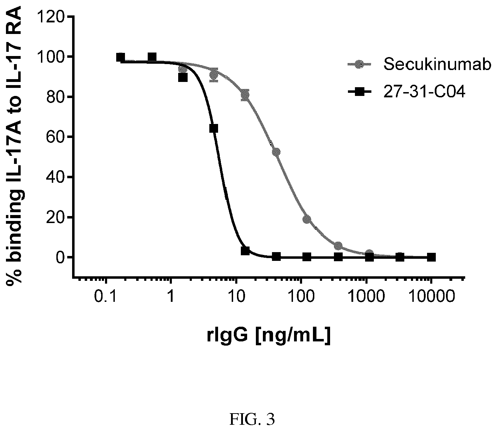

The inhibition of hIL-17A binding to hIL-17RA was assessed by competitive ELISA. hIL-17RA was coated on the ELISA plate by adding 50 µl of PBS containing 4 µg/ml IL-17RA (Sino Biological Cat. No. 10895-H08H) ON at 4°C. Biotinylated hIL-17A was preincubated with the rabbit monoclonal anti-bodies for 1 h, the mixture was then added to the ELISA plate to allow binding to the hIL-17RA for 1.5 h. Then, 50 µl of 10 ng/ml streptavidin-polyHRP40 (SDT Cat. No. SP40C), used to detect the biotinylated IL-17A, was added and the plate was incubated for 1 h. Finally, Tetramethylbenzidin solution (KPL, Cat. No. 53-00-00) was added to develop the plate for 5 to 10 minutes and the reaction was stopped with 1 M HCl. The plate was read using a microtiter plate reader (Infinity reader M200 Pro, Tecan) at a wavelength of 450 nm and 570 nm (reference wavelength). Dose-response curve obtained for the selected rabbit IgG is represented in FIG. 3. IC₅₀ values and IC₉₀ values compared to the reference secukinumab are summarized in TABLE 3. As mentioned in the previous section, neutralization assays can distinguish potencies of target blocking antibodies only if they bind their target with an equilibrium binding constant (K_{D}) that is higher than the target concentration used in the potency assay (K_{D} > target concentration). For the HT-29 assay a IL-17A concentration of 32 pM was used while in the IL-7A/IL-7RA inhibition ELISAs a IL-17A concentration of 193 pM was used. Therefore, theoretically, the HT-29 assay can differentiate potencies between IgGs with K_{D} > 32 pM, while the inhibition ELISA can only differentiate potencies between IgGs with K_{D} > 193 pM. Like in the HT-29 assay, clone 27-31-C04 showed a higher potency than secukinumab. The clone 27-31-C04 was shown to have a potency to block IL-17A/IL-17RA interaction relative to that of secukinumab (relative potency), determined in an ELISA assay, of 8 for IC₅₀ and 21 for IC₉₀, and wherein said relative potency is the ratio of the IC₅₀ or IC₉₀ value in ng/mL of secukinumab in the ELISA assay to the IC₅₀ or IC₉₀ value in ng/mL of the monoclonal antibody of the clone 27-31-C04 in the ELISA assay (see TABLE 3).

### 1.3.5 Cross-reactivity to cynomolgus monkey IL-17A (by HT-29 assay)

50'000 HT-29 cells were plated in each well of a 96-well plate. In addition to serial dilutions of rabbit monoclonal antibodies and the internal reference secukinumab, pre-dilutions of human TNFα (50 pg/ml) and human or cynomolgus monkey IL-17A (1 ng/ml), respectively, were added to the HT-29 cells. After 24 h incubation at 37°C and 5% CO2, supernatants were collected and GROα (CXCL1 chemokine) secretion was quantified by ELISA. In order to render IC₅₀ values obtained with cynomolgus IL-17A directly comparable to each other, individual IC₅₀ values on each plate were calibrated against the IC₅₀ obtained with human IL-17A that was taken along on each plate (relative IC₅₀: IC₅₀, cynomolgus _{IL-7A}/IC₅₀, _{human IL-7A}). Potency of the selected clone is presented in TABLE 3. For the clone 27-31-C04 the potency (IC₅₀) to cynomolgus IL-17A-induced GROα secretion was determined to be 0.49 ng/ml (see TABLE 3).

**TABLE 3. Pharmacodynamic properties of clone 27-31-C04 monoclonal rabbit anti-IL-7A IgG selected for immunization.**

| **IgG** | **Affinity for human IL-17A (SPR)** | | | **Affinity for cynomolgus IL-17A (SPR)** | | | **Ratio IL-17A binding SPR (KD_{cynoIL-17A})** /(KD_{human IL-17A}) | **Potency in HT-29 assay** | | **Potency in IL17AIL17RA ELISA** | | **HT-29 assay with human and cyno IL17A** | | | **Relative binding to IL. 17F (ELISA)** |
|---|---|---|---|---|---|---|---|---|---|---|---|---|---|---|---|
| Clone ID | kₐ[M⁻¹s⁻¹] | k_{d} [s⁻¹] | K_{D}[M] | kₐ[M⁻¹s⁻¹] | k_{d}[s⁻¹] | K_{D}[M] | | rel. IC₅₀* ICⱼₒ^{*} | rel. IC₉₀* | rel. IC_{M}* | rel. IC₉₀* | IC₅₀ [ng/mL] human IL17A | IC₅₀ [ng/mL] cyno IL17A | rel. IC₅₀** | Rel. OD₍₄₅₀₋₆₉₀ₙₘ₎ [OD,_{IL17F} /OD,_{IL17A}] |
| 27-31-C04 | 1.99E+06 | <1E-06 | <5.03E-13 | 1.69E+06 | 4.22E-05 | 2.50E-11 | >5.0 | 286.79 | 703.62 | 7.93 | 21.31 | 0.41 | 0.49 | 1.19 | 19% |

| | | | | | | | | | | | | | | | |
|---|---|---|---|---|---|---|---|---|---|---|---|---|---|---|---|
| *: ICₓₓ, Secukinumab/ICₓₓ, Test Sample **: ICₓₓ, cyno IL17A/ICₓₓ, human IL17A | | | | | | | | | | | | | | | |

**TABLE 4. Binding of rabbit monoclonal antibodies to IL-17F. The OD(450-690nm) for IL-17F binding by IgG (10 µg/ml) is calculated relative to a) OD(450-690nm) for secukinumab binding and b) OD(450-690nm) for IL-17A binding.**

| **IgG** | **Relative binding IL-17F compared to secukinumab at 10 µg/ml** | **Relative binding IL-17F compared to IL-17A at 10 µg/ml** |
|---|---|---|
| Clone ID | Binding IL-7F rel. to 10 µg/ml secukinumab (%) | OD_{IL-17F}/OD_{IL-17A} (%) |
| 27-31-C04 | 19.7% | 19.1% |

### 1.3.6 Selectivity for IL-17A versus IL-17F

Selectivity of IgG binding to IL-17A over other IL-17 family members was determined to ensure target-specific binding. To determine the binding of anti-IL-17A IgG to IL-17F a direct ELISA was developed in which the plate is coated with IL-17A or IL-17F (Peprotech Cat. No. 200-25) and incubated with 10 µg/ml recombinant anti-IL-17A IgG (including secukinumab as control). The OD(450-690nm) for IL-17F binding is calculated relative to a) OD(450-690nm) for IL-17A binding and b) OD(450-690nm) for secukinumab binding. Data is summarized inTABLE 4.

### Example 2: Humanization and Generation of scFv

### 2.1 Generation of Humanized scFv Antibodies

Based on data obtained during hit confirmation, clone 27-31-C04 was selected for humanization. The humanization of the selected clone comprised the transfer of the rabbit CDRs onto an scFv acceptor framework of the Vκ1-λcap/VH3 type as described in WO 2014/206561. In this process, the amino acid sequence of the six CDR regions was identified on the donor sequence (rabbit mAb) and grafted into the acceptor scaffold sequence, resulting in the constructs termed "CDR graft" (see SEQ ID NO: 24, PRO571). In addition, a second graft was designed for clone 27-31-C04 (SEQ ID NO: 25, PRO592), which included additional amino acids modifications from the rabbit donor in certain framework positions, which have been described to potentially influence CDR positioning and thus antigen binding and/or stability (Borras et al., 2010; J. Biol. Chem., 285:9054-9066). These humanized construct are termed "structural (STR) graft". Further, two mutations, namely R20T and Q141P (according to AHo numbering) were introduced in variable heavy chain of the structure-based graft SEQ ID NO: 25, PRO592) in order to improve affinity.

In case the comparison of the characterization data for these constructs revealed a significant advantage of the STR constructs additional variants can be designed that combine the CDR grafted VL with STR grafted VH. This combination has been proven to be often sufficient to retain the activity of the STR graft (Borras et al. JBC. 2010;285:9054-9066) and would generally be preferred as fewer non-human alterations in the human acceptor scaffold reduce the risk for impaired stability and also the potential for immunogenicity.

### 2.2 Manufacture of Humanized scFv

Once the in-silico construct design was completed, the corresponding genes were synthesized and bacterial expression vectors were constructed. The sequence of the expression constructs was confirmed on the level of the DNA and the constructs were manufactured according to generic expression and purification protocols.

The heterologous expression of the proteins was performed in *E.coli* as insoluble inclusion bodies by induced overnight expression in small scale (55 mL). The inclusion bodies were isolated from the homogenized cell pellet by a centrifugation protocol that included several washing steps to remove cell debris and other host cell impurities. The purified inclusion bodies were solubilized in a denaturing buffer (100 mM Tris/HCl pH 8.0, 6 M Gdn-HCl, 2 mM EDTA) and the scFvs were refolded by a scalable refolding protocol that generated milligram amounts of natively folded, monomeric scFv. At this point a standardized protocol was employed to purify the scFvs. The product after refolding was captured by an affinity chromatography to yield the purified scFvs. In addition, melting temperatures of scFvs were determined by differential scanning fluorimetry (DSF) measurement in order to support the selection of molecules entering more extensive stability assessment after completion of pharmacodynamic characterization described in the following paragraphs. DSF measurement of selected molecules is shown in more detail under paragraph 2.4.2.

### 2.3 Functional Characterization of humanized scFvs

In the following the humanized scFvs were characterized for the primary pharmacodynamics properties, using the same assay systems as described for the Hit confirmation phase, with certain adaptations though to accommodate for the different format of the scFv molecules.

### 2.3.1 Affinity to human IL-17A and to cynomolgus monkey IL-17A

Affinity of humanized scFvs to human IL-17A as well as Cynomolgus monkey (Macaca fascicularis) IL-17A was determined by SPR analysis on a T200 device (Biacore, GE Healthcare). In this experiment, biotinylated human IL-17A was captured using the Biotin-CAPture kit from Biacore ("capture setup"). After each analyte injection cycle the CAP sensor chip was regenerated and new antigen was captured. The scFvs were injected as analyte using a dose response multi-cycle kinetic assay with concentrations of the analyte ranging from 0.35 to 90 nM diluted in running buffer. Obtained sensorgrams were fitted using the 1:1 binding model. In addition, the scFvs were analyzed in an alternative SPR assay setup ("direct setup"). IL-17A was immobilized on a CM5 sensor chip (GE Healthcare) by amine-coupling. Serial dilutions of scFvs ranging from 0.35 to 90 nM were injected over the immobilized IL-17A.When using the direct setup affinities were 2- to more than 50-fold higher for the scFv tested compared to the capture setup. The lower affinities using the capture setup could be due to interference of biotin with binding of the scFvs.

Cross-reactivity to cynomolgus IL-17A was measured with the IL-17A produced by Trenzyme in a similar assay as used to measure binding to human IL-17A.

The scFvs PRO571 (CDR) and PRO592 (STR) bound to human IL-17A with affinities of 1.2 x 10⁻¹⁰ and 3.7 x 10⁻¹⁰ M, respectively (TABLE 5), as measured in the SPR capture setup. In the direct SPR setup, the scFvs PRO571 (CDR) and PRO592 (STR) bound to human IL-17A with affinities of 4 x 10⁻¹¹ and 3.8 x 10⁻¹¹ M, respectively (TABLE 5). The scFvs PRO571 (CDR) and PRO592 (STR) bound to Cynomolgus IL-17A with affinities of 3.3 x 10⁻¹¹ and 1.6 x 10⁻¹⁰ M, respectively (TABLE 5).

### 2.3.2 HT-29 assay (neutralization of human IL-17A)

The HT29 assay tested the ability of the humanized scFvs to inhibit IL-17A-induced GROα secretion of the human colon carcinoma cell line HT-29. As described above in sections 1.3.3 and 1.3.5 TNFα was added in addition to IL-17A to increase the sensitivity of the assay. 50'000 HT-29 cells were plated in each well of a 96-well plate. In addition to the serially diluted scFvs as well as the internal reference secukinumab, pre-dilutions of human TNFα (50 pg/ml) and human IL-17A (1 ng/ml) were added to the HT-29 cells. After 24 h incubation at 37°C and 5% CO2, supernatants were collected and GROα (CXCL1 chemokine) secretion was quantified by ELISA.

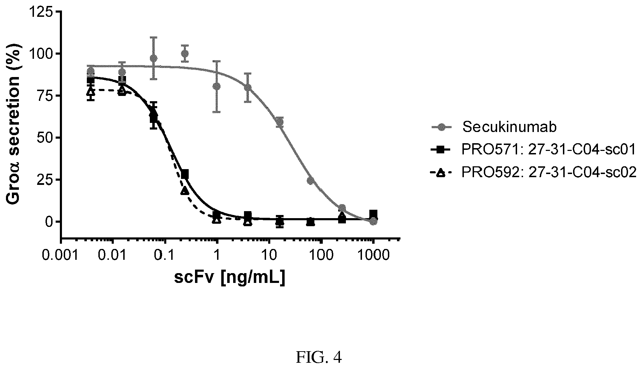

Inhibition curves for the humanized scFvs PRO571 and PRO592 are shown in FIG. 4. All potency data are summarized in TABLE 5 and TABLE 6. The relative IC₅₀ values reported for each molecule were calibrated to the reference antibody secukinumab to allow for direct comparison of IC₅₀ values from different assay plates. Relative IC₅₀ values were calculated in mass units (ng/ml) of secukinumab and the scFvs. The humanized scFvs derived from 27-31-C04 antibodies, PRO571 and PRO592, inhibited IL-17A-induced GROα secretion with lower IC₅₀ than secukinumab (see TABLE 5 and TABLE 6). The humanized scFvs derived from 27-31-C04 antibodies, PRO571 and PRO592, show more than 100-fold higher potency compared to secukinumab.

**TABLE 5. Pharmacodynamic properties of the humanized scFvs.**

| Protein number | scFv | Affinity to human IL17A (Capture setup) | | | Affinity to human IL17A (Direct setup) | | | Affinity to cyno IL17A | | | K_{D,cyno}/K_{D, human} | Neutralization of IL17A in HT-29 assay | Blocking of IL17AIL17RA interaction |
|---|---|---|---|---|---|---|---|---|---|---|---|---|---|
| | Clone ID | ka(M⁻¹ s⁻¹) | kd (s⁻¹) | K_{D}(M) | ka(M⁻¹s⁻¹) | kd (s⁻¹) | K_{D}(M) | ka (M⁻¹ s⁻¹) | kd (s⁻¹) | K_{D}(M) | | rel. IC₅₀* | rel. IC₅₀* |
| PRO571 | 27-31-C04-sc01 | 1.21E+06 | 1.44E-04 | 1.19E-10 | 1.13E+06 | 4.55E-05 | 4.03E-11 | 1.17E+06 | 3.88E-05 | 3.31E-11 | 0.28 | 190.8 | 22.9 |
| PRO592 | 27-31-C04-sc02 | 3.87E+05 | 1.42E-04 | 3.67E-10 | 1.08E+06 | 4.07E-05 | 3.76E-11 | 3.36E+05 | 5.38E-05 | 1.6E-10 | 0.44 | 196.0 | 40.9 |

| | | | | | | | | | | | | | |
|---|---|---|---|---|---|---|---|---|---|---|---|---|---|
| *: IC_{50, secukinumab}/IC_{50, Test Sample} | | | | | | | | | | | | | |

**TABLE 6. Potencies of anti-IL-17A scFv to inhibit IL-17A-induced GROα secretion of the human colon carcinoma cell line HT-29.**

| Protein ID | scFv | Potency in HT-29 assay | |
|---|---|---|---|
| | Clone ID | IC₅₀ [ng/mL] | rel. IC₅₀* |
| PRO571 | 27-31-C04-sc01 | 0.1 | 190.8 |
| PRO592 | 27-31-C04-sc02 | 0.13 | 196.0 |

| | | | |
|---|---|---|---|
| *: IC_{50, secukinumab}/IC_{50, Test Sample} | | | |

### 2.3.3 Competitive ELISA (inhibition of hIL-17A binding to hIL-7-RA)

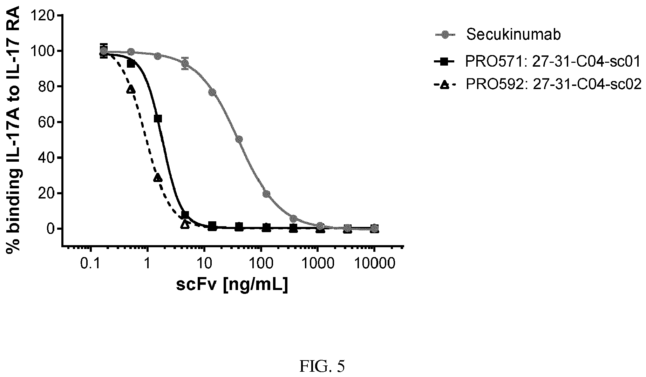

In addition to the HT-29 assay, the potency of each humanized scFv to inhibit the interaction between human IL-17A and human IL-17RA was assessed by ELISA with the same procedure as described in section 1.3.4. Similarly to the HT-29 assay, individual IC₅₀ values on each plate are calibrated against the IC₅₀ of the reference molecule secukinumab that is taken along on each plate and relative IC₅₀ (relative IC₅₀: IC_{50, secukinumab}/IC_{50, test antibody}) (TABLE 5 and **Fehler! Ungültiger Eigenverweis auf Textmarke.**). In this assay 6 ng/ml IL-17A is implemented which means that the assay can only resolve potencies between IgGs with K_{D} > 193 pM. Inhibition curves for PRO571 and PRO592 are shown in FIG. 5 and potencies are summarized in TABLE 5 and **Fehler! Ungültiger Eigenverweis auf Textmarke..**

**TABLE 7. Potencies of anti-IL-17A scFv to block of the human IL-17A/IL-17RA interaction (ELISA).**

| Protein ID | scFv | Potency in IL-7A/IL-7RA ELISA | |
|---|---|---|---|
| | Clone ID | IC₅₀ [ng/mL] | rel. IC₅₀* |
| PRO571 | 27-31-C04-sc01 | 1.8 | 22.9 |
| PRO592 | 27-31-C04-sc02 | 0.9 | 40.9 |

| | | | |
|---|---|---|---|
| *: IC₅₀, _{secukinumab}/IC_{50, Test Sample} | | | |

### 2.3.4 Selectivity against IL-7B, IL-7C, IL-7D, IL-7E and IL-7F (competition ELISA)

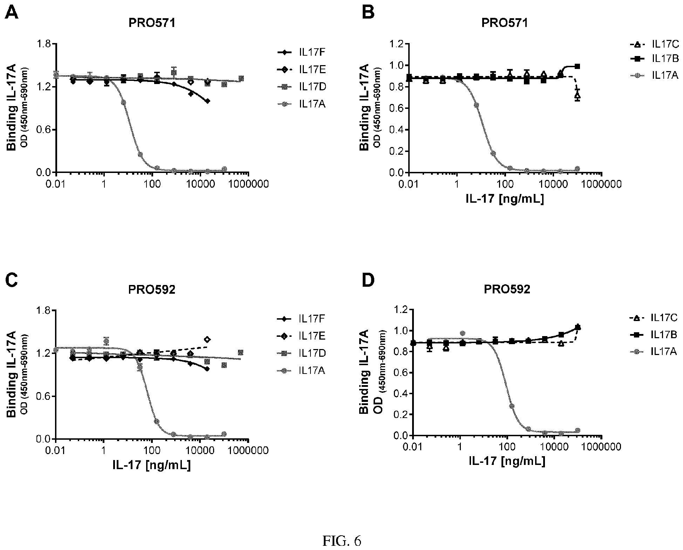

The relative potential of IL-17B to F, as compared to IL-17A to half-maximally inhibit IL-7A binding to each scFv was assessed by competition ELISA. The potential to inhibit the interaction of biotinylated IL-17A with scFvs by unlabeled IL-17B, IL-17C, IL-17D, IL-17E and IL-17F was analyzed by competition ELISA. For this purpose, the scFvs were coated on a 96-well ELISA plate. Binding of biotinylated IL-17A to the coated scFvs in presence of serially diluted IL-17A or IL-17B to IL-17F was detected using the biotin-binding streptavidin conjugated to HRP. For the dose-response curve with IL-17A data were analyzed using a four-parameter logistic curve fit, and the concentration of IL-17A required to block the interaction of biotinylated IL-17A with the coated scFv by 50% (EC₅₀) was calculated. IL-17B to IL-17F did not show any significant inhibition of the interaction between biotinylated TNF and scFvs (see FIG. 6). To quantify the relative potential of these IL-17 family members as compared to IL-17A to inhibit IL-17A binding to each scFv the EC₅₀ to inhibit the interaction by IL-17 family members relative to IL-17A was calculated. Since no significant inhibition was observed when using IL-17 members at an approximately 100 to 40'000-fold higher concentration than the EC₅₀ of IL-17A, the selectivity for binding to IL-17A over IL-17 family members was determined to be significantly higher than 100 to 40'000-fold.

As shown in FIG. 6 and summarized TABLE 8 for PRO571 and PRO592, the interaction between the scFv PRO571 and PRO592 with biotinylated IL-17A was blocked by unlabeled IL-17A, while IL-17B, IL-17C, IL-17D, IL-17E and IL-17F did not show any significant effect even at the highest concentration of cytokine tested (20 to 500 µg/ml). Hence, PRO571 and PRO592 bind specifically to IL-17A but not to its closest homologues, IL-17B to IL-17F. Therefore, off-target binding of the scFvs described herein appears highly unlikely.

### 2.4 Biophysical Characterization of the humanized scFvs

### 2.4.1 Manufacture of stability material

The scFvs were produced again using the same manufacture process as described above (see section 2.2) at higher scale (1.2L-2.4 L expression volume). Additionally, protein samples were concentrated to >10 mg/mL using centrifugal concentration tubes after purification. High scale manufacture of material for stability assessment is compiled in TABLE 9. All molecules could be produced in sufficient amounts and purity (>95% monomeric).

The producibility of the scFv constructs was characterized by different reporting points (TABLE 9). The producibility criterion shall ensure that the selected scFv entity can be expressed, refolded and purified in sufficient amounts to support later development of the lead molecule. The defined criteria were the expression yield of scFv per liter of fermentation broth, as assessed by SDS-PAGE, and the purification yield achieved in the generic lab-scale process, as assessed by measurement of the amount of purified protein by UV spectrometry, calculated back to 1 liter of refolding solution.

The expression titer was determined on the level of the crude E.coli lysate after the harvest of the cells by centrifugation. During the harvest a small loss of cells was anticipated, however, this factor was neglected for the calculation of the expression yield in favor of a more conservative estimation of the productivity. For the quantification of the scFv product in the lysate coomassie stained reducing SDS-PAGE was chosen due to the high specificity of the method that allows discriminating the product from the host cell proteins in the sample.

A second criterion to assess the producibility was the purification yield of scFv calculated per liter of refolding solution. This parameter addresses the potential bottleneck in the anticipated manufacturing process that includes a protein refolding step. Since the efficiency of the refolding procedure has proven to be limiting in comparable manufacturing processes the performance of the different constructs can be compared with respect to the producibility normalized to a defined refolding volume. For the calculation of the yield the final protein sample from each batch was quantified by UV absorbance and divided by the actual refolding volume of the respective purification.

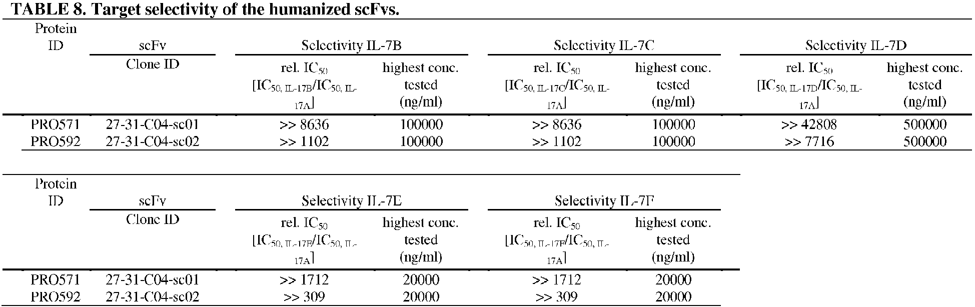

**TABLE 9. Manufacture of material for stability assessment.**

| Protein ID | Construct ID | Expression volume [mL] | Yield post Capto L [mg] | Final yield [mg] | Yield per L refolding [mg/L] | Yield per L expression [mg/L] | Purity SE-HPLC [% monomer] |
|---|---|---|---|---|---|---|---|
| PRO592 | 27-31-C04-sc02 | 1200 | 74.0 | 51.7 | 25.8 | 43.1 | 97.6 |
| PRO571 | 27-31-C04-sc01 | 2400 | 124.0 | 88.5 | 22.1 | 36.9 | 98.9 |

### 2.4.2 Thermal unfolding

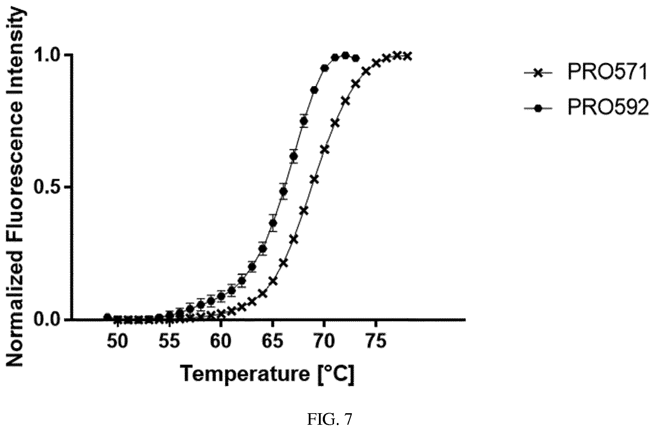

Thermal unfolding curves obtained from DSF measurements of the scFvs constructs PRO571 and PRO592 are presented in FIG. 7. For DSF measurement material produced in small scale expression was used. Samples in 50mM phosphate-citrate buffer at pH6.4 were prepared at a final protein concentration of 50 µg/mL and a final concentration of 5x SYPRO® Orange in a total volume of 100 µl. Twenty-five microliters of prepared samples were added in triplicate to white-walled AB gene PCR plates. The assay was performed in a qPCR machine used as a thermal cycler, and the fluorescence emission was detected using the software's custom dye calibration routine. The PCR plate containing the test samples was subjected to a temperature ramp from 25°C to 96°C in increments of 1°C with 30 s pauses after each temperature increment. The total assay time was about two hours. The Tm was calculated by the software GraphPad Prism using a mathematical second derivative method to calculate the inflection point of the curve. Measurements were done in triplicates as shown by error bars. Resulting Tm values have been determined by fitting of data to a Boltzmann equation.

FIG. 7 shows melting curve of selected domain which was used for calculation of Tm by fitting the data to a Boltzmann equation. TABLE 10 summarizes calculated melting temperature and purities measured by SE-HPLC of PRO571 and PRO592.

**TABLE 10. Monomeric content and Tm of selected domains.**

| **Protein ID** | **Purity SE-HPLC [% monomer]** | **Tm [°C]** |
|---|---|---|
| PRO592 | 99.7 | 66.1 |
| PRO571 | 98.9 | 68.8 |

### 2.4.3 Storage stability study

The scFvs PRO571 and PRO592 were subjected to further stability studies such as a four-week stability study, in which the scFv was be formulated in an aqueous buffer at 10 mg/ml and stored at < -65°C, 4°C and 37°C for four weeks. At one week, two weeks and at the end of each study, the fraction of monomers and oligomers in the formulation were evaluated by integration of SE-HPLC peak areas. concentration of >10 mg/mL.

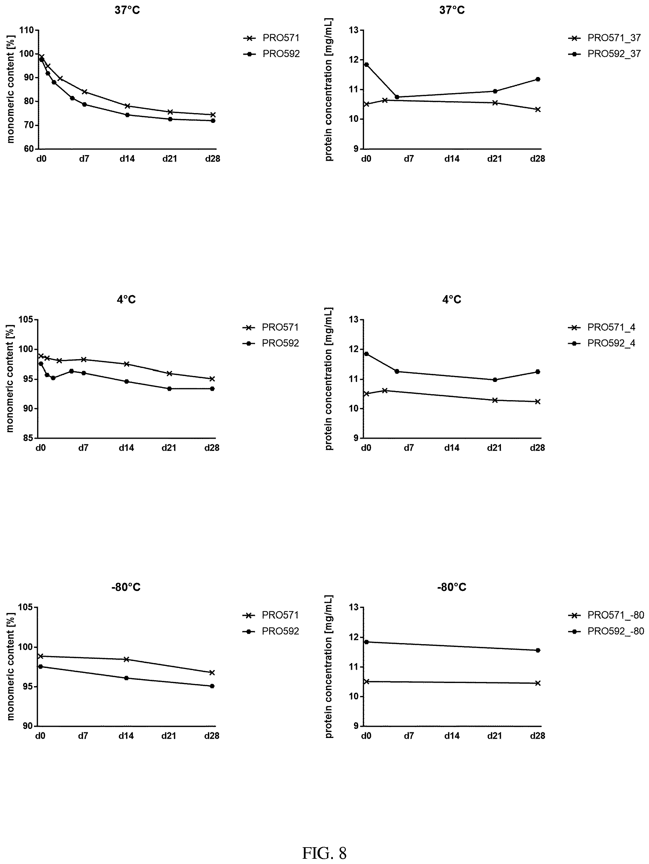

TABLE 11 compares endpoint measurements obtained at d28 of the study. Monomeric content in % and protein concentration in mg/mL over time course of 28 days is shown in FIG. 8. The scFvs of the invention were shown to have 5% or less monomer loss upon storage at a temperature of 4°C and a concentration of >10 mg/mL.

**TABLE 11. 4w stability study at 10 mg/mL and storage temperatures of 37°C, 4°C and-80°C.**

| **Protein ID** | **Temp. [°C]** | **Conc. [mg/mL]** | **[% monomer content]** | | **% monomer loss** | **% content loss** |
|---|---|---|---|---|---|---|
| | | | d0 | d28 | d28 | d28 |
| PRO592 | 37 | | | 71.9 | 26.3 | 4.2 |
| | 4 | >10 | 97.6 | 93.4 | 4.26 | 5.03 |
| | -80 | | | 95.1 | 2.53 | 2.38 |
| PRO571 | 37 | | | 74.4 | 24.76 | 1.71 |
| | 4 | >10 | 98.9 | 95.1 | 3.84 | 2.52 |
| | -80 | | | 96.8 | 2.09 | 0.49 |

### 2.4.4 Freeze-thaw stability study

In addition to the storage stability study described above the compatibility of the scFvs PRO571 and PRO592 were assessed with respect to freeze-thawing (F/T) cycles (colloidal stability).

For the F/T stability assessment the same analytical methods and parameters (% monomer content and % monomer loss) as for the storage stability study (SE-HPLC, SDS-PAGE) were applied to monitor the quality of the molecules after five F/T cycles. Samples were formulated in PBS and were concentrated using VivaSpin concentration devices to 10 mg/mL prior to study initiation. Small sample volumes (<20µL) allowed rapid freezing and thawing intervals. Samples were subjected to five repeated freeze-thawing cycles. They were frozen at a temperature of -80°C and thawed at RT. To assess stability of PRO561 after each cycle analytical read-outs, such as protein content and purity, were recorded at different time points by SE-HPLC and UV₂₈₀ measurement. Data are presented in TABLE 12. FIG. 9 illustrates the course of monomer content in % over five repeated F/T cycles.

### 2.4.5 pH stability study

The scFvs PRO571 and PRO592 were subjected to a short-term stress stability study, in which the scFv molecules were formulated at 20 mg/ml in a set of aqueous (phosphate-citrate) buffer systems with pH values be-tween 3.5 and 7.5. Monomeric content in % and % monomer loss was analyzed after storage for 48h at 4°C and 8h at 25°C (TABLE 13). PRO571 and PRO592 exhibited <5% monomer loss in any of the tested buffer systems at any timepoint measured (TABLE 13 and TABLE 14).

It is important to note that the different studies suggested within the scope of this assessment address distinct mechanistic aspects of protein stability. While both methods are designed to give an estimation of the potential product shelf live and stability the mechanisms addressed are profoundly different. The midpoint of transition (Tm) assessed by thermal unfolding is a qualitative measure for protein domain stability (does not allow for thermodynamic determination of ΔG). Highly stable protein domains (high Tm) are less likely to spontaneously unfold at ambient temperature and thus less prone to irreversible aggregation/precipitation driven by unfolded domain interactions. High domain stability indicates dense packaging of amino acid residues, which also correlates with resistance towards protease cleavage. The SE-HPLC assessment on the other hand quantitatively determines the content of the monomeric fraction as well as of soluble oligomers/aggregates. Such soluble oligomers are oftentimes reversible and relatively loose associations driven by electrostatic or hydrophobic interactions between correctly folded proteins. There is some correlation between Tm as assessed by thermal unfolding and the propensity for oligomer/aggregate formation as assessed by SE-HPLC particularly for proteins with "border line" stability. Beyond a certain threshold Tm of approximately 60°C antibody variable domains are generally sufficiently stable to be resistant toward aggregation/precipitation and proteolytic degradation due to partial domain unfolding at ambient temperature. Oligomerization driven by hydrophobic and/or electrostatic interactions of surface residues may, however, still occur. Importantly, in an accelerated (stress) stability study at elevated temperature (e.g. 37°C) the various mechanisms of oligomer formation and precipitation may occur simultaneously.

**TABLE 12. Freeze-thaw stability assessment of selected clones.**

| **Protein ID** | **Freeze/Thaw cycles** | **Conc. [mg/mL]** | **[% monomer content]** | | | | | | **% monomer loss** | | | | |
|---|---|---|---|---|---|---|---|---|---|---|---|---|---|
| | | | FT0 | FT1 | FT2 | FT3 | FT4 | FT5 | FT1 | FT2 | FT3 | FT4 | FT5 |
| PRO592 | 5 | 10 | 97.6 | 96.1 | 95.6 | 95.4 | 95.2 | 95.2 | 1.5 | 2.0 | 2.2 | 2.4 | 2.4 |
| PRO571 | 5 | 10 | 98.9 | 98.8 | 98.8 | 98.6 | 98.5 | 98.5 | 0.1 | 0.1 | 0.3 | 0.4 | 0.4 |

**TABLE 13. Monitoring of monomeric content and monomer loss at two defined time points (t8, t48) and temperatures (4°C, 25°C) during short-term pH stress stability.**

| **Protein ID** | **pH** | **[% monomer content]** | | | **% monomer loss** | |
|---|---|---|---|---|---|---|
| | | **t0** | **t8** | **t48** | **t8** | **t48** |
| PRO571 | 3.5 | 98.0 | 95.6 | 95.7 | 2.5 | 2.3 |
| | 4.5 | 97.9 | 97.1 | 97.0 | 0.8 | 0.9 |
| | 5.5 | 96.5 | 95.8 | 95.4 | 0.8 | 1.1 |
| | 6.5 | 97.4 | 95.6 | 95.0 | 1.9 | 2.5 |
| | 7.5 | 96.2 | 95.2 | 95.0 | 1.0 | 1.3 |
| PRO592 | 3.5 | 99.5 | 98.3 | 98.4 | 1.2 | 1.1 |
| | 4.5 | 99.5 | 99.0 | 99.1 | 0.5 | 0.5 |
| | 5.5 | 99.5 | 98.8 | 98.8 | 0.7 | 0.7 |
| | 6.5 | 99.5 | 98.7 | 98.7 | 0.8 | 0.8 |
| | 7.5 | 99.5 | 98.8 | 98.8 | 0.7 | 0.7 |

**TABLE 14. Monitoring of protein concentration at two defined time points (t8, t48) and temperatures (4°C, 25°C) during short-term pH stress stability**

| **Protein ID** | **pH** | **protein concentration [mg/mL]** | | | **% content loss** | |
|---|---|---|---|---|---|---|
| | | t0 | t8 | t48 | t8 | t48 |
| PRO571 | 3.5 | 27.7 | 26.4 | 26.3 | 4.8 | 5.0 |
| | 4.5 | 26.5 | 27.1 | 26.2 | -2.3 | 1.1 |
| | 5.5 | 29.3 | 27.9 | 28.4 | 4.8 | 3.1 |
| | 6.5 | 29.5 | 27.4 | 29.2 | 7.4 | 1.1 |
| | 7.5 | 29.3 | 29.3 | 29.2 | 0.1 | 0.2 |
| PRO592 | 3.5 | 28.3 | 27.8 | 28.0 | 1.8 | 1.1 |
| | 4.5 | 26.2 | 25.1 | 26.4 | 4.5 | -0.7 |
| | 5.5 | 25.3 | 25.8 | 25.3 | -2.0 | 0.1 |
| | 6.5 | 28.4 | 27.7 | 28.1 | 2.5 | 1.1 |
| | 7.5 | 28.7 | 28.4 | 28.5 | 1.0 | 0.9 |

**TABLE 15. Summary of the biophysical characterization data.**

| **Protein ID** | **Clone ID** | **Tm [°C]** | **% monomer loss, 10 mg/mL, 5xFT** | **% monomer loss, 28d, 10 mg/mL, 37°C** | **% monomer loss, 28d, 10 mg/mL, 4°C** | **% monomer loss, 28d, 10 mg/mL,-80°C** | **% monomer loss, pH3.5-7.5, 20 mg/mL, RT, t8** | **% monomer loss, pH3.5-7.5, 20 mg/mL, 4°C, t48** |
|---|---|---|---|---|---|---|---|---|
| PRO592 | 27_31_C04_sc02 | 68.4 | 2.4 | 26.3 | 4.26 | 2.53 | 0.5-1.2 | 0.5-1.1 |
| PRO571 | 27_31_C04_sc01 | 68.8 | 0.4 | 24.76 | 3.84 | 2.09 | 0.8-2.5 | 0.9-2.3 |

### 2.4.6 Methods

### 2.4.6.1 Reducing SDS-PAGE

Sodium Dodecyl Sulfate Polyacrylamide Gel Electrophoresis (SDS-PAGE) is an analysis technique used for qualitative characterization and to control purity of proteins. According to the United States Pharmacopeia (USP) (USP Chapter 1056) analytical gel electrophoresis is an appropriate and routine method to identify and to assess the homogeneity of proteins in drug substances.

The method is used to quantify the amount of scFv product from E.coli lysates to derive the expression yield after fermentation. Another application of the method is to verify the identity of test substances based on their molecular weight with respect to the theoretical values. For supportive purposes this method is used to quantify the purity of test samples with respect to process-related impurities (host cell proteins) and product related impurities (degradation products or adducts).

The SDS-PAGE analyses were performed with commercially available precast gel system "Mini Protean" obtained from Bio-Rad Laboratories Inc. Humanized scFvs were analyzed on "Any kD" resolving gels (#456-9036). In both cases the Tris/Glycine buffer system recommended by the manufacturer was used. For the detection of protein bands coomassie staining with SimplyBlueTM staining solution (Life Technologies Corp., #LC6060) was employed. For the staining procedures the protocols of the supplier was followed.

The documentation and analysis of the stained protein gels was performed with the documentation system ChemiDoc XRS System (Bio-Rad Laboratories Inc., #170-8265) and software Image Lab, Version 4.0.1 (Bio-Rad Laboratories Inc., # 170-9690).

### 2.4.6.2 UV Absorbance at 280 nm

The method UV absorbance at 280 nm is a total protein assay as outlined in USP Chapter 1057. Protein solutions absorb UV light at a wavelength of 280 nm due to the presence of aromatic amino acids. The UV absorbance is a function of the content of tyrosine and tryptophan residues in the protein and is proportional to the protein concentration. The absorbance of an unknown protein solution can be determined according to USP Chapter 851 on spectroscopy by applying Beer's law: A= ε*1*c, where the absorbance (A) is equal to the product of the molar absorptivity (ε), the absorption path length and the concentration of the substance. The molar absorptivity for the scFv was calculated with the software Vector NTI® (Life Technologies Corporation).

The measurement of the UV absorbance is performed with the Infinity reader M200 Pro equipped with Nanoquant plate (Tecan Group Ltd.). The absorbance of the protein samples is measured at 280 nm and 310 nm, where the latter wavelength is serving as a reference signal that is subtracted from the 280 nm signal. To account for potential interference of the sample matrix a blank subtraction is performed for each measurement. The final absorbance signal of a protein sample obtained is used to calculate the protein concentration using Lambert-Beer's law.

All measurements are performed within the range given by the instruments specifications in the measurement range of 0-4 OD, where a reproducibility of < 1% and a uniformity of < 3% is specified by the manufacturer.

### 2.4.6.3 SE-HPLC (Size Exclusion High-pressure Liquid Chromatography)

SE-HPLC is a separation technique based on a solid stationary phase and a liquid mobile phase as outlined by the USP chapter 621. This method separates molecules based on their size and shape utilizing a hydrophobic stationary phase and aqueous mobile phase. The separation of molecules is occurring between the void volume (V0) and the total permeation volume (VT) of a specific column. Measurements by SE-HPLC are performed on a Chromaster HPLC system (Hitachi High-Technologies Corporation) equipped with automated sample injection and a UV detector set to the detection wavelength of 280 nm. The equipment is controlled by the software EZChrom Elite (Agilent Technologies, Version 3.3.2 SP2) which also supports analysis of resulting chromatograms. Protein samples are cleared by centrifugation and kept at a temperature of 4-6°C in the autosampler prior to injection. For the analysis of scFv samples the column Shodex KW402.5-4F (Showa Denko Inc., #F6989201) is employed with a standardized buffered saline mobile phase (50 mM Sodium acetate pH 6.0, 250 mM sodium chloride) at the recommended flow rate of 0.35 mL/min. The target sample load per injection was 5 µg. Samples are detected by an UV detector at a wavelength of 280 nm and the data recorded by a suitable software suite. The resulting chromatograms are analyzed in the range of V0 to VT thereby excluding matrix associated peaks with >10 min elution time.

To ensure intermediate precision of the method, a reference standard is routinely measured at the beginning and end of each HPLC sequence. The reference standard used for this system suitability test is an scFv that has been produced as a batch and is aliquoted to be used for each measurement timepoint.

### 2.4.6.4 DSF (Differential Scanning Fluorimetry)

The midpoint of transition for the thermal unfolding of the tested scFv constructs was determined by Differential Scanning Fluorimetry (DSF), essentially as described by Niesen (Niesen et al., Nat Protoc. 2 (2007) 2212-21). The method DSF is a non-compendial method to measure temperature-dependent protein unfolding. The measurement of the thermal unfolding temperature by DSF are performed with a MX3005P qPCR machine (Agilent Technologies) controlled with the MX Pro software package (Agilent Technologies) and equipped with an excitation/emission filter set at 492/610 nm. The reactions are set-up in Thermo fast 96 white PCR plates (Abgene; #AB-0600/W). For the detection of protein unfolding a commercially available stock solution of the dye SYPRO orange (Molecular Probes; # S6650) is used at a final dilution of 1:1'000. The protein samples are diluted for the unfolding measurements to a final concentration of 50 µg/mL in a standardized buffered saline solution. The thermal unfolding is performed by a temperature program starting at 25°C ramping up to 96°C in 1°C steps with a duration of 30 seconds. During the temperature program the fluorescence emission of each sample is recorded. The recorded raw data is processed and evaluated with a package of Microsoft Excel templates (Niesen, Nature Protocols 2007, Vol. 2 No.9) and the fluorescence data is fitted with a Boltzmann equation using the program GraphPad Prism (GraphPad Software, Inc.) to obtain the midpoint of transition (Tm).

In order to produce reliable and robust measurements of the midpoint of unfolding at least duplicate measurements are performed. For an assessment of the intermediate precision a reference standard (known characterized scFv) is included with every measurement to allow for comparison of assay performance on different days.

### 2.4.6.5 Stability Study

In order to assess the stability of different scFv constructs as a read-out for the developability of these molecules a short-term stability study protocol can be designed. The protein constructs are concentrated in a simple buffered saline formulation (see above) to the target concentrations of 10 mg/mL. The monomer content is determined by SE-HPLC to confirm that the purity is exceeding the success criteria of > 95%. Subsequently the protein samples are stored at <-80°C, 4°C and 37°C for the duration of 4 weeks and aliquots are analyzed at various time points. The primary read-out is the analysis by SE-HPLC, which allows the quantification of soluble higher molecular weight oligomers and aggregates. As supportive measurements the protein content is determined by UV absorbance at 280 nm, which gives an indication whether during the storage period substantial amounts of protein were lost by precipitation. Additionally, purity at the end point of the study is determined by SDS-PAGE that indicates the stability of the construct with respect to degradation or covalent multimerization.

## Claims

1. An isolated antibody having a binding specificity for human IL-17A, which comprises a heavy chain variable region (VH) and a light chain variable region (VL), wherein:
(a) said VH comprises, in sequence, the three complementary determining regions HCDR1, HCDR2 and HCDR3, and
(b) said VL comprises, in sequence, the three complementary determining regions LCDR1, LCDR2 and LCDR3, and
wherein said HCDR1, HCDR2, and HCDR3 sequences are set forth in SEQ ID NOs: 1, 2, and 3, respectively, and LCDR1, LCDR2, and LCDR3 sequences are set forth in SEQ ID NOs: 12, 13, and 14, respectively.

2. The antibody of claim 1, wherein said VH is VH3 or VH4, preferably VH3.

3. The antibody of claim 1 or claim 2, wherein said VL comprises Vκ frameworks FR1, FR2 and FR3, particularly Vκ1 or VK3 FR1 to FR3, preferably Vκ1 FR1 to FR3, and a framework FR4, which is selected from a Vκ FR4, particularly Vκ1 FR4, Vκ3 FR4, and Vλ FR4, particularly Vλ FR4 comprising the amino acid sequence having at least 60, 70, 80, 90 percent identity to an amino acid sequence selected from any of SEQ ID NO: 26 to SEQ ID NO: 32, preferably Vλ FR4 as set forth in any of SEQ ID NO: 26 to SEQ ID NO: 32, preferably Vλ FR4 as set forth in SEQ ID NO: 26 or 27, more preferably Vλ FR4 as set forth in SEQ ID NO: 27.

4. The antibody of anyone of claims 1 to 3, wherein said VH comprises an amino acid sequence that is at least 90 percent identical to the amino acid sequence SEQ ID NO: 10; and/or said VL comprises an amino acid sequence that is at least 90 percent identical to the amino acid sequence SEQ ID NO: 21.

5. The antibody of claim 4, comprising a VH sequence of SEQ ID NO: 10 and/or a VL sequence of SEQ ID NO: 21.

6. The antibody of any of the preceding claims, wherein said antibody:
(a) has the ability to block interaction between IL-17A and IL-17RA with a potency relative to that of secukinumab (relative potency), determined in ELISA assay, greater than 5, preferably greater than 10, more preferably greater than 20, and wherein said relative potency is the ratio of the IC₅₀ value in ng/mL of secukinumab as measured by ELISA to the IC₅₀ value in ng/mL of the antibody of the invention in the scFv format as measured by ELISA; and/or
(b) has the ability to neutralize IL-17A with a potency relative to that of secukinumab (relative potency), determined by measuring Gro-α secretion in an HT-29 assay, greater than 50, preferably greater than 100, more preferably greater than 150, and wherein said relative potency is the ratio of the IC₅₀ value in ng/mL of secukinumab as measured in the HT-29 assay to the IC₅₀ value in ng/mL of the antibody of the invention in the scFv format as measured in the HT-29 assay; and/or
(c) capable of inhibiting the activity of 1 ng human IL-17A at a concentration of 1 ng/mL or less, preferably 0.5 ng/mL or less, more preferably 0.2 ng/mL or less, by 50%, said inhibitory activity is determined by measuring Gro-α secretion induced by human IL-17A in HT-29 assay in the presence of 50 pg/ml TNFα.

7. The antibody of any of the preceding claims, wherein said antibody:
(a) binds to human IL-17A with a dissociation constant (K_{D}) of less than 5 nM, e.g., less than 1 nM, less than 0.5 nM, less than 0.2 nM, preferably less than 100 pM, more preferably less than 50 pM, as measured by surface plasmon resonance, preferably as measured by surface plasmon resonance in a direct setup; and
(b) optionally, binds to Cynomolgus IL-17A with a K_{D} of less than 10 nM, e.g., less than 7 nM, less than 5 nM, less than 2 nM, less than 1 nM, preferably less than 0.5 nM as measured by surface plasmon resonance, preferably as measured by surface plasmon resonance in a capture setup.

8. The antibody of any of the preceding claims, wherein said antibody:
(a) when in scFv format, has a melting temperature (Tm), determined by differential scanning fluorimetry, of at least 60°C, preferably of at least 62°C, more preferably at least 65°C;
(b) has a loss in monomer content, after five consecutive freeze-thaw cycles, of less than 5%, preferably less than 3%, when the antibody of the invention is at a starting concentration of 10 mg/ml; and/or
(c) has a loss in monomer content, after storage for at least two weeks, particularly for at least four weeks, at 4°C, of 5% or less, when the antibody of the invention is at a starting concentration of 10 mg/ml.

9. The antibody of any of the preceding claims, wherein the antibody is selected from the group consisting of: a monoclonal antibody, a chimeric antibody, a Fab, an Fv, an scFv, dsFv, an scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. F-star's Modular Antibody Technology™), preferably scFv.

10. The antibody of any one of claims 1 to 9 which is a multispecific molecule.

11. A pharmaceutical composition comprising the antibody of any one of claims 1 to 10, and a pharmaceutically acceptable carrier.

12. The antibody of any one of claims 1 to 10, or the composition of claim 11 for use as a medicament.

13. The antibody of any one of claims 1 to 10, or the composition of claim 11 for use in the treatment of a disorder mediated by IL-17A or a disorder that can be treated by inhibiting Gro-α secretion.

14. A nucleic acid encoding the antibody of claims 1-10.

15. A kit comprising the antibody of any one of claims 1 to 10, or the composition of claim 11.
